(19) Europäisches Patentamt
European Patent Office
Office européen des brevets

(11) EP 2 231 710 B1

(12) **EUROPEAN PATENT SPECIFICATION**

(45) Date of publication and mention of the grant of the patent:
**20.06.2012 Bulletin 2012/25**

(21) Application number: **08861560.4**

(22) Date of filing: **15.12.2008**

(51) Int Cl.:
***C08B 31/12*** (2006.01) ***A61K 47/48*** (2006.01)

(86) International application number:
**PCT/EP2008/010659**

(87) International publication number:
**WO 2009/077153 (25.06.2009 Gazette 2009/26)**

(54) **METHOD FOR PRODUCING A HYDROXYALKYL STARCH DERIVATIVE WITH TWO LINKERS**

VERFAHREN ZUR HERSTELLUNG EINES HYDROXYALKYLSTÄRKEDERIVATS MIT ZWEI LINKERN

PROCÉDÉ DE PRODUCTION D'UN DÉRIVÉ D'AMIDON HYDROXYALKYLÉ COMPORTANT DEUX SÉQUENCES DE LIAISON

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **14.12.2007 EP 07024351**

(43) Date of publication of application:
**29.09.2010 Bulletin 2010/39**

(73) Proprietor: **Fresenius Kabi Deutschland GmbH**
**61352 Bad Homburg (DE)**

(72) Inventors:
- **HACKET, Frank**
  **63674 Altenstadt (DE)**
- **EICHNER, Wolfram**
  **33510 Butzbach (DE)**
- **KNOLLER, Helmut**
  **61169 Friedberg (DE)**
- **MITSCH, Andreas**
  **93138 Lappersdorf (DE)**
- **SCHIMMEL, Martin**
  **61440 Oberursel (DE)**
- **ZANDER, Norbert**
  **61231 Bad Nauheim (DE)**
- **HAUSCHILD, Franziska**
  **61231 Bad Nauheim (DE)**

(74) Representative: **Altmann, Andreas et al**
**Herzog Fiesser & Partner**
**Patentanwälte**
**Isartorplatz 1**
**80331 München (DE)**

(56) References cited:
**US-A1- 2005 234 230** **US-A1- 2005 238 723**

EP 2 231 710 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

The invention relates to a method of producing a hydroxyalkyl starch (HAS) derivative, comprising a) reacting optionally oxidized hydroxyalkyl starch with a compound (D) comprising at least two functional groups $-O-NH_2$ or a salt thereof, and b) reacting the hydroxyalkyl starch derivative obtained in step a) with a compound (L) comprising at least two functional groups independently selected from the group consisting of an aldehyde group, a suitably protected aldehyde group, a keto group, and a suitably protected keto group. In particular, compound (L) comprises at least two functional groups $W_1$ and $W_2$ independently selected from the group consisting of an aldehyde group, a hemiacetal group, $-CH(OH)_2$, an acetal group, a keto group, and a ketal group. In case either $W_1$ or $W_2$ or $W_1$ and $W_2$ is/are a keto group -C(O)-R, R is preferably selected from the group consisting of a saturated or unsaturated, cyclic or linear, branched or unbranched, substituted or unsubstituted alkyl and a substituted or unsubstituted aryl group. Additionally, the present invention relates to hydroxyalkyl starch derivatives, in particular hydroxyethyl starch derivatives, obtainable and/or obtained by said method, and the use thereof. Moreover, the present invention relates to specific hydroxyalkyl starch derivatives, in particular hydroxyethyl starch derivatives as such.

Hydroxyalkyl starch (HAS), in particular hydroxyethyl starch (HES), is a substituted derivative of naturally occurring carbohydrate polymer amylopectin, which is present in corn starch at a concentration of up to 95 % by weight, and is degraded by alpha-amylase in the body. HES, in particular, exhibits advantageous biological properties and is used as a blood volume replacement agent and in hemodilution therapy in the clinics (Sommermeyer et al., 1987, Krankenhauspharmazie, 8(8), 271-278; Weidler et al., 1991, Arzrieimittelforschung/Drug Res., 41, 494-498).

Some ways of producing a hydroxyethyl starch derivative are described in the art.

DE 26 16 086 discloses the conjugation of hemoglobin to hydroxyethyl starch wherein, in a first step, a cross-linking agent, e.g. bromocyane, is bound to hydroxyethyl starch and subsequently hemoglobin is linked to the intermediate product.

One important field in which HES is used is the stabilization of polypeptides which are applied, e.g., to the circulatory system in order to obtain a particular physiological effect. One specific example of these polypeptides is erythropoietin, an acid glycoprotein of approximately 34,000 kDa which is essential in regulating the level of red blood cells in the circulation.

A well-known problem with the application of polypeptides and enzymes is that these proteins often exhibit an unsatisfactory stability. Especially erythropoietin has a relatively short plasma half live (Spivak and Hogans, 1989, Blood 73, 90; McMahon et al., 1990, Blood 76, 1718). This means that therapeutic plasma levels are rapidly lost and repeated intravenous administrations must be carried out. Furthermore, in certain circumstances an immune response against the peptides is observed.

It is generally accepted that the stability of polypeptides can be improved and the immune response against these polypeptides is reduced when the polypeptides are coupled to polymeric molecules.

WO 94/28024 discloses that physiologically active polypeptides modified with polyethylene glycol (PEG) exhibit reduced immunogenicity and antigenicity and circulate in the bloodstream considerably longer than unconjugated proteins, i.e. have a longer clearance rate. However, PEG-drug conjugates exhibit several disadvantages, e.g. they do not exhibit a natural structure which can be recognized by elements of in vivo degradation pathways. Therefore, apart from PEG-conjugates, other conjugates and protein polymerates have been produced.

WO 02/080979 discloses compounds comprising a conjugate of an active agent and a hydroxyalkyl starch wherein active agent and hydroxyalkyl starch are either linked directly or via a linker compound. As far as the direct linkage is concerned, the reaction of active agent and hydroxyalkyl starch is carried out in an aqueous medium which comprises at least 10 wt.-% of water. No examples are given which are directed to a hydroxyalkyl starch derivative which is linked to a carbonyl group comprised in the active reagent, neither an aldehyde or keto group nor an acetal or a hemiacetal group.

WO 03/074087 discloses hydroxyalkyl starch protein conjugates in which the bonding between the hydroxyalkyl starch molecule and the protein is covalent and is the result of a coupling of a terminal aldehyde or a functional group which resulted from the reaction of the aldehyde group with a functional group of a protein.

WO 03/074088 discloses hydroxyalkyl starch conjugates with a low molecular weight compound in which the bonding between the hydroxyalkyl starch and the low molecular weight compound is covalent and is the result of a coupling of a terminal aldehyde or a functional group which resulted from the reaction of the aldehyde group with a functional group of the low molecular weight compound.

WO 2005/014024 discloses polymers functionalized by an aminooxy group or a derivative thereof, conjugates, wherein the functionalized polymers are covalently coupled with a protein by an oxime linking group, a process for preparing the functionalized polymers, a process for preparing the conjugates, functionalized polymers as obtainable by the process of the present invention, conjugates as obtainable by the process, and pharmaceutical compositions comprising at least one conjugate and the use of said conjugates and compositions for the prophylaxis or therapy of the human or animal body.

WO 2005/092390 discloses conjugates of hydroxyalkyl starch and a protein wherein these conjugates are formed by a covalent linkage between the hydroxyalkyl starch or a derivative of the hydroxyalkyl starch and the protein and a method of producing these conjugates and the use of these conjugates.

WO 2004/024777 discloses hydroxyalkyl starch derivates, particularly hydroxyalkyl starch derivatives obtainable by a process in which hydroxyalkyl starch is reacted with a primary or secondary amino group of a linker compound. According to an especially preferred embodiment, WO 2004/024777 discloses hydroxyalkyl starch derivatives obtainable by a process according to which hydroxyalkyl starch is reacted with a primary or secondary amino group of a linker compound and the resulting reaction product is reacted with a polypeptide, preferably with a glycoprotein and especially preferably with erythropoietin, via at least one other reactive group of the linker compound. A hydroxyalkyl starch which is especially preferred is hydroxyethyl starch. According to WO 2004/024777, the hydroxyalkyl starch and preferably the hydroxyl ethyl starch is reacted with the linker compound at its reducing end which is not oxidized prior to the reaction.

WO 2004/024776 discloses hydroxyalkyl starch derivates, particularly hydroxyalkyl starch derivatives obtainable by a process in which hydroxyalkyl starch is reacted with a primary or secondary amino group of a crosslinking compound or with two crosslinking compounds wherein the resulting hydroxyalkyl starch derivative has at least one functional group X which is capable of being reacted with a functional group Y of a further compound and wherein this group Y is an aldehyde group, a keto group, a hemiacetal group, an acetal group, or a thio group. According to an especially preferred embodiment, WO 2004/024776 relates to hydroxyalkyl starch derivatives obtainable by a process according to which hydroxyalkyl starch is reacted with a primary or secondary amino group of a crosslinking compound, the resulting reaction product optionally being further reacted with a second crosslinking compound, wherein the resulting hydroxyalkyl starch derivative has at least one functional group X which is capable of being reacted with a functional group Y of a further compound and wherein this group Y is an aldehyde group, a keto group, a hemiacetal group, an acetal group, or a thio group, and the resulting reaction product is reacted with a polypeptide, preferably with a polypeptide such as AT III, IFN-beta or erythropoietin and especially preferably with erythropoietin, which comprises at least one of these functional groups Y. A hydroxyalkyl starch which is especially preferred is hydroxyethyl starch. According to WO 2004/024776 the hydroxyalkyl starch and preferably the hydroxyethyl starch is reacted with the linker compound at its reducing end which is optionally oxidized prior to the reaction. WO 2004/024776 is silent on a process wherein a HAS derivative, prepared by reacting HAS with a first linker compound, is reacted with a second linker compound via the reaction of a functional group $-O-NH_2$ of the HAS derivative and a functional group of the second linker compound wherein an oxime linkage is obtained.

Also DE 30 29 307 A1, relating to a process wherein hemoglobin is linked to a polysaccharide via a spacer, is silent on a process wherein a HAS derivative, prepared by reacting HAS with a first linker compound, is reacted with a second linker compound via the reaction of a functional group $-O-NH_2$ of the HAS derivative and a functional group of the second linker compound wherein an oxime linkage is obtained.

WO 2005/092928 discloses conjugates of hydroxyalkyl starch, preferably hydroxyethyl starch, and a protein, wherein these conjugates are formed by a reductive amination reaction between at least one aldehyde group of the hydroxyalkyl starch or of a derivative of the hydroxyalkyl starch, and at least one amino group of the protein, so that the hydroxyalkyl starch or the derivative thereof is covalently linked to the protein via an azomethine linkage or a aminomethylene linkage. WO 2005/092928 also relates to a method of producing these conjugates and specific uses of the conjugates.

US 2006/0194940 A1 discloses water-soluble polymer alkanals. Among others, protected aldehyde reagents are disclosed which are reacted with a polymer. While poly(saccharides) are generically mentioned, especially preferred polymers are polyethylene glycols. Starches or, in particular, modified starches such as hydroxyalkyl starches are not disclosed in US 2006/0194940 A1. Consequently, US 2006/0194940 A1 contains no disclosures concerning specific ways of coupling a given linker compound to hydroxyalkyl starch. The same applies to US 7,157,546 B2, EP 1591 467 A1 and WO 2004/022630 A2.

US 6,916,962 B2 discloses an aminoacetal crosslinking compound in unprotected and protected form. No disclosure is contained in this document relating to a possible coupling of this crosslinking compound with polymers other than polyethylene glycols. In particular, starches, let alone modified starches such as hydroxyalkyl starches are not disclosed in US 6,916,962 B2. Consequently, US 6,916,962 B2 contains no disclosures concerning specific ways of coupling a given linker compound to hydroxyalkyl starch. The same applies to US 6,956,135 B2 and WO 03/049699 A2.

US 3.990,237 discloses structures containing a protected aldehyde group. Compounds comprising these structures are preferably coupled to polyethylene glycol, and coupling is carried out via a halide as functional group comprised in the protected aldehyde group containing compounds, which halide group reacts with a hydroxy group of the polyethylene glycol.

It is an object of the present invention to provide a novel method to obtain hydroxyalkyl starch derivatives.

It is a further object of the present invention to provide a novel method to obtain hydroxyalkyl starch derivatives allowing for preferred reaction conditions of a given hydroxyalkyl starch derivative with the amino group of a biologically active substance.

It is further object of the present invention to provide novel hydroxyalkyl starch derivatives, in particular hydroxyethyl starch derivatives which, in particular, are characterized by a novel spacer structure between hydroxyalkyl starch, in particular hydroxyethyl starch, and a given biologically agent, in particular proteins.

Therefore, the present invention relates to a method of producing a hydroxyalkyl starch (HAS) derivative, comprising

a) reacting optionally oxidized hydroxyalkyl starch with a compound (D) comprising at least two functional groups $-O-NH_2$ or a salt thereof, and

b) reacting the "hydroxyalkyl starch derivatives obtained in step a) with a compound (L) comprising at least two functional groups $W_1$ and $W_2$ independently selected from the group consisting of an aldehyde group, a suitably protected aldehyde group, a keto group, and a suitably protected keto group.

wherein compound (L) used in step b) has the structure according to formula (III)

$$W_1\text{-}R_9\text{-}W_2 \qquad \text{(III)}$$

wherein $R_9$ is selected from a chemical bond, preferably a single bond, a saturated or unsaturated, cyclic or linear, branched or unbranched, substituted or unsubstituted alkylene, possibly containing heteroatoms in the alkylene chain, a substituted or unsubstituted arylene and a substituted or unsubstituted heteroarylene, a substituted or unsubstituted aralkylene, a substituted or unsubstituted alkarylene, and a substituted or unsubstituted heteroarylene, a substituted or unsubstituted heteroaralkylene, and a substituted or unsubstituted alkheteroarylene.

1. Hydroxyalkyl Starch

In the context of the present intention, the term "hydroxyalkyl starch" (HAS) refers to a starch derivative which has been substituted by at least one hydroxyalkyl group. A preferred hydroxyalkyl starch of the present invention has a constitution according to formula (I')

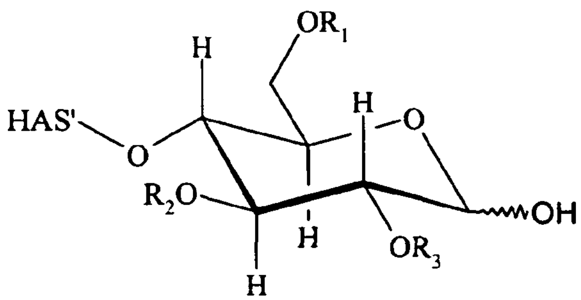

(I)

wherein HAS' is the remainder of the hydroxyalkyl starch molecule and $R_1$, $R_2$ and $R_3$ are independently hydrogen, a linear or branched hydroxyalkyl group or the group

$$—[(CR^1R^2)_mO]_n[CR^3R^4]_o—OH$$

wherein $R^1$, $R^2$, $R^3$, and $R^4$ are independently selected from the group consisting of hydrogen, and alkyl group, preferably hydrogen and methyl group,
m is 2 to 4, wherein the residues $R^1$ and $R^2$ may be the same or different in the m groups $CR^1R^2$;
n is 0 to 20, preferably 0 to 4;
o is 2 to 20, preferably 2 to 4, wherein the residues $R^3$ and $R^4$ may be the same or different in the o groups $CR^3R^4$.

Preferably, $R_1$, $R_2$ and $R_3$ are independently a group $-(CH_2CH_2O)_n-H$, wherein n is an integer, preferably 0, 1, 2, 3, 4, 5, or 6, and in particular, $R_1$, $R_2$ and $R_3$ are independently hydrogen or 2-hydroxyethyl.

In formula (I) the reducing end of the starch molecule is shown in the non-oxidised form and the terminal saccharide unit of HAS is shown in the hemiacetal form which, depending on e.g. the solvent, may be in equilibrium with the (free) aldehyde form. The abbreviation HAS' as used in the context of the present invention refers to the HAS molecule without the terminal saccharide unit at the reducing end of the HAS molecule. This is meant by the term "remainder of the hydroxyalkyl starch molecule" as used in the context of the present invention.

The term "hydroxyalkyl starch" as used in the present invention is not limited to compounds where the terminal carbohydrate moiety comprises hydroxyalkyl groups $R_1$, $R_2$ and/or $R_3$ as depicted, for the sake of brevity, in formula (I), but also refers to compounds in which at least one hydroxy group which is present anywhere, either in the terminal carbohydrate moiety and/or in the remainder of the hydroxyalkyl starch molecule, HAS', is substituted by a hydroxyalkyl group $R_1$, $R_2$ and/or $R_3$.

Hydroxyalkyl starch comprising two or more different hydroxyalkyl groups is also possible.

The at least one hydroxyalkyl group comprised in HAS may contain one or more, in particular two or more hydroxy groups. According to a preferred embodiment, the at least one hydroxyalkyl group comprised in HAS contains one hydroxy group.

The expression "hydroxyalkyl starch" also includes derivatives wherein the alkyl group is mono- or polysubstituted. In this context, it is preferred that the alkyl group is substituted with a halogen, especially fluorine, or with an aryl group. Furthermore, the hydroxy group of a hydroxyalkyl group may be esterified or etherified.

Furthermore, instead of alkyl, also linear or branched substituted or unsubstituted alkenyl groups may be used.

Hydroxyalkyl starch is an ether derivative of starch. Besides of said ether derivatives, also other starch derivatives can be used in the context of the present invention. For example, derivatives are useful which comprise esterified hydroxy groups. These derivatives may be e.g. derivatives of unsubstituted mono- or dicarboxylic acids with 2-12 carbon atoms or of substituted derivatives thereof. Especially useful are derivatives of unsubstituted monocarboxylic acids with 2-6 carbon atoms, especially derivatives of acetic acid. In this context, acetyl starch, butyryl starch and propionyl starch are preferred.

Furthermore, derivatives of unsubstituted dicarboxylic acids with 2-6 carbon atoms are preferred.

In the case of derivatives of dicarboxylic acids, it is useful that the second carboxy group of the dicarboxylic acid is also esterified. Furthermore, derivatives of monoalkyl esters of dicarboxylic acids are also suitable in the context of the present invention.

For the substituted mono- or dicarboxylic acids, the substitute groups may be preferably the same as mentioned above for substituted alkyl residues.

Techniques for the esterification of starch are known in the art (see e.g. Klemm D. et al, Comprehensive Cellulose Chemistry Vol. 2, 1998, Wiley-VCH, Weinheim, New York, especially chapter 4.4, Esterification of Cellulose (ISBN 3-527-29489-9).

According to a preferred embodiment of the present invention, hydroxyalkyl starch according to above-mentioned formula (I) is employed. The other saccharide ring structures comprised in HAS' may be the same as or different from the explicitly described saccharide ring, with the difference that they lack a reducing end.

As far as the residues $R_1$, $R_2$ and $R_3$ according to formula (I) are concerned there are no specific limitations. According to a preferred embodiment, $R_1$, $R_2$ and $R_3$ are independently hydrogen or a hydroxyalkyl group, a hydroxyaralkyl group or a hydroxyalkaryl group having of from 2 to 10 carbon atoms in the respective alkyl residue. Hydrogen and hydroxyalkyl groups having of from 2 to 10 carbon atoms are preferred. More preferably, the hydroxyalkyl group has from 2 to 6 carbon atoms, more preferably from 2 to 4 carbon atoms, and even more preferably from 2 to 3 carbon atoms. In a preferred embodiment, hydroxyalkyl starch is hydroxyethyl starch in which $R_1$, $R_2$ and $R_3$ are independently hydrogen or a group $(CH_2CH_2O)_n$-H, wherein n is an integer, preferably 0, 1, 2, 3, 4, 5, or 6.

"Hydroxyalkyl starch" therefore preferably comprises hydroxyethyl starch, hydroxypropyl starch and hydroxybutyl starch, wherein hydroxyethyl starch and hydroxypropyl starch are particularly preferred and hydroxyethyl starch is most preferred.

The alkyl, aralkyl and/or alkaryl group may be linear or branched and suitably substituted.

Therefore, the present invention also relates to a method and a HAS derivative as described above wherein $R_1$, $R_2$ and $R_3$ are independently hydrogen or a linear or branched hydroxyalkyl group with from 2 to 6 carbon atoms.

Thus, $R_1$, $R_2$ and $R_3$ preferably may be H, hydroxyhexyl, hydroxypentyl, hydroxybutyl, hydroxypropyl such as 2-hydroxypropyl, 3-hydroxypropyl, 1-hydroxyisopropyl, hydroxyethyl such as 2-hydroxyethyl, hydrogen and the 2-hydroxyethyl group being especially preferred.

Therefore, the present invention also relates to a method and a HAS derivative as described above wherein $R_1$, $R_2$ and $R_3$ are independently hydrogen or a 2-hydroxyethyl group, an embodiment wherein at least one residue $R_1$, $R_2$ and $R_3$ being 2-hydroxyethyl being especially preferred.

Hydroxyethyl starch (HES) is most preferred for all embodiments of the present invention.

Therefore, the present invention relates to the method and a HAS derivative as described above, wherein the polymer is hydroxyethyl starch and the derivative is a hydroxyethyl starch (HES) derivative.

HAS, in particular HES, is mainly characterized by the molecular weight distribution, the degree of substitution and the ratio of $C_2$ : $C_6$ substitution. There are two possibilities of describing the substitution degree:

The degree of substitution (DS) of HAS is described relatively to the portion of substituted glucose monomers with respect to all glucose moieties.

The substitution pattern of HAS can also be described as the molar substitution (MS), wherein the number of hydroxyethyl groups per glucose moiety is counted.

In the context of the present invention, the substitution pattern of HAS, preferably HES, is referred to as MS, as described above (see also Sommermeyer et al., 1987, Krankenhauspharmazie, 8(8), 271-278, in particular p. 273).

MS is determined by Gas Chromatography after total hydrolysis of the HES molecule. MS values of respective HAS, in particular HES starting material are given. It is assumed that the MS value is not affected during the derivatization procedure in steps a) and b) of the process of the invention.

HAS and in particular HES solutions are present as polydisperse compositions, wherein each molecule differs from the other with respect to the polymerization degree, the number and pattern of branching sites, and the substitution pattern. HAS and in particular HES is therefore a mixture of compounds with different molecular weight. Consequently, a particular HAS and in particular HES solution is determined by average molecular weight with the help of statistical means. In this context, $M_n$ is calculated as the arithmetic mean depending on the number of molecules. Alternatively, $M_w$ (or MW), the weight average molecular weight, represents a unit which depends on the mass of the HAS, in particular HES.

In this context the number average molecular weight is defined by equation 1:

$$\overline{M}_n = \frac{\sum_i n_i \cdot M_i}{\sum_i n_i}$$

where $n_i$ is the number of molecules of species i of molar mass $M_i$.
$\overline{M}_n$ indicates that the value is an average, but the line is normally omitted by convention.

$M_w$ is the weight average molecular weight, defined by equation 2:

$$\overline{M}_w = \frac{\sum_i n_i \cdot M_i^2}{\sum_i n_i M_i}$$

where $n_i$ is the number of molecules of species i of molar mass $M_i$
$\overline{M}_w$ indicates that the value is an average, but the line is normally omitted by convention.

Preferably, the hydroxyalkyl starch, in particular the hydroxyethyl starch, used in the invention has a mean molecular weight (weight mean) of from 1 to about 1000 kDa, more preferably from about 1 to about 800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa. Hydroxyethyl starch can further exhibit a preferred molar substitution of from 0.1 to 3, preferably 0.1 to 2, more preferred 0.1 to 0.9 or 0.4 to 2, preferably 0.4 to 1.3, and a preferred ratio between $C_2$ : $C_6$ substitution in the range of from 2 to 20 with respect to the hydroxyethyl groups.

The term "mean molecular weight" as used in the context of the present invention relates to the weight as determined according to the LALLS-(low angle laser light scattering)-GPC method as described in Sommermeyer et al., 1987, Krankenhauspharmazie, 8(8), 271-278; and Weidler et al., 1991, Arzneim.-Forschung/Drug Res., 41, 494-498. For mean molecular weights of 10 kDa and smaller, additionally, the calibration was carried out with a standard which had previously been qualified by LALLS-GPC.

According to a preferred embodiment of the present invention, the mean molecular weight of hydroxyethyl starch employed is from about 1 to about 1000 kDa, more preferably from about 1 to about 800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa.

Further, the molar substitution of HAS and in particular HES is preferably from about 0.1 to about 3, preferably about 0.4 to about 1.3, such as 0.4, 0.5, 0.6, 0.7 0.8, 0.9, 1.0, 1.1, 1.2, or 1.3.

An example of HES having a mean molecular weight of about 5 to 300 kDa, preferably 50 to 150 kDa is a HES with a molar substitution of 0.1 to 3, preferably 0.4 to 1.3, such as 0.4, 0.5, 0.6, 0.7 0.8, 0.9, 1.0, 1, 1 ,12, or 1.3.

As far as the ratio of $C_2$ : $C_6$ substitution is concerned, said substitution is preferably in the range of from 2 to

20, more preferably in the range of from 2 to 15 and even more preferably in the range of from 3 to 12.

II. Step a)

In step a) of the method of the invention, optionally oxidized HAS is reacted with a compound (D) comprising at least two functional groups $-O-NH_2$ or a salt thereof.

In a preferred embodiment, HAS is not oxidized prior to the reaction with compound (D). In a particular preferred embodiment, HAS is reacted with compounds D) at the at least one reducing end of HAS which is not oxidized prior to the reaction with compounds (D).

The term "the HAS is reacted at the at least one reducing end" as used in the context of the present invention may relate to a process according to which the HAS is reacted predominantly via its reducing end.

This term "predominantly via its reducing end" relates to processes according to which statistically more than 50 %, preferably at least 55 %, more preferably at least 60 %, more preferably at least 65 %, more preferably at least 70 %, more preferably at least 75 %, more preferably at least 80 %, more preferably at least 85 %, more preferably at least 90 %, and still more preferably, at least 95 % such as 95 %, 96 %, 97 %, 98 %, or 99 % of the HAS molecules employed for a given reaction are reacted via at least one reducing end per HAS molecule, wherein a given HAS molecule which is reacted via at least one reducing end can be reacted in the same given reaction via at least one further suitable functional group which is comprised in said polymer molecule and which is not a reducing end. If one or more HAS molecules) is (are) reacted vie at least one reducing end and simultaneously via at least one further suitable functional group which is comprised in this (these) HAS molecule(s) and which is not a reducing end, statistically preferably more than 50 %, preferably at least 55 %, more preferably at least 60 %, more preferably at least 65 %, more preferably at Least 70 %, more preferably at least 75 %, more preferably at least 80 %, more preferably at least 85 %, more preferably at least 90 %, and still more preferably at least 95 % such as 95 %, 96 %, 97 %, 98 %, or 99 % of all reacted functional groups of these HAS molecules, said functional groups including the reducing ends, are reducing ends.

The term "reducing end" as used in the context of the present invention relates to the terminal aldehyde group of a HAS molecule which may be present as aldehyde group and/or as corresponding hemiacetal form and/or as acetal group, the acetal group having the following structure

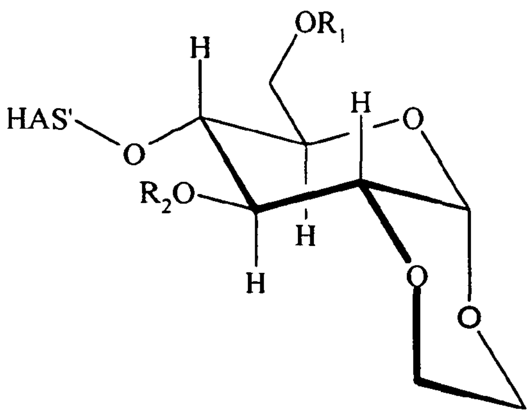

which can be present if residue $-OR_3$ according to formula (I) above is $-O-CH-CH_2-OH$.

If HAS is oxidized prior to the reaction with compound (D) the reaction can, in one embodiment, be carried out so that at least two aldehyde groups are introduced into HAS according to the following formula

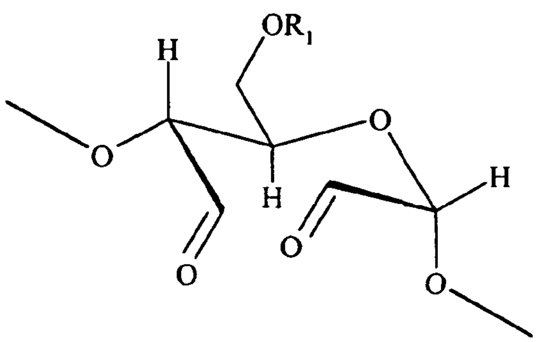

According to this embodiment of the present invention, each oxidation agent or combination of oxidation agents may be employed which is capable of oxidizing at least one saccharide ring of the polymer to give an opened saccharide ring having at least one, preferably at least two aldehyde groups. This reaction is illustrated by the following reaction scheme which shows a saccharide ring of the polymer which is oxidized to give an opened ring having two aldehyde groups:

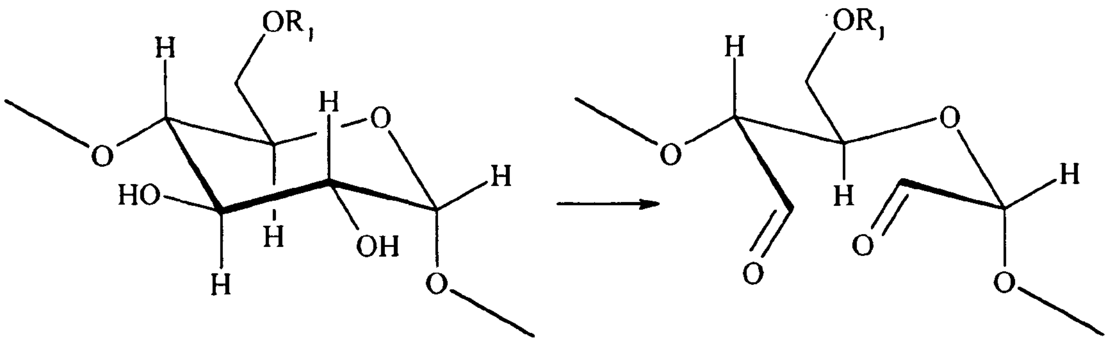

Suitable oxidizing agents are, among others, periodates such as alkaline metal periodates or mixtures of two or more thereof, with sodium periodate and potassium periodate being preferred.

In a preferred embodiment, compound (D) or a salt thereof is reacted via at least one of the at least two functional groups $-O-NH_2$ with an aldehyde and/or hemiacetal group and/or acetal group of the hydroxyalkyl starch in step a), in particular compound (D) or a salt thereof is reacted via at least one of the at least two functional groups $-O-NH_2$ with the at least one reducing end of the hydroxyalkyl starch and wherein the hydroxyalkyl starch is not oxidized prior to the reaction in step a).

In a preferred embodiment, compound (D) used in step a) has the structure according to formula (II)

$$H_2N-O-R_4-O-NH_2 \qquad (II)$$

or a salt thereof wherein $R_4$ is selected from a saturated or unsaturated, cyclic or linear, branched or unbranched, substituted or unsubstituted alkylene, possibly containing heteroatoms in the alkylene chain, a substituted or unsubstituted arylene, a substituted or unsubstituted aralkylene, a substituted or unsubstituted alkarylene, and a substituted or unsubstituted heteroarylene, a substituted or unsubstituted heteroaralkylene, and a substituted or unsubstituted alkheteroarylene. A salt of compound D) is preferably a compound D) in which the functional groups $-O-NH_2$ are protonated and independently of each other groups $-O-NH_3^+$ with a pharmaceutically acceptable anion, such as chloride, hydrogen sulfate, sulfate, carbonate, hydrogen carbonate, citrate, phosphate and/or hydrogen phosphate.

In a preferred embodiment, wherein $R_4$ is selected from a saturated or unsaturated, cyclic or linear, branched or unbranched, substituted or unsubstituted alkylene, and does not contain heteroatoms in the alkylene chain, a substituted or unsubstituted arylene, a substituted or unsubstituted aralkylene, a substituted or unsubstituted alkarylene, and a substituted or unsubstituted heteroarylene, a substituted or unsubstituted heteroaralkylene, and a substituted or unsubstituted alkheteroarylene, $R_4$ may be substituted with 1 to 4, preferably with 1 substituent, selected from the group consisting of $C_1$-$C_6$ alkyl, such as methyl, ethyl, propyl, iso-propyl, butyl and t-butyl, halogen, such as chloride and bromide, hydroxy and thiol.

In a preferred embodiment $R_4$ in compound (D) of formula (II) is selected from the group consisting of $C_1$-$C_{12}$ alkylene, $C_6$-$C_{14}$ arylene, and $-[(CR_5R_6))_mO]_n[CR_7R_8]_o-$, wherein $R_5$, $R_6$, $R_7$ and $R_8$ are independently of each other selected from the group consisting of hydrogen, alkyl and aryl, m is 2 to 4; n is 0 to 20; and o is 0 to 20, wherein in case n is 0, o is not 0.

Preferably, $R_4$ in compound (D) of formula (II) is $-[(CR_5R_6)_mO]_n[CR_7R_8]_o-$, wherein $R_5$, $R_6$, $R_7$ and $R_8$ are independently of each other selected from the group consisting of hydrogen, $C_1$-$C_6$ alkyl and $C_6$-$C_{14}$ aryl, m is 1 or 2; n is 1 to 5; and o is 1 or 2, most preferred wherein $R_5$, $R_6$, $R_7$ and $R_8$ are independently of each other selected from the group consisting of hydrogen and $C_1$-$C_6$ alkyl, preferably selected from the group consisting of hydrogen, optionally substituted, preferably non-substituted methyl, ethyl, n-propyl, isopropyl, n-butyl, isobutyl, and tert-butyl, in particular hydrogen, m is 2, n is 1 and o is 2.

In a particular preferred embodiment, compound (D) used in step a) is

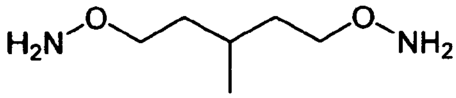

and/or

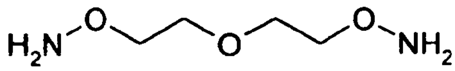

and/or

$H_2N-O-(CH_2CH_2O)_6$ $-NH_2$

or a salt thereof, in particular,

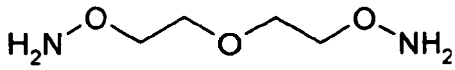

or a salt thereof.

The reaction in step a) is preferably conducted in that HAS is dissolved in an aqueous medium, preferably in a reaction buffer, and compound (D) is added. Preferred reaction buffers are, e.g., sodium citrate buffer, sodium acetate buffer, sodium phosphate buffer, sodium carbonate buffer, sodium borate buffer, water. Preferred pH values of the reaction buffers are in the range of from 2 to 9, more preferably of from 3 to 7, more preferably of from 4 to 6, and most preferably of about 5.

The molar ratio of compound (D) to HAS is preferably $\leq$ 200, more preferably $\leq$100 based on $M_n$ of the HAS derivative. Especially preferred molar ratios of compound (D) to HAS are in the range of from 90 to 10, more preferably from 80 to 30 and even more preferably from 70 to 50.

The reaction mixture is stirred at a temperature of about 0 °C to about 40°C, more preferred about 10 °C to about 30 °C, preferably at room temperature.

The reaction is preferably conducted for about 5 to about 30 h, more preferred from about 15 to about 25 h.

The hydroxyalkyl starch derivative obtained is preferably isolated from the reaction mixture by ultrafiltration or dialysis, preferably ultrafiltration followed by lyophilisation of the isolated hydroxyalkyl starch derivative. In an alternative embodiment the hydroxyalkyl starch derivative is precipitated form the reaction mixture, in particular by adding an alcohol, preferably 2-propanol or separated by ultrafiltration and/or lyophilisation. The obtained precipitate may be purified with conventional steps, in particular by centrifugation, dialysis, ultrafiltration and/or lyophilisation.

In an alternative embodiment step a) additionally comprises that the hydroxyalkyl starch derivative obtained is reduced prior to step b). In particular, according to this embodiment, the oxime linkage group-CH=N-O- obtained from the reaction of either the reducing end, preferably HES, or at least one aldehyde group obtained from the ring-opening oxidation reaction described above, preferably the reducing end, with group $NH_2$-O- of compound (D) is reduced to the group -$CH_2$-NH-O-. In this embodiment, the reduction may be carried out at a temperature of about 10°C to about 80 °C for about 5 h to about 24 h, such as over night, in the presence of a suitable reducing agent, such as sodium borohydride, sodium cyanoborohydride, sodium triacetoxy borohydride, organic borane complex compounds such as a 4-(dimethylamin)pyridine borane complex, N-ethyldiisopropylamine borane complex, N-ethylmorpholine borane complex, N-methylmorpholine borane complex, N-phenylmorpholine borane complex, lutidine borane complex, triethylamine borane complex, or trimethylamine borane complex, preferably $NaCNBH_3$ or $NaBH_4$.

In the alternative embodiment described above, the hydroxyalkyl starch derivative obtained in step a) is directly used in step b) without any further chemical reaction, preferably without a reduction.

<u>III. Step b)</u>

In step b) of the method of the invention, the hydroxyalkyl starch derivative obtained in step a) is reacted with a compound (L) comprising at least two functional groups $W_1$ and $W_2$ independently selected from an aldehyde group, a suitably protected aldehyde group, a keto group, and a suitably protected keto group.

The term "aldehyde group" as used in this context of the present invention also encompasses the aldehyde hydrate, namely -$CH(OH)_2$, and a hemiacetal group corresponding to the aldehyde group.

As possible "protected aldehyde group", suitable acetal groups may be mentioned by way of example. As possible "protected keto group", suitable ketal groups may be mentioned by way of example.

Therefore, according to a preferred embodiment of the present invention, compound (L) comprises at least two functional groups $W_1$ and $W_2$ independently selected from the group consisting of an aldehyde group, a hemiacetal group, $-CH(OH)_2$, an acetal group, a keto group, and a ketal group.

Even more preferably, compound (L) comprises at least two functional groups independently selected from the group consisting of a hemiacetal group,$-CH(OH)_2$, an acetal group, and a ketal group, and the group -C(O)-R, wherein R is selected from the group consisting of hydrogen, a saturated or unsaturated, cyclic or linear, branched or unbranched, substituted or unsubstituted alkyl and a substituted or unsubstituted aryl group.

In a preferred embodiment, in case group -C(O)-R is a keto group, the residue R is selected from the group consisting of $C_1$-$C_6$ alkyl and $C_6$-$C_{14}$ aryl, even more preferably selected from the group consisting of optionally substituted, preferably non-substituted methyl, ethyl, n-propyl, isopropyl, n-butyl, isobutyl, and tert-butyl.

In a preferred embodiment, compound (L) is reacted via at least one of the at least two functional groups $W_1$ and $W_2$ with at least one of the functional groups $H_2N$-O- of the hydroxyalkyl starch derivative obtained in step a) or a salt thereof.

As far as the acetal group or ketal group, denoted as "A" hereinunder, is concerned, no specific limitations exist. In the context of the present invention, the term "acetal group" also comprises sulphur acetals and nitrogen acetals, and the term "ketal group" also comprises also sulphur ketals and nitrogen ketals. According to a preferred embodiment of the present invention, group A is a residue according to formula (IIa)

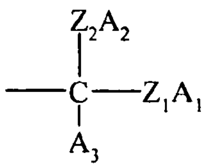

**(IIa)**

wherein

$Z_1$ and $Z_2$ are each independently O or S or $NR_x$, preferably O, wherein $R_x$ is H or lower alkyl such as methyl, ethyl, or propyl such as n-propyl or i-propyl, or C(O)-Ry wherein $R_y$ is preferably selected from the group consisting of $C_1$-$C_6$ alkyl and $C_6$-$C_{14}$ aryl, even more preferably selected from the group consisting of optionally substituted, preferably non-substituted methyl, ethyl, n-propyl, isopropyl, n-butyl, isobutyl, and tert-butyl;

$A_1$ and $A_2$ are each independently methyl, ethyl, n-propyl, isopropyl, n-butyl, tert-butyl, benzyl, 1,1,1-trichloroethyl, nitrobenzyl, methoxybenzyl, ethoxybenzyl, or are forming a ring according to formula (IIb)

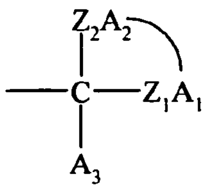

**(IIb)**

wherein $A_1$ and $A_2$, taken together, are optionally suitably substituted $-(CH_2)_2-$ or optionally suitably substituted $-(CH_2)_3-$ or optionally suitably substituted $-(CH_2CH(CH_3))-$, and

wherein $A_3$ is H or methyl, ethyl, n-propyl, isopropyl, n-butyl, isobutyl, pentyl, benzyl. Preferably, at least one of $Z_1$ and $Z_2$ is O, more preferably both $Z_1$ and $Z_2$ are O. As far as the residue $A_3$ is concerned, acetal groups are preferred according to the present invention, i.e. $A_3$ is preferably H.

If A is a ketal group, it is preferred that $A_3$ is methyl. Therefore, conceivable ketal groups A according to the present invention are, among others,

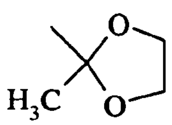

or

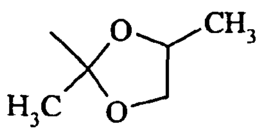

or

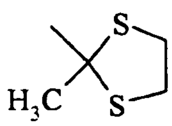

or

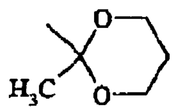 or 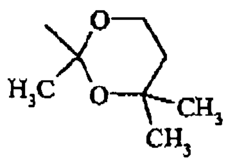 or 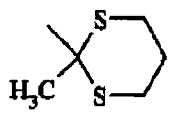

According to a preferred embodiment, $A_1$ and $A_2$ are each methyl or ethyl, even more preferably ethyl. Therefore, a particularly preferred acetal group A according to the present invention is $-CH(OCH_3)_2$ or $-CH(OC_2H_3)_2$, in Particular $-CH(OC_2H_5)_2$. According to a further embodiment wherein $A_1$ and $A_2$ are forming a ring according to formula (IIb), $A_1$ and $A_2$, taken together, are preferably $-(CH_3)_2-$. As far as this embodiment is concerned, particularly preferred acetal groups A according to the present invention are

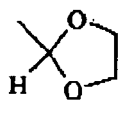 and 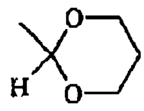

Further conceivable acetal groups may be, e.g.,

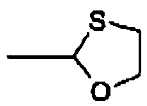 or 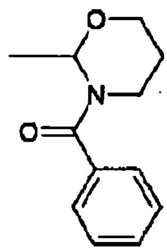 or 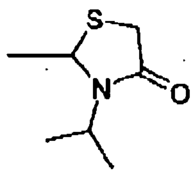

Compound (L) used in step b) is a bi-functional crass-linking compound. Such bi-functional cross-linking compound contains, apart from the two functional groups $W_1$ and $W_2$ independently selected from the group consisting of an aldehyde group, a suitably protected aldehyde group, a keto group, and a suitably protected keto group, no further functional group. Such further functional groups may be optionally suitably protected aldehyde and/or keto groups, but also other functional groups such as, in each case optionally suitably protected, amino groups, thio groups, carboxy groups, hydroxyl groups, halide groups, and the like.

Compound (L) used in step b) has the structure according to formula (III)

$$W_1\text{-}R_9\text{-}W_z \qquad \text{(III)}$$

wherein $R_9$ is selected from a chemical bond, preferably a single bond, a saturated or unsaturated, cyclic or linear, branched or unbranched, substituted or unsubstituted alkylene, possibly containing heteroatoms in the alkylene chain, a substituted or unsubstituted arylene and a substituted or unsubstituted heteroarylene, a substituted or unsubstituted aralkylene, a substituted or unsubstituted alkarylene, and a substituted or unsubstituted heteroarylene, a substituted or unsubstituted heteroaralkylene, and a substituted or unsubstituted alkheteroarylene,

either or both groups $W_1$ and $W_2$ are - as defined above - independently selected from the group consisting of an aldehyde group, a suitably protected aldehyde group, a keto group, and a suitably protected keto group, preferably from the group consisting of an aldehyde group, a hemiacetal group, $-CH(OH)_2$, an acetal group, a keto group, and a ketal group, and even more preferably from the group consisting of a hemiacetal group,$-CH(OH)_2$, an acetal group, and a ketal group, and the group -C(O)-R, wherein R is selected from the group consisting of hydrogen, a saturated or unsaturated, cyclic or linear, branched or unbranched, substituted or unsubstituted alkyl and a substituted or unsubstituted aryl group. Even more preferably, group R is selected from the group consisting of $C_1$-$C_6$ alkyl and $C_6$-$C_{14}$ aryl, even more preferably selected from the group consisting of optionally substituted, preferably non-substituted methyl, ethyl, n-propyl, isopropyl, n-butyl, isobutyl, and tert-butyl.

Preferably $R_9$ is a substituted or unsubstituted arylene or substituted or unsubstituted alkylene. In particular, $R_9$ is an unsubstituted $C_6$-$C_{14}$ arylene or $-CH_2)_n$, with n being preferably 1-6, most preferably phenylene. Preferably $R_9$

is unsubstituted. If $R_9$ is substituted, it may be substituted with 1 to 4, preferably with 1 substituent, selected from the group consisting of $C_1$-$C_6$ alkyl, such as methyl, ethyl, propyl, iso-propyl, butyl and t-butyl, halogen, such as chloride and bromide, hydroxy and thiol.

In a preferred embodiment compound (L) used in step b) is selected from benzene which is substituted in the 1,2-, 1,3 or 1,4 position with two functional groups $W_1$ and $W_2$ independently selected from the group consisting of -CH=O, hemiacetal group, acetal group, ketal group, $-CH(OH)_2$ and the group -C(O)-R, wherein R is selected from the group consisting of a saturated or unsaturated, cyclic or linear, branched or unbranched, substituted or unsubstituted alkyl and a substituted or unsubstituted aryl group, preferably from the group consisting of $C_1$-$C_6$ alkyl and $C_6$-$C_{14}$ aryl, even more preferably selected from the group consisting of hydrogen, optionally substituted, preferably non-substituted methyl, ethyl, n-propyl, isopropyl, n-butyl, isobutyl, and tert-butyl.

According to a preferred embodiment of the present invention, both functional groups $W_1$ and $W_2$ of compound (L) are -CH=O.

This embodiment of the present invention is particularly preferred if

- either the oxime linkage resulting from the reaction of group -CH=O of compound (L) with the group $-O-NH_2$ of the HAS derivative obtained according to step a)
- or the oxime linkage resulting from the reaction of compound (D) with the hydroxyalkyl starch, as described above, and the oxime linkage resulting from the reaction of group -CH=O of compound (L) with the group $-O-NH_2$ of the HAS derivative obtained according to step a),

is/are not reduced prior to the reaction of the HAS derivative obtained from step b) with a biologically active agent BA as described hereinunder in detail.

As far as this preferred embodiment of the present invention is concerned, preferred compounds (L) are, e.g.,

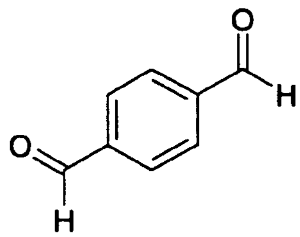
or
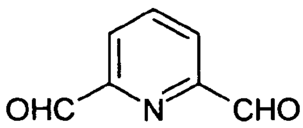
or
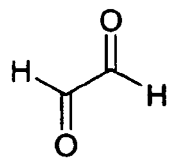

According to another preferred embodiment of the present invention, one functional group $W_1$ or $W_2$ of compound (L) is a suitably protected aldehyde group or keto group, preferably a suitably protected aldeyhde group, the other functional group preferably being an unprotected aldehyde group or keto group, more preferably an unprotected aldehyde group.

This embodiment of the present invention is particularly preferred if

- either the oxime linkage resulting from the reaction of group -CH=O of compound (L) with the group $-O-NH_2$ of the HAS derivative obtained according to step a)
- or the oxime linkage resulting from the reaction of compound (D) with the hydroxyalkyl starch, as described above, and the oxime linkage resulting from the reaction of group -CH=O of compound (L) with the group $-O-NH_2$ of the HAS derivative obtained according to step a),

is/are reduced prior to the reaction of the HAS derivative obtained from step b) with a biologically active agent BA as described hereinunder in detail. In particular, the functional group $W_1$ or $W_2$ of compound (L) is a protected aldehyde group or keto group, preferably a suitably protected aldehyde group, which is stable under the reaction conditions which are applied for reducing above-mentioned oxime linkage(s). Such reaction conditions under which a suitable protecting group should be stable are, e.g., reducing conditions at a pH in the range of from 3 to 8 in the presence of, e.g. $NaCNBH_3$ as reducing agent. Suitable protecting groups for functional group $W_1$ and $W_2$ are known to the person skilled in the art and, thus, can be chosen by the skilled person depending on the respective reducing conditions to be applied for the reduction of above-mentioned oxime linkage(s).

As far as this preferred embodiment of the present invention is concerned, conceivable compounds (L) are, e.g.,

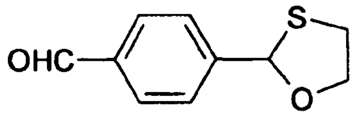

or

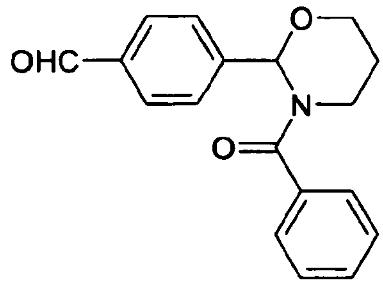

As described hereinunder, this embodiment of the present invention is especially chosen if the HAS derivative obtained from reaction of the derivative obtained from step b) with a biologically active agent BA - as described hereinunder in detail - shall contain two reduced oxime linkages, namely $-CH_2-NH-O-$, and, preferably, a methyleneamine linkage, namely $-CH_2-NH-$, between the HAS derivative obtained from step b) and BA.

The reaction in step b) is preferably conducted in a polar solvent, preferably DMF or a mixture of water/polar solvent, preferably water/DMF with an amount of polar solvent, preferably DMF of ≤ 50 % (v:v), more preferred ≤ 30 % (v:v).

The molar ratio of compound (L) to HAS derivative as obtained in step a) is preferably ≤ 200, more preferably ≤ 100, in particular ≤ 20, based $M_n$ of the HAS derivative. More preferably, the molar ratio of compound (L) to HAS derivative as obtained in step a) is in the range of from 70 to 1, more preferably from 40 to 2 and even more preferably from 10 to 5.

The reaction mixture is preferably stirred at a temperature of from 5 to 80 °C, more preferably of from 10 to 60 °C, more preferably of from 20 to 50°C, and even more preferably of from 30 to 50 °C, and even more preferably of from 35 to 45 °C such as about 35, 40, or 45 °C.

The reaction is preferably conducted for about 5 to about 30 h, more preferably from about 15 to about 25h.

The hydroxyalkyl starch derivative obtained is preferably isolated from the reaction mixture by ultrafiltration or dialysis, preferably ultrafiltration, followed by lyophilisation of the isolated hydroxyalkyl starch derivative. In an alternative embodiment the hydroxyalkyl starch derivative is precipitated form the reaction mixture, in particular by adding an alcohol and/or ketone, preferably a mixture of acetone and ethanol. The obtained precipitate may be purified with conventional steps, in particular by centrifugation, dialysis, ultrafiltration and/or lyophilisation.

In an alternative embodiment step b) additionally comprises that the hydroxyalkyl starch derivative obtained is reduced. In particular, according to this embodiment, the oxime linkage group -O-N=CH- obtained through the reaction of the $-O-NH_2$ group of compound (D) and group $W_1$ or $W_2$ of compound (L) is reduced to the group $-O-NH-CH_2-$ and/or, if applicable, group -CH=N-O- obtained in step a) by the reaction of the hydroxyalkylstarch and group $NH_2-O-$ of compound (D) is reduced to the group $-CH_2-NH-O-$. In this embodiment, the reduction may be carried out under at a temperature of 10 to 80 °C for 5 to 24 h, such as over night, in the presence of a suitable reducing agent, such as $NaBH(OAc)_3$, sodium borohydride, sodium cyanoborohydride, organic borane complex compounds such as a 4-(dimethylamin)pyridine borane complex, N-ethyldiisopropylamine borane complex, N-ethylmorpholine borane complex, N-methylmorpholine borane complex, N-phenylmorpholine borane complex, lutidine borane complex, triethylamine borane complex, or trimethylamine borane complex, preferably $NaCNBH_3$ or $NaBH_4$, possibly after an appropriate protection of the possibly present aldehyde group or keto group of compound (L) e.g. in the form of suitable protecting group stable under the reaction conditions applied for reducing above-mentioned oxime linkage group(s), e.g. in the form of a suitable acetal. As already described above, instead of protecting group $W_1$ or $W_2$ after step b), it is also possible to employ a compound (L) as starting material for step b) which already contains a suitably protected group $W_1$ or $W_2$ which is stable under the reaction conditions applied for reducing above-mentioned oxime linkage group(s).

In an alternative embodiment, the hydroxyalkylstarch derivative obtained in step b) can be directly used in step c), without any further chemical reaction, preferably without a reduction.

In a preferred embodiment, the hydroxyalkyl derivative obtained in step a) may be reacted in step b) with about 2 to about 70 equivalents based on $M_n$ of compound (L), in particular

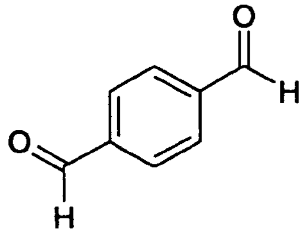

at room temperature or at about 40°C and subsequently room temperature, for a reaction time of about 10 to about 24 h, in a polar solvent, such as DMF or a mixture of DMF/water (10:90, v:v). The obtained HAS derivative may then be precipitated with a 1:1 mixture (v:v) of ethanol/acetone and the precipitate may then be dissolved in a polar solvent, such as DMF, and again precipitated. The precipitate may then be dissolved in water and ultrafiltrated and lyophilized. Preferably, the reaction mixture may be diluted with water 1:1 (v:v), the obtained precipitate may be filtered or centrifuged and again diluted with water 1:1 (v:v) and the obtained solution may then be ultrafiltrated and lyophilized.

Accordingly, the present invention relates to HAS derivatives having the following structures (in the following, it is assumed that the reaction of compound (L) with the HAS derivative obtained from step a) occurs via functional group $W_1$ of compound (L)):

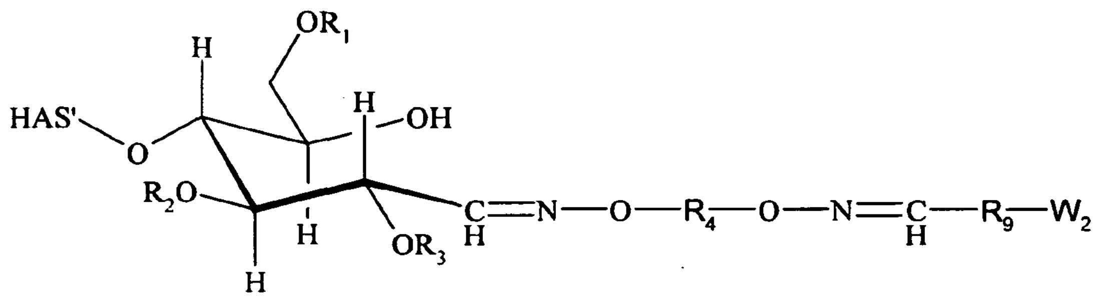

and/or the corresponding ring structure (in all following structure formulas containing a non-reduced oxime linkage between the reducing end of HAS or HES and compound (D), the open-ring structure shown above shall also encompass the corresponding ring structure shown below)

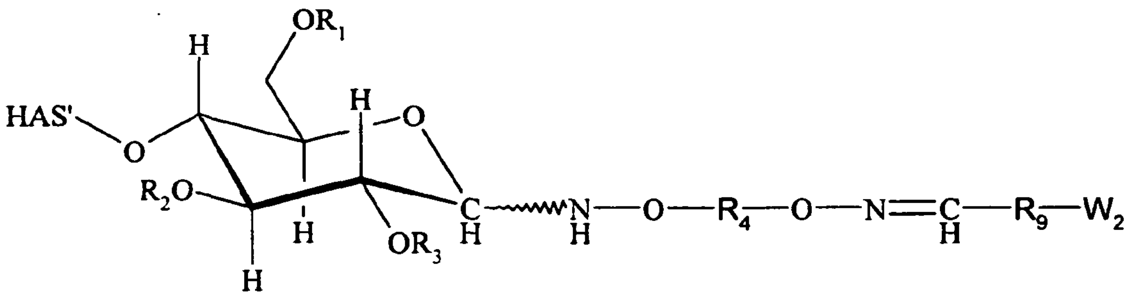

and/or

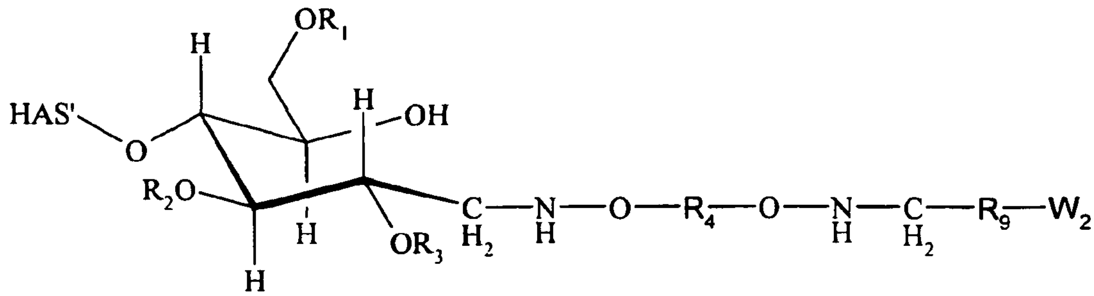

Depending on the reaction conditions and/or the specific chemical nature of the crosslinking compound, the C-N double bond may be present in E or Z conformation where also a mixture of both forms may be present having a certain equilibrium distribution; as far as the corresponding ring structure is concerned which for the purposes of the present invention shall be regarded as identical to the open structure above, and depending on the reaction conditions and/or the specific chemical nature of crosslinking compound, these HAS derivatives may be present with the N atom in equatorial or axial position where also a mixture of both forms may be present having a certain equilibrium distribution.

Therefore, according to a preferred embodiment, the present invention also relates to a HAS derivative, preferably a HES derivative, wherein, even more preferably, HES has a mean molecular weight from about 1 to about 1000 kDa, more preferably from about 1 to about 800 kDa, more preferably from about 1 to about 500 kDa, more preferably

from about 2 to about 400 kDa, more preferably from about 5 to about 300 kDa, more preferably from about 10 to about 200 kDa, in particular from about 50 to about 150 kDa, a molar substitution of 0.1 to 3, preferably 0.4 to 1.3, such as 0.4, 0.5, 0.6, 0.7 0.8, 0.9, 1.0, 1.1, 1.2, or 1.3, and a ratio of $C_2$ : $C_6$ substitution of preferably in the range of from 2 to 20, more preferably in the range of from 2 to 15 and even more preferably in the range of from 3 to 12:

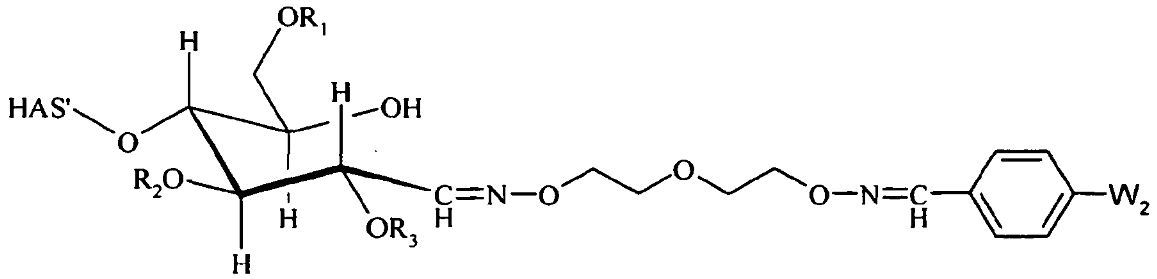

and/or

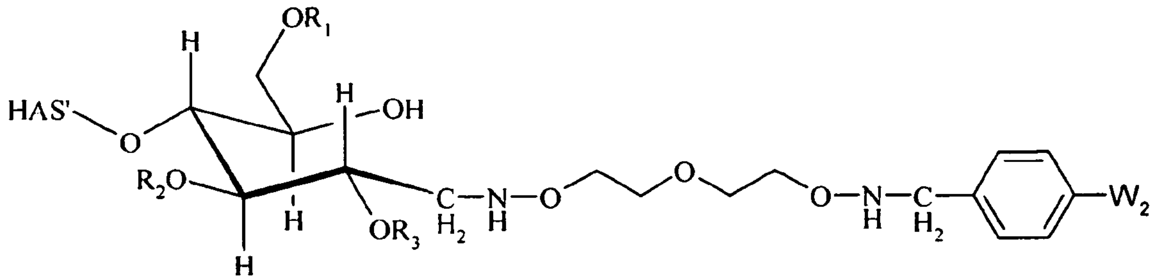

According to other preferred embodiments, the present invention also relates to the HAS derivatives, preferably HES derivatives according to the foregoing 2 structures wherein, instead of

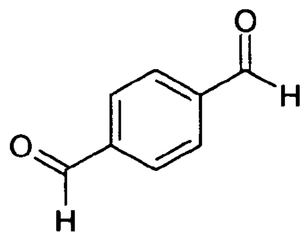

the compound

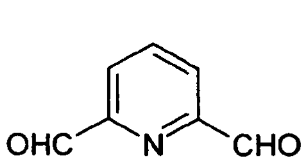

or

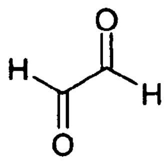

had been employed as compound (L).

According to a further embodiment, the present invention also relates to a HAS derivative, preferably a HES derivative, wherein, even more preferably, HES has a mean molecular weight from about 1 to about 1000 kDa, more preferably from about 1 to about 800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa, a molar substitution of 0.1 to 3, preferably 0.4 to 1.3, such as 0.4, 0.5, 0.6, 0.7 0.8, 0.9, 1.0, 1.1, 1.2, or 1.3, and a ratio of $C_2$ $C_6$ substitution of preferably in the range of from 2 to 20, more preferably in the range of from 2 to 15 and even more preferably in the range of from 3 to 12:

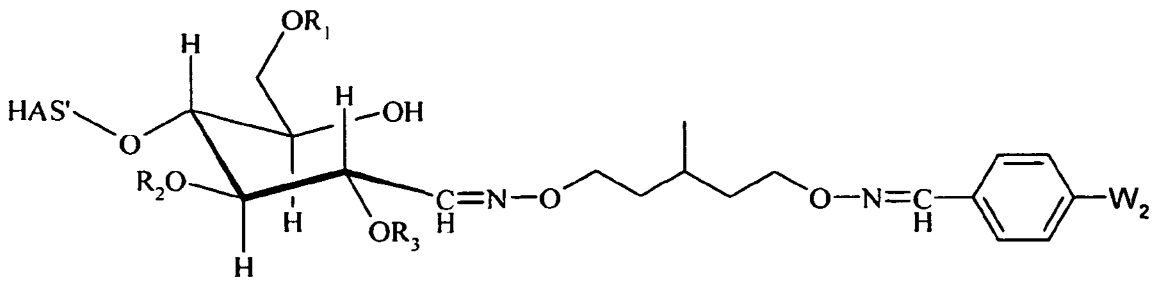

and/or

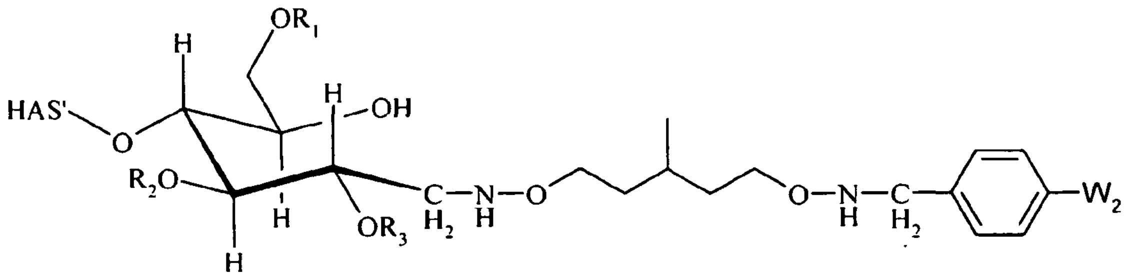

According to other preferred embodiments, the present invention also relates to the HAS derivatives, preferably HES derivatives according to the foregoing 2 structures wherein, instead of

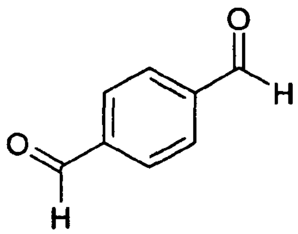

the compound

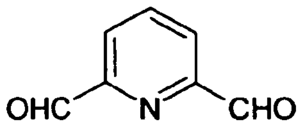
or
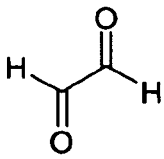

had been employed as compound (L).

According to a further embodiment, the present invention also relates to a HAS derivative, preferably a HES derivative, wherein, even more preferably, HES has a mean molecular weight from about 1 to about 1000 kDa, more preferably from about 1 to about 800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa, a molar substitution of 0.1 to 3, preferably 0.4 to 1.3, such as 0.4, 0.5, 0.6, 0.7 0.8, 0.9, 1.0, 1.1, 1.2, or 1.3, and a ratio of $C_2$: $C_6$ substitution of preferably in the range of from 2 to 20, more preferably in the range of from 2 to 15 and even more preferably in the range of from 3 to 12:

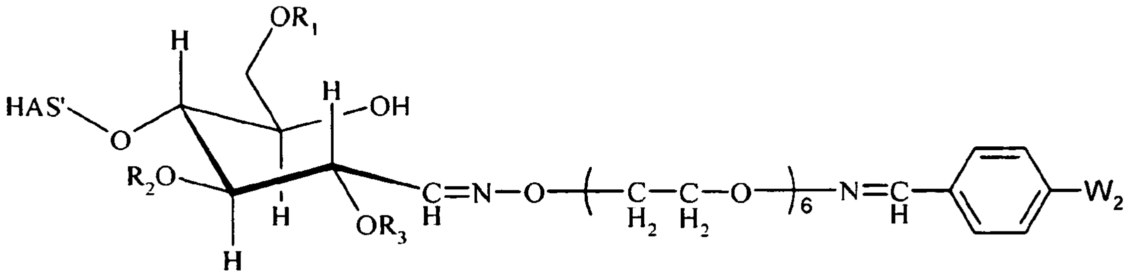

and/or

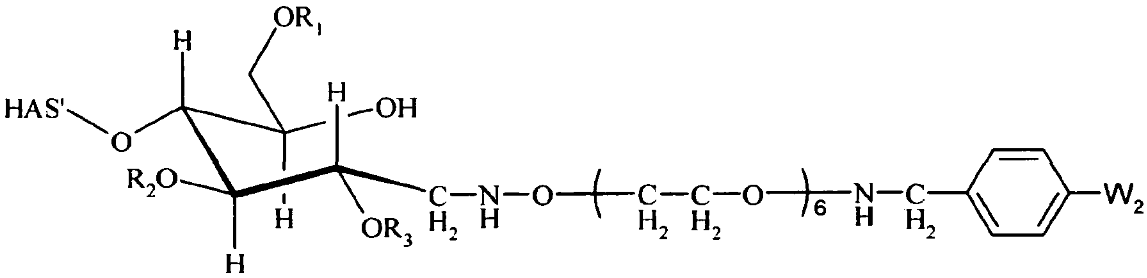

According to other preferred embodiments, the present invention also relates to the HAS derivatives, preferably HES derivatives according to the foregoing 2 structures wherein, instead of

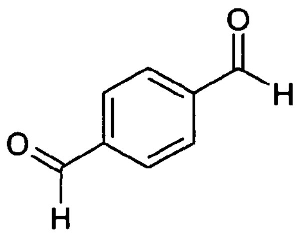

the compound

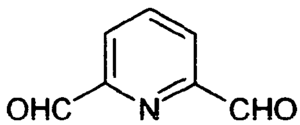

or

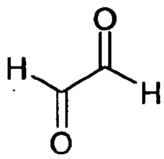

had been employed as compound (L).

In each of above-disclosed HAS derivatives comprising the terminal group $W_2$, $W_2$ is selected from the group consisting of an aldehyde group, a suitably protected aldehyde group, a keto group, and a suitably protected keto group, preferably from the group consisting of an aldehyde group, a hemiacetal group, $-CH(OH)_2$, an acetal group, a keto group, and a ketal group, and even more preferably from the group consisting of a hemiacetal group, $-CH(OH)_2$, an acetal group, a ketal group, and the group -C(O)-R, wherein R is selected from the group consisting of hydrogen, a saturated or unsaturated, cyclic or linear, branched or unbranched, substituted or unsubstituted alkyl and a substituted or unsubstituted aryl group. Even more preferably, in case the group -C(O)-R is a keto group, R is selected from the group consisting of $C_1$-$C_6$ alkyl and $C_6$-$C_{14}$ aryl, even more preferably selected from the group consisting of optionally substituted, preferably non-substituted methyl, ethyl, n-propyl, isopropyl, n-butyl, isobutyl, and tert-butyl.

According to one of the preferred embodiments of the present invention, according to which the oxime linkage (s) are not reduced prior to the reaction according to step c) as described hereinunder, group $W_2$ is preferably a non-protected aldehyde group or non-protected keto group, in particular a non-protected aldehyde group. Therefore, by way of example, the present invention also relates to a HAS derivative, preferably a HES derivative, wherein, even more preferably, HES has a mean molecular weight from about 1 to about 1000 kDa, more preferably from about 1 to about

800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa, a molar substitution of 0.1 to 3, preferably 0.4 to 1.3, such as 0.4, 0.5, 0.6, 0.7 0.8, 0.9, 1.0, 1.1, 1.2, or 1.3, and a ratio of $C_2$ $C_6$ substitution of preferably in the range of from 2 to 20, more preferably in the range of from 2 to 15 and even more preferably in the range of from 3 to 12:

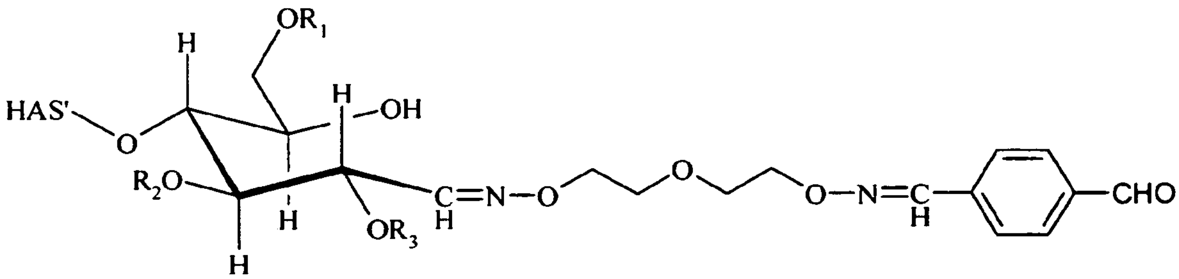

and/or

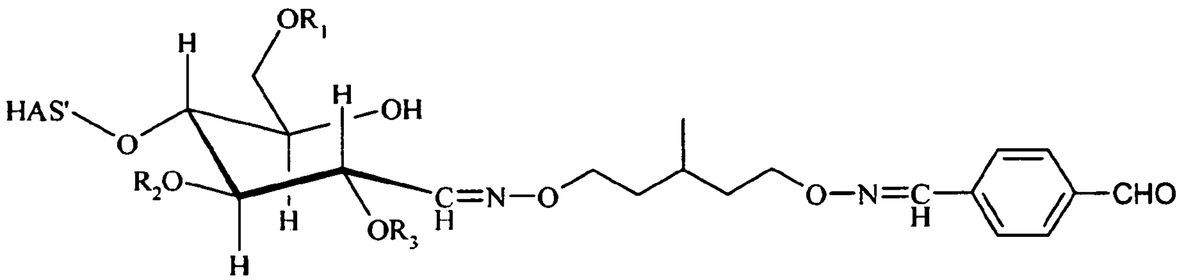

and/or

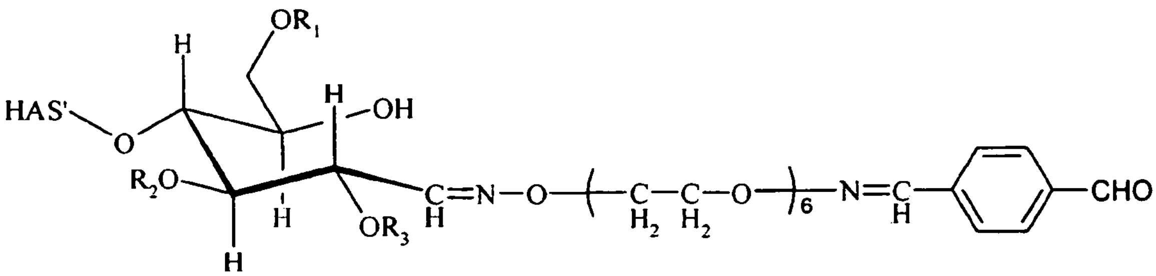

According to other preferred embodiments, the present invention also relates to the HAS derivatives, preferably HES derivatives according to the foregoing 3 structures wherein, instead of

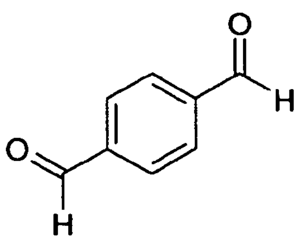

the compound

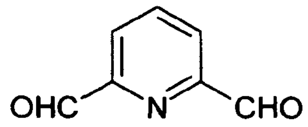

or

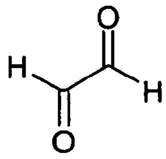

had been employed as compound (L).

According to another preferred embodiment of the present invention, according to which the oxime linkage(s) are reduced prior to the reaction according to step c) as described hereinunder, group $W_2$ is

- either, if present as unprotected aldehyde group or keto group prior to the reduction process, suitably protected before the reduction of the oxime linkages is carried out;
- already present as a suitably protected aldehyde group or suitably protected keto group in compound (L) employed as starting material in step b).

Therefore, as far as the HAS derivatives comprising reduced oxime linkages are concerned, and by way of example, the present invention also relates to a HAS derivative, preferably a HES derivative, wherein, even more preferably, HES has a mean molecular weight from about 1 to about 1000 kDa, more preferably from about 1 to about 800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa, a molar substitution of 0.1 to 3, preferably 0.4 to 1.3, such as 0.4, 0.5, 0.6, 0.7 0.8, 0.9, 1.0, 1.1, 1.2, or 1.3, and a ratio of $C_2$ $C_6$ substitution of preferably in the range of from 2 to 20, more preferably in the range of from 2 to 15 and even more preferably in the range of from 3 to 12:

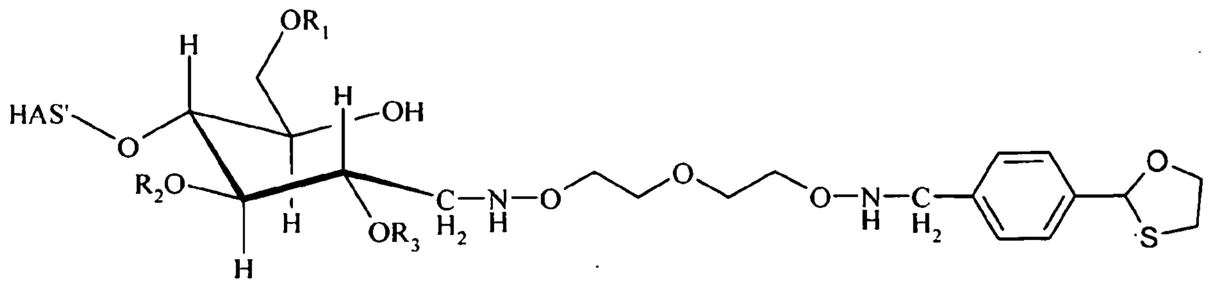

and/or

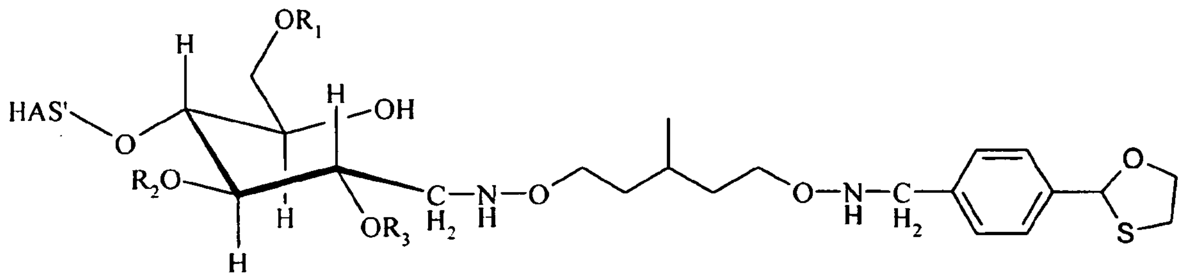

and/or

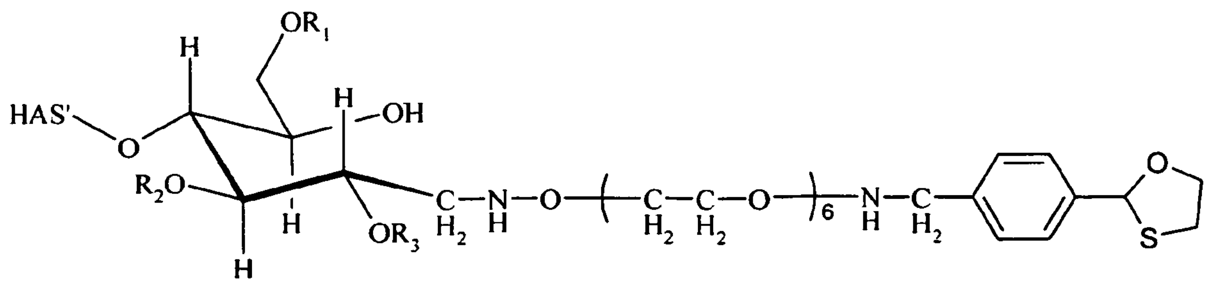

According to another conceivable embodiment concerning the three structures above, each exhibiting reduced oxime linkages, the terminal group $W_2$ may be, e.g.,

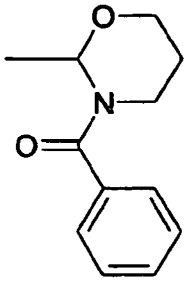

or

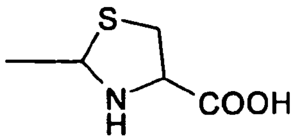

instead of

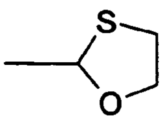

According to other preferred embodiments, the present invention also relates to the HAS derivatives, preferably HES derivatives according to the foregoing 3 structures wherein, instead of

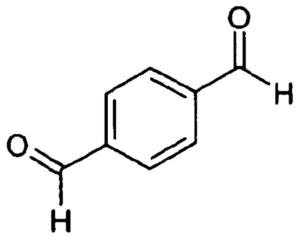

the compound

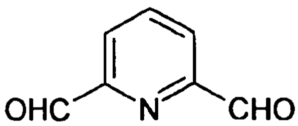

or

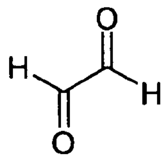

had been employed as compound (L).

IV. Step c)

In a preferred embodiment, the hydroxyalkyl starch derivative obtained in step b) is reacted in an additional step c) with at least one biologically active agent BA. The at least one biologically active agent BA used in step c) comprises at least one functional group $NH_2$. For such cases and for the purposes of the present invention, BA is also represented as $H_2N$-BA' wherein BA' is the remainder of BA.

According to one embodiment of the present invention, the HAS derivative obtained from step b) having a non-protected aldehyde group or non-protected keto group, preferably a non-protected aldehyde group as group $W_2$, is - optionally after suitable purification by methods such as, e.g., ultrafiltration, dialysis, and/or precipitation - reacted with BA in step c) of the present invention.

According to another embodiment of the present invention, the HAS derivative obtained from step b) having a protected aldehyde group or protected keto group, preferably a protected aldehyde group as group $W_2$, is - optionally after suitable purification by methods such as, e.g., ultrafiltration, dialysis, and/or precipitation - suitably subjected to a transformation of the protected group to the corresponding aldehyde or keto group wherein the resulting HAS derivative

is subjected to a suitable purification and/or isolation step prior to the reaction with BA. The transformation to the aldehyde or keto group is preferably performed by an acid-catalyzed hydrolysis reaction. The reaction is preferably carried out at a temperature of from 0 to 100 °C, more preferably from 10 to 80 °C and more preferably from 20 and 60 °C, at a pH which is preferably in the range of from 1 to 6, more preferably from 1 to 5, more preferably from 1 to 4, more preferably from 1 to 3 and even more preferably from 1 to less than 3. Purification and buffer-exchange of the hydrolysis reaction product can be achieved by methods well-known to those skilled in the art, e.g. by dialysis or ultrafiltration. The transformed material can be recovered from the solution as a solid e.g. by freeze-drying.

According to yet another embodiment of the present invention, the HAS derivative obtained from step b) which has been preferably purified is suitably subjected to a transformation of protected group $W_2$ to the corresponding aldehyde or keto group wherein the resulting HAS derivative is directly reacted with BA, i.e. without a separate suitable purification and/or isolation step of the HAS derivative comprising the aldehyde or keto group. The transformation to the aldehyde or keto group is preferably performed by an acid-catalyzed hydrolysis reaction. The reaction is preferably carried out at a temperature of from 0 to 100°C, more preferably from 10 to 80°C and more preferably from 20 and 60 °C, at a pH which is preferably in the range of from 1 to 6, more preferably from 1 to 5, more preferably from 1 to 4, more preferably from 1 to 3 and even more preferably from 1 to less than 3. The hydrolysis reaction product can be combined with the BA in a buffered solution either directly or after having adjusted the pH to a value compatible with the reaction with the BA.

According to yet another conceivable embodiment of the present invention, the HAS derivative obtained from step b) which has been preferably purified is directly reacted with BA, i.e. reacted with BA under reaction conditions allowing for the *in situ* transformation of the protected group to the corresponding aldehyde or keto group without a separate suitable purification and/or isolation step and without a separate step for the transformation of the protected group to the corresponding aldehyde or keto group.

As far as the biologically active substances (BA) of the present invention are concerned, these compounds may comprise one or more amino groups for coupling according to stage (ii) of the present invention. For cases where BA as such does not comprise an amino group suitable for this coupling, it is conceivable that at least one such amino group is introduced into BA by suitable functionalisation via methods known to the skilled person, prior to subjecting BA to (ii).

The term "biologically active substance" (BA) as used in the context of the present invention relates to a substance which can affect any physical or biochemical property of a biological organism including, but not limited to, viruses, bacteria, fungi, plants, animals, and humans. In particular, the term "biologically active substance" as used in the context of the present invention relates to a substance intended for diagnosis, cure, mitigation, treatment, or prevention of disease in humans or animals, or to otherwise enhance physical or mental well-being of humans or animals. Examples of active substances include, but are not limited to, peptides, polypeptides, proteins, enzymes, small molecule drugs, dyes, lipids, nucleosides, nucleotides, oligonucleotides, polynucleotides, nucleic acids, cells, viruses, liposomes, microparticles, and micelles. Preferably, a biologically substance according to the present invention contains a native amino group.

Examples of proteins include, but are not limited to, erythropoietin (EPO), such as recombinant human EPO (rhEPO) or an EPO mimetic, colony-stimulating factors (CSF), such as G-CSF like recombinant human G-CSF (rhG-CSF), alpha-Interferon (IFN alpha), beta-Interferon (IFN beta) or gamma-Interferon (IFN gamma), such as IFN alpha and IFN beta like recombinant human IFN alpha or IFN beta (rhIFN alpha or (rhIFN beta), interleukines, e.g. IL-1 to IL-34 such as IL-2 or IL-3 or IL-11 like recombinant human IL-2 or IL-3 (rhIL-2 or rhIL-3), serum proteins such as coagulation factors II-XIII like factor VIII, factor VII, factor IX, factor II, factor III, factor IV, factor V, factor VI, factor X, factor XI, factor XII, factor XIII, serine protease inhibitors such as alphal-antitrypsin (A1AT), activated protein C (APC), plasminogen activators such as tissue-type plasminogen activator (tPA), such as human tissue plasminogen activator (hTPA), AT III such as recombinant human AT III (rhAT III), myoglobin, albumin such as bovine serum albumin (BSA), growth factors, such as epidermal growth factor (EGF), thrombocyte growth factor (PDGF), fibroblast growth factor (FGF), brain-derived growth factor (BDGF), nerve growth factor (NGF), B-cell growth factor (BCGF), brain-derived neurotrophic growth factor (BDNF), ciliary neurotrophic factor (CNTF), transforming growth factors such as TGF alpha or TGF beta, BMP (bone morphogenic proteins), growth hormones such as human growth hormone (hGH), tumor necrosis factors such as TNF alpha or TNF beta, somatostatine, somatotropine, somatomedines, hemoglobin, hormones or prohormones such as insulin, gonadotropin, melanocyte-stimulating hormone (alpha-MSH), triptorelin, hypthalamic hormones such as antidiuretic hormones (ADH and oxytocin as well as releasing hormones and release-inhibiting hormones, parathyroid hormone, thyroid hormones such as thyroxine, thyotropin, thyroliberin, calcitonin, glucagon, glucagon-like peptides (GLP-1, GLP-2 etc.), exendines such as exendin-4, leptin, vasopressin, gastrin, secretin, integrins, glycoprotein hormones (e.g. LH, FSH etc.), melanoside-stimulating hormones, lipoproteins and apo-lipoproteins such as apo-B, apo-E, apo-$L_a$, immunoglobulins such as IgG, IgE, IgM, IgA, IgD and fragments thereof, hirudin, tissue-pathway inhibitor, plant proteins such as lectin or ricin, bee-venom, snake-venom, immunotoxins, antigen E, alpha-proteinase inhibitor, ragweed allergen, melanin, oligolysine proteins, RGD proteins or optionally corresponding receptors for one of these proteins; prolactin or a mutant thereof, such as G129R, in which the wild type amino acid at position 129, glycine, is replaced by arginine (a

tradename of this mutant is "LactoVert") and a functional derivative or fragment of any of these proteins or receptors, an antibody, or an antibody fragment, or an alternative protein scaffold. The term "alternative protein scaffold" as used in the context of the present invention relates to a molecule having binding abilities similar to a given antibody wherein the molecule is based on an alternative non-antibody protein framework. In this context, the articles by A. Skerra, T. Hey et al., and H.K. Binz (see list of references below) may be mentioned.

The active substance is preferably selected from the group composed of antibiotics, antidepressants, antidiabetics, antidiuretics, anticholinergics, antiarrhythmics, antiemetics, antitussives, antiepileptics, antihistamines, antimycotics, antisympathotonics, antithrombotics, androgens, antiandrogens, estrogens, antiestrogens, antiosteoporotics, antitumor agents, vasodilators, other antihypertensive agents, antipyretic agents, analgesics, antiinflammatory agents, beta blockers, immunosuppressants and vitamins.

Some additional, non-restrictive examples of active substances are alendronate, amikazin, atenolol, azathioprine, cimetidine, clonidine, cosyntropin, cycloserine, desmopressin, dihydroergotamine, dobutamine, dopamine, ε-aminocaproic acid, ergometrine, esmolol, famotidine, flecainide, folic acid, flucytosine, furosemide, ganciclovir, glucagon, hydrazaline, isoproterenol, ketamine, liothyronine, LHRH, merpatricin, methyldopa, metoprolol, neomicin, nimodipine, nystatin, oxytocin, phentolamine, phenylephrine, procainamide, procaine, propranolol, ritodrine, sotalol, terbutaline, thiamine, tiludronate, tolazoline, trimethoprim, tromethamine, vasopressin; amifostine, amiodarone, aminocaproic acid, aminohippurate sodium, aminoglutethimide, aminolevulinic acid, aminosalicylic acid, amsacrine, anagrelide, anastrozole, asparaginase, anthracyclines, bexarotene, bicalutamide, bleomycin, buserelin, busulfan, cabergoline, capecitabine, carboplatin, carmustine, chlorambucin, cilastatin sodium, cisplatin, cladribine, clodronate, cyclophosphamide, cyproterone, cytarabine, camptothecins, 13-cis retinoic acid, all trans retinoic acid; dacarbazine, dactinomycin, daunorubicin, deferoxamine, dexamethasone, diclofenac, diethylstilbestrol, docetaxel, doxorubicin, epirubicin, estramustine, etoposide, exemestane, fexofenadine, fludarabine, fludrocortisone, fluorouracil, fluoxymesterone, flutamide, gemcitabine, epinephrine, L-Dopa, hydroxyurea, idarubicin, ifosfamide, imatinib, irinotecan, itraconazole, goserelin, letrozole, leucovorin, levamisole, lisinopril, lovothyroxine sodium, lomustine, mechlorethamine, medroxyprogesterone, megestrol, melphalan, mercaptopurine, metaraminol bitartrate, methotrexate, metoclopramide, mexiletine, mitomycin, mitotane, mitoxantrone, naloxone, nicotine, nilutamide, octreotide, oxaliplatin, pamidronate, pentostatin, pilcamycin, porfimer, prednisone, procarbazine, prochlorperazine, ondansetron, raltitrexed, sirolimus, streptozocin, tacrolimus, tamoxifen, temozolomide, teniposide, testosterone, tetrahydrocannabinol, thalidomide, thioguanine, thiotepa, topotecan, tretinoin, valrubicin, vinblastine, vincristine, vindesine, vinorelbine, dolasetron, granisetron; forinoterol, fluticasone, leuprolide, midazolam, alprazolam, amphotericin B, podophylotoxins, nucleoside antivirals, aroyl hydrazones, sumatriptan; macrolides such as erythromycin, oleandomycin, troleandomycin, roxithromycin, clarithromycin, davercin, azithromycin, flurithromycin, dirithromycin, josamycin, spiromycin, midecamycin, leucomycin, miocamycin, rokitamycin, andazithromycin, and swinolide A; fluoroquinolones such as ciprofloxacin, ofloxacin, levofloxacin, trovafloxacin, alatrofloxacin, moxifloxicin, norfloxacin, enoxacin, grepafloxacin, gatifloxacin, lomefloxacin, sparfloxacin, temafloxacin, pefloxacin, amifloxacin, fleroxacin, tosufloxacin, prulifloxacin, irloxacin, pazufloxacin, clinafloxacin, and sitafloxacin; aminoglycosides such as gentamicin, netilmicin, paramecin, tobramycin, amikacin, kanamycin, neomycin, and streptomycin, vancomycin, teicoplanin, rampolanin, mideplanin, colistin, daptomycin, gramicidin, colistimethate; polymixins such as polymixin B, capreomycin, bacitracin, penems; penicillins including penicillinase-sensitive agents like penicillin G, penicillin V; penicillinase-resistant agents like methicillin, oxacillin, cloxacillin, dicloxacillin, floxacillin, nafcillin; gram negative microorganism active agents like ampicillin, amoxicillin, and hetacillin, cillin, and galampicillin; antipseudomonal penicillins like carbenicillin, ticarcillin, azlocillin, mezlocillin, and piperacillin; cephalosporins like cefpodoxime, cefprozil, ceftbuten, ceftizoxime, ceftriaxone, cephalothin, cephapirin, cephalexin, cephradrine, cefoxitin, cefamandole, cefazolin, cephaloridine, cefaclor, cefadroxil, cephaloglycin, cefuroxime, ceforamide, cefotaxime, cefatrizine, cephacetrile, cefepime, cefixime, cefonicid, cefoperazone, cefotetan, cefinetazole, ceftazidime, loracarbef, and moxalactam, monobactams like aztreonam; and carbapenems such as imipenem, meropenem, pentamidine isethiouate, albuterol sulfate, lidocaine, metaproterenol sulfate, beclomethasone diprepionate, triamcinolone acetamide, budesonide acetonide, fluticasone, ipratropium bromide, flunisolide, cromolyn sodium, and ergotamine tartrate; taxanes such as paclitaxel; SN-38, and tyrphostines.

Therefore, also chemical compounds known to the skilled person as "small molecules" are conceivable biologically active substances according to the present invention. The term "small molecule" as used in this context of the present invention relates to a biologically active chemical compound other than a protein and an oligonucleotide, including, however, peptides of up to 50 amino acids. Typical examples of such small molecules are listed in the foregoing paragraph.

Examples for an oligonucleotide are aptamers. Also to be mentioned are peptide nucleic acids (PNA) as conceivable biologically active substances.

In a particular preferred embodiment, the at least one biologically active agent BA used in step c) is selected from the group consisting of a peptide, polypeptide, a protein and a functional derivative, fragment or mimetic of the polypeptide or protein.

Preferably, the polypeptide is selected from the group consisting of erythropoietin (EPO), such as recombinant human EPO (rhEPO) or an EPO mimetic, colony-stimulating factors (CSF), such as G-CSF like recombinant human G-CSF (rhG-CSF), alpha-Interferon (IFN alpha), beta-Interferon (IFN beta) or gamma-Interferon (IFN gamma), such as IFN alpha and IFN beta like recombinant human IFN alpha or IFN beta (rhIFN alpha or rhIFN beta), interleukines, e.g. IL-1 to IL-18 such as IL-2 or IL-3 like recombinant human IL-2 or IL-3 (rhIL-2 or rhIL-3), serum proteins such as coagulation factors II-XIII like factor VIII, factor VII, factor IX, factor II, factor III, factor IV, factor V, factor VI, factor X, factor XI, factor XII, factor XIII, alphal-antitrypsin (A1AT), activated protein C (APC), plasminogen activators such as tissue-type plasminogen activator (tPA), such as human tissue plasminogen activator (hTPA), AT III such as recombinant human AT III (rhAT III), myoglobin, albumin such as bovine serum albumin (BSA), growth factors, such as epidermal growth factor (EGF), thrombocyte growth factor (PDGF), fibroblast growth factor (FGF), brain-derived growth factor (BDGF), nerve growth factor (NGF), B-cell growth factor (BCGF), brain-derived neurotrophic growth factor (BDNF), ciliary neurotrophic factor (CNTF), transforming growth factors such as TGF alpha or TGF beta, BMP (bone morphogenic proteins), growth hormones such as human growth hormone, tumor necrosis factors such as TNF alpha or TNF beta, somatostatine, somatotropine, somatomedines, hemoglobin, hormones or prohormones such as insulin, gonadotropin, melanocyte-stimulating hormone (alpha-MSH), triptorelin, hypthalamic hormones such as antidiuretic hormones (ADH and oxytocin as well as releasing hormones and release-inhibiting hormones, parathyroid hormone, thyroid hormones such as thyroxine, thyrotropin, thyroliberin, calcitonin, glucagon, glucagon-like peptides (GLP-1, GLP-2 etc.), exendines such as exendin-4, leptin, vasopressin, gastrin, secretin, integrins, glycoprotein hormones (e.g. LH, FSH etc.), melanoside-stimulating hormones, lipoproteins and apo-lipoproteins such as apo-B, apo-E, apo-$L_a$, immunoglobulins such as IgG, IgE, IgM, IgA, IgD and fragments thereof, hirudin, tissue-pathway inhibitor, plant proteins such as lectin or ricin, bee-venom, snake-venom, immunotoxins, antigen E, alpha-proteinase inhibitor, ragweed allergen, melanin, oligolysine proteins, RGD proteins or optionally corresponding receptors for one of these proteins; and a functional derivative or fragment of any of these proteins or receptors.

The polypeptide is even more preferably selected from the group consisting of erythropoietin (EPO), such as recombinant human EPO (rhEPO), colony-stimulating factors (CSF), such as G-CSF like recombinant human G-CSF (rhG-CSF), alpha-1-antitrypsin (A1AT), factor IX, alpha-Interferon (IFN alpha), beta-Interferon (IFN beta), and gamma-Interferon (IFN gamma), such as recombinant human IFN alpha or IFN beta (rhIFN alpha or rhIFN beta), in particular A1AT, factor IX, IFN alpha, G-CSF, and EPO. In a preferred embodiment, the hydroxyalkyl starch derivative obtained in step b) is reacted in step c) with an amino group of the at least one biologically active agent via functional group $W_2$ selected from the group consisting of an aldehyde group or a keto group, preferably selected from the group consisting of-CH=O, hemiacetal group, $-CH(OH)_2$ and the group -C(O)-R, wherein R is selected from the group consisting of a saturated or unsaturated, cyclic or linear, branched or unbranched, substituted or unsubstituted alkyl and a substituted or unsubstituted aryl group, introduced into the hydroxyalkyl starch derivative through compound (L) in step b). In a preferred embodiment, in case group -C(O)-R is a keto group, the residue R is selected from the group consisting of $C_1$-$C_6$ alkyl and $C_6$-$C_{14}$ aryl, even more preferably selected from the group consisting of optionally substituted, preferably non-substituted methyl, ethyl, n-propyl, isopropyl, n-butyl, isobutyl, and tert-butyl.

In one embodiment, the hydroxyalkyl starch derivative obtained in step b) is reacted in step c) via the at least one functional group $-NH_2$ of the biologically active agent with functional group W2 from the group consisting of an aldehyde group or a keto group, preferably selected from the group consisting of -CH=O, hemiacetal group, $-CH(OH)_2$ and the group -C(O)-R, introduced into the hydroxyalkyl starch derivative through compound (L) in step b), wherein the at least one functional group $-NH_2$ comprised in the at least one biologically active agent used in step c) is the N-terminal amino group of a polypeptide or a protein.

In a preferred embodiment, step c) is performed under the reaction conditions for a reductive amination. The reaction conditions for reductive amination are known to the person skilled in the art. In particular, under these conditions the group -CH=N- obtained through the reaction of functional group $W_2$ selected from the group consisting of -CH=O, hemiacetal group,$-CH(OH)_2$ and the group -C(O)-R and the $NH_2$-group of "biologically active agent BA is reduced to $-CH_2$-NH-.

In an alternative embodiment, neither the hydroxyalkyl starch derivative obtained in step a) nor the hydroxyalkyl starch derivative obtained in step b) is reduced after the respective steps, and only the reaction in step c) is conducted under reducing conditions, more preferred, wherein step c) is conducted with a suitable reducing agent, such as NaBH $(OAc)_3$, sodium borohydride, sodium cyanoborohydride, organic borane complex compounds such as a 4-(dimethylamin) pyridine borane complex, N-ethyldiisopropylamine borane complex, N-ethylmorpholine borane complex, N-methylmorpholine borane complex, N-phenylmorpholine borane complex, lutidine borane complex, triethylamine borane complex, or trimethylamine borane complex, preferably $NaCNBH_3$ or $NaBH_4$. Therefore, the present invention also relates to a HAS derivative, preferably a HES derivative, wherein, even more preferably, HES has a mean molecular weight from about I to about 1000 kDa, more preferably from about 1 to about 800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa, a molar substitution of 0.1 to 3, preferably 0.4 to 1.3, such as 0.4, 0.5, 0.6, 0.7 0.8, 0.9, 1.0, 1.1, 1.2, or 1.3, and a ratio of $C_2$ : $C_6$ substitution of preferably in the range of from 2 to 20, more preferably in the range of from 2 to 15 and even more preferably in the range of from 3 to 12:

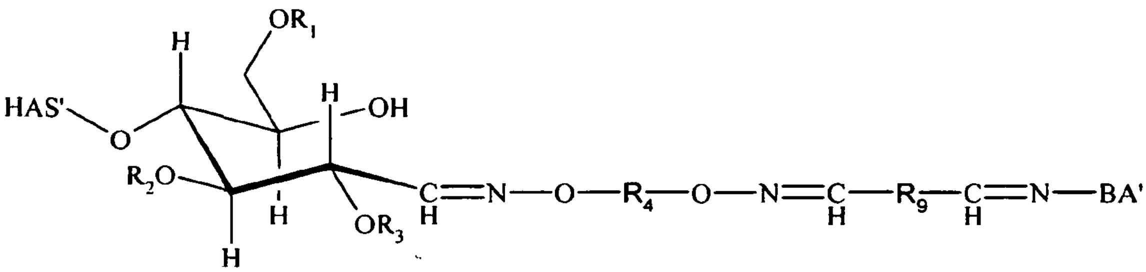

and/or

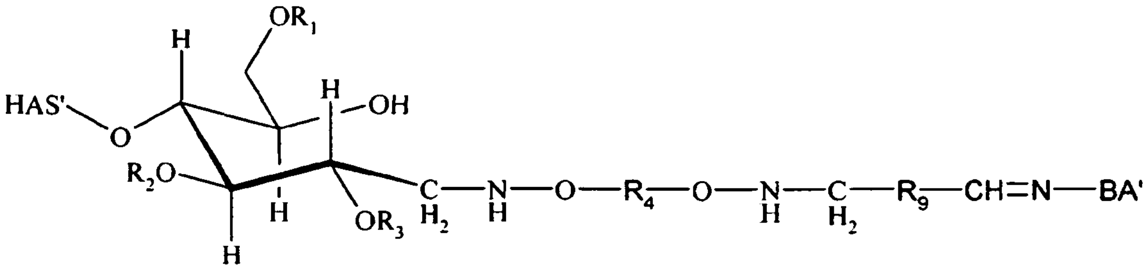

and/or

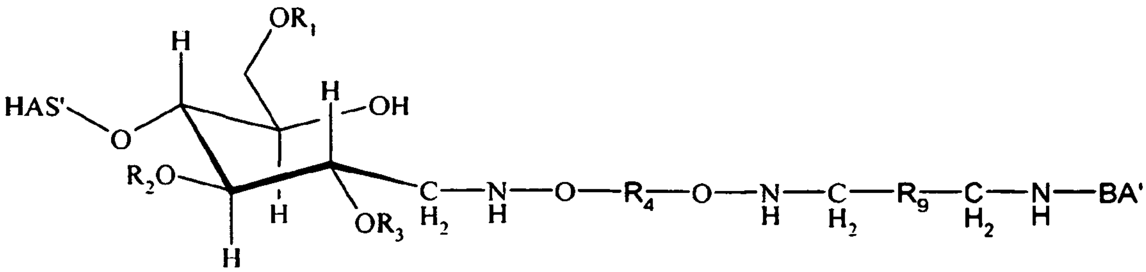

and/or

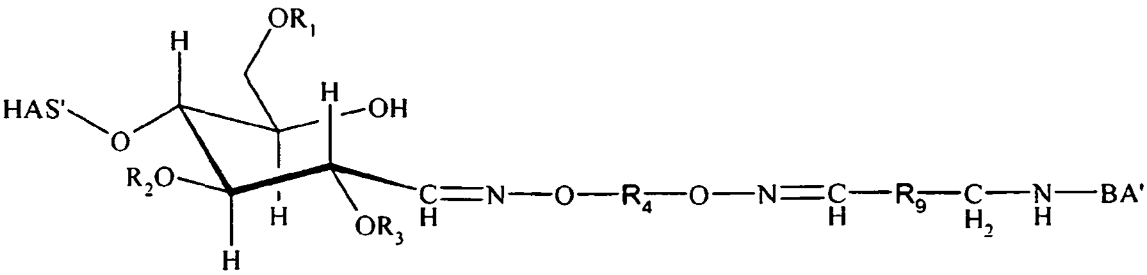

Preferably, among others, the present invention relates to a HAS derivative, preferably a HES derivative, wherein, even more preferably, HES has a mean molecular weight from about 1 to about 1000 kDa, more preferably from about 1 to about 800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa, a molar substitution of 0.1 to 3, preferably 0.4 to 1.3, such as 0.4, 0.5, 0.6, 0.7 0.8, 0.9, 1.0, 1.1, 1.2, or 1.3, and a ratio of $C_2$ : $C_6$ substitution of preferably in the range of from 2 to 20, more preferably in the range of from 2 to 15 and even more preferably in the range of from 3 to 12.

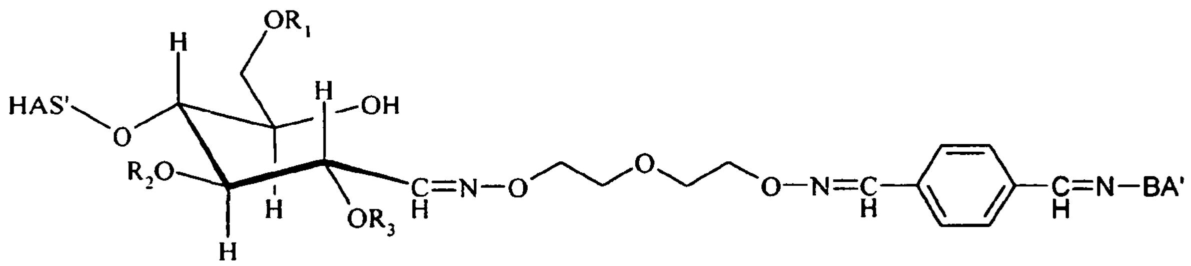

and/or

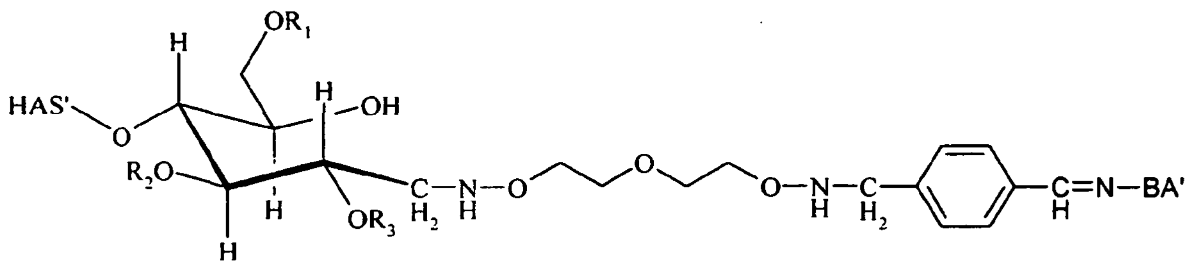

and/or

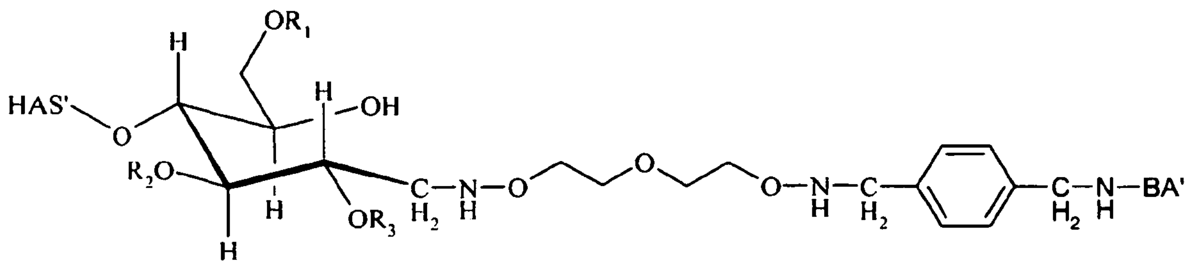

and/or

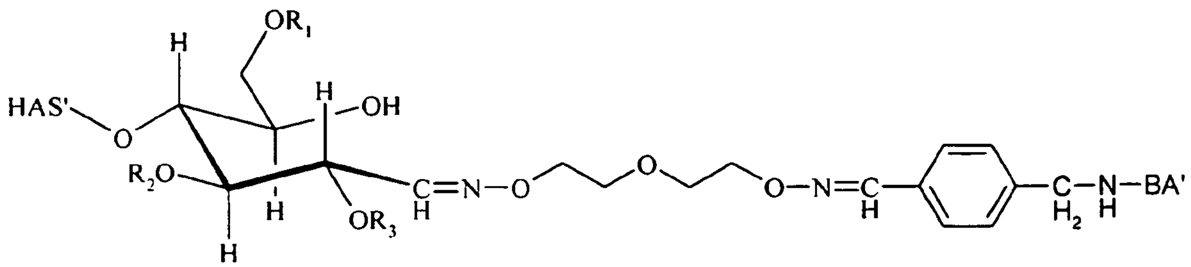

According to other preferred embodiments, the present invention also relates to the HAS derivatives, preferably HES derivatives according to the foregoing 4 structures wherein, instead of

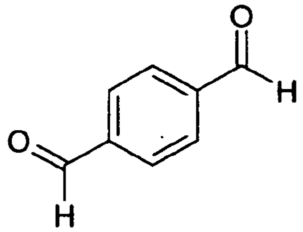

the compound

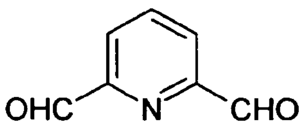

or

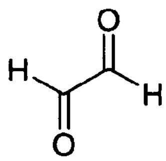

had been employed as compound (L).

According to a further embodiment, the present invention also relates to a HAS derivative, preferably a HES derivative, wherein, even more preferably, HES has a mean molecular weight from about 1 to about 1000 kDa, more preferably from about 1 to about 800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa, a molar substitution of 0.1 to 3, preferably 0.4 to 1.3, such as 0.4, 0.5, 0.6, 0.7 0.8, 0.9, 1.0, 1.1, 1.2, or 1.3, and a ratio of $C_2$ : $C_6$ substitution of preferably in the range of from 2 to 20, more preferably in the range of from 2 to 15 and even more preferably in the range of from 3 to 12:

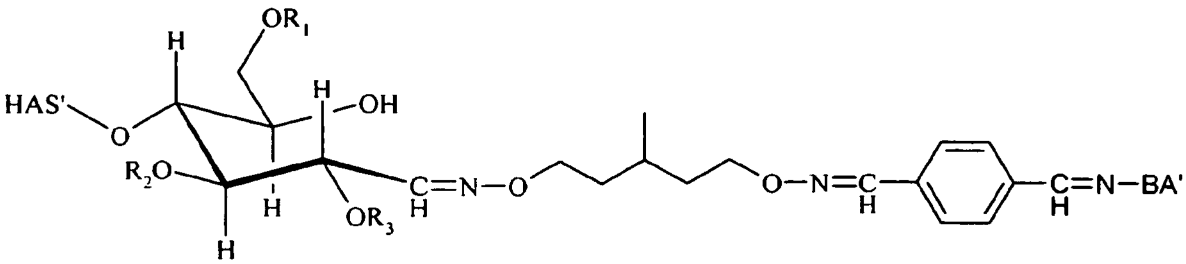

and/or

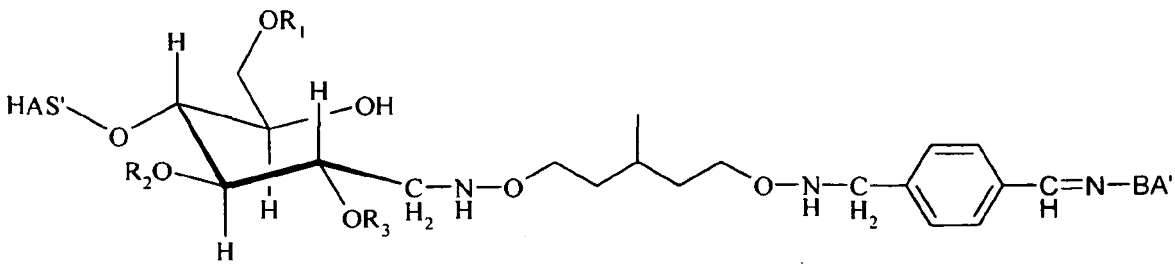

and/or

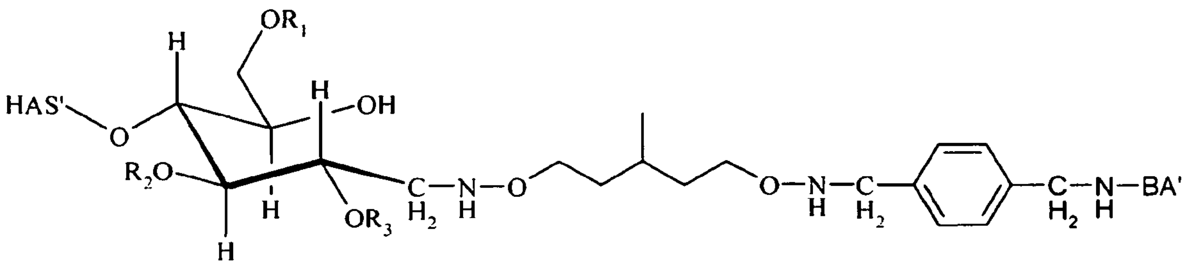

and/or

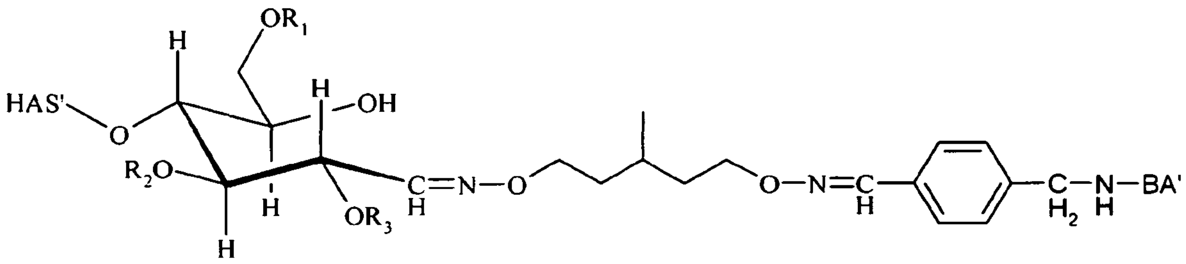

According to other preferred embodiments, the present invention also relates to the HAS derivatives, preferably HES derivatives according to the foregoing 4 structures wherein, instead of

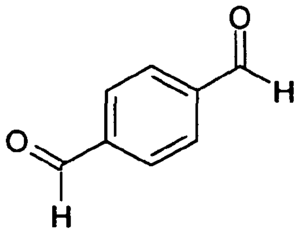

the compound

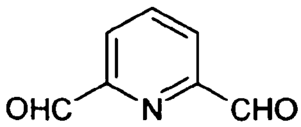 or 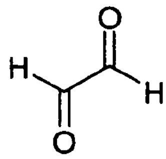

had been employed as compound (L).

According to a further embodiment, the present invention also relates to a HAS derivative, preferably a HES derivative, wherein, even more preferably, HES has a mean molecular weight from about 1 to about 1000 kDa, more preferably from about 1 to about 800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa, a molar substitution of 0.1 to 3, preferably 0.4 to 1.3, such as 0.4, 0.5, 0.6, 0.7 0.8, 0.9, 1.0, 1.1, 1.2, or 1.3, and a ratio of $C_2 : C_6$ substitution of preferably in the range of from 2 to 20, more preferably in the range of from 2 to 15 and even more preferably in the range of from 3 to 12:

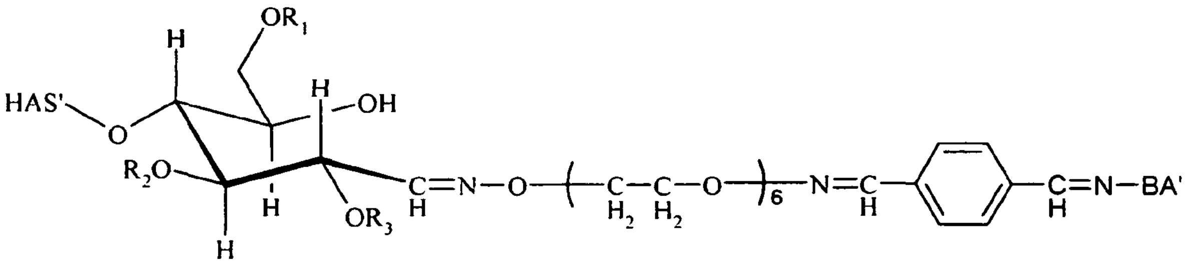

and/or

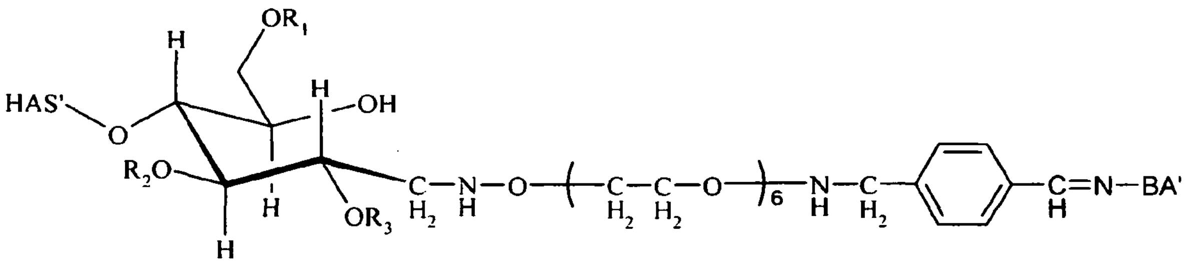

and/or

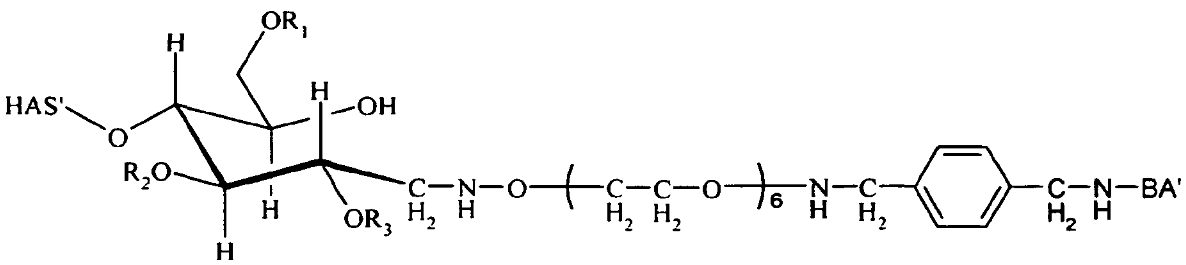

and/or

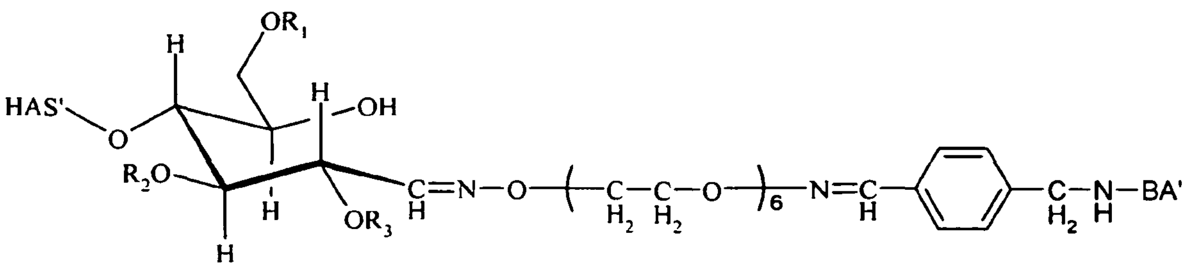

According to other preferred embodiments, the present invention also relates to the HAS derivatives, preferably HES derivatives according to the foregoing 4 structures wherein, instead of

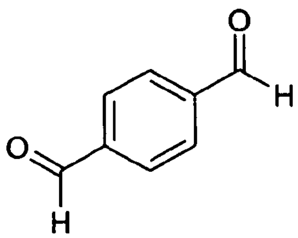

the compound

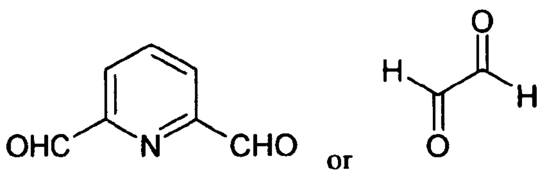

had been employed as compound (L).

In each of above-identified HAS derivatives, preferably HES derivatives containing BA', BA' is preferably selected from the group consisting of IFN-alpha', G-CSF', EPO', A1AT', and factor IX', in particular IFN-alpha'.

Thus, the present invention, according to a particularly preferred embodiment, relates to a HAS derivative, preferably a HES derivative, wherein, even more preferably, HES has a mean molecular weight from about 1 to about 1000 kDa, more preferably from about 1 to about 800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa, a molar substitution of 0.1 to 3, preferably 0.4 to 1.3, such as 0.4, 0.5, 0.6, 0.7 0.8, 0.9, 1.0, 1.1, 1.2, or 1.3, and a ratio of $C_2$ : $C_6$ substitution of preferably in the range of from 2 to 20, more preferably in the range of from 2 to 15 and even more preferably in the range of from 3 to 12:

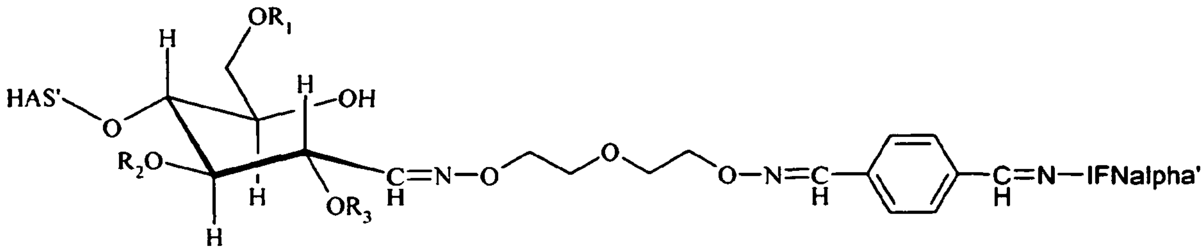

and/or

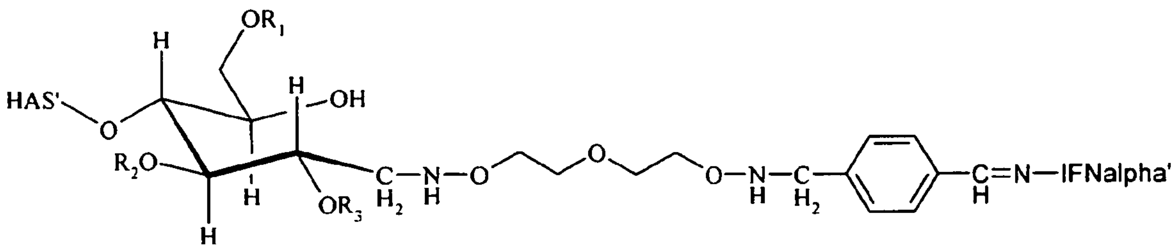

and/or

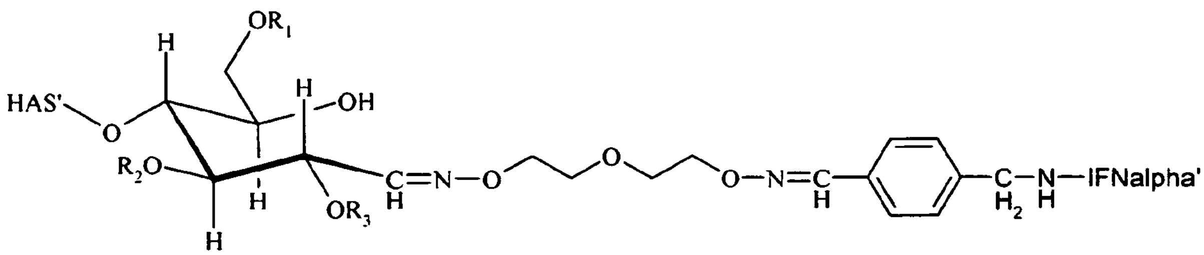

in particular

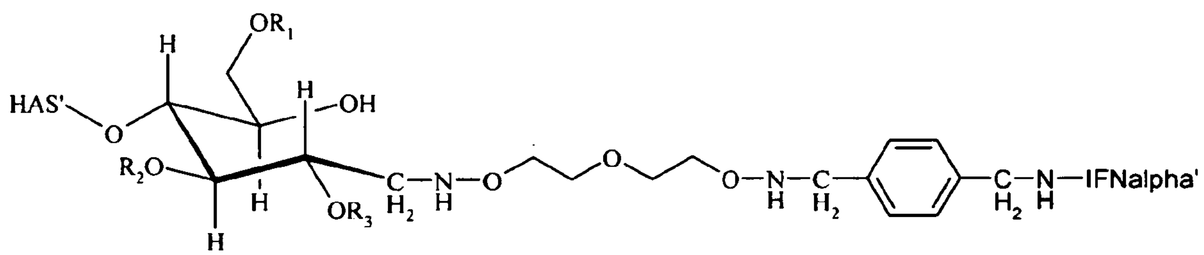

In a preferred embodiment, the biologically active agent BA, preferably selected from the group consisting of IFN-alpha, GCSF, EPO, A1AT, and factor IX, in particular IFN-alpha, is dissolved in an aqueous medium, preferably a buffer, in particular a sodium acetate buffer. The aqueous medium may contain additionally additives, such as detergents and/or dispersants, in particular selected from the group consisting of SDS, Chaps, Tween 20, Tween 80, Nonidet P-40 and Triton X 100. If a detergent and/or a dispersant is used, it is preferably present in an amount of about 0.05 to about 3 % by weight, preferably about 0.5 % by weight.

The aqueous solution of biologically active agent BA preferably has a pH value of about 3 to about 9, in particular of about 4 to about 7.

In one embodiment, the hydroxyalkyl starch derivative obtained in step b) is added to the aqueous solution of biologically active agent BA as described above. Preferably, subsequently a suitable reducing agent, such as $NaBH(OAc)_3$, sodium borohydride, sodium cyanoborohydride, organic borane complex compounds such as a 4-(dimethylamin)pyridine borane complex, N-ethyldiisopropylamine borane complex, N-ethylmorpholine borane complex, N-methylmorpholine borane complex, N-phenylmorpholine borane complex, lutidine borane complex, triethylamine borane complex, or trimethylamine borane complex, preferably $NaCNBH_3$ or $NaBH_4$, is added.

In an alternative embodiment, the hydroxyalkyl starch derivative obtained in step b) may be brought into an optionally aqueous solution and then biologically active agent BA, preferably a protein, is added. Preferably, subsequently a suitable reducing agent, such as $NaBH(OAc)_3$, sodium borohydride, sodium cyanoborohydride, organic borane complex compounds such as a 4-(dimethylamin)pyridine borane complex, N-ethyldiisopropylamine borane complex, N-ethylmorpholine borane complex, N-methylmorpholine borane complex, N-phenylmorpholine borane complex, lutidine borane complex, triethylamine borane complex, or trimethylamine borane complex, preferably $NaCNBH_3$ or $NaBH_4$, is added.

The reaction of the hydroxyalkyl starch derivative obtained in step b) and the biologically active agent BA, preferably selected from the group consisting of IFN alpha, G-CSF, EPO, factor IX, and A1AT, in particular IFN-alpha, in step c) is preferably carried out at a pH value of about 3 to about 7, in particular of about 4 to about 6.

The reaction of the hydroxyalkyl starch derivative obtained in step b) and the biologically active agent BA, preferably selected from the group consisting of IFN alpha, G-CSF, EPO, A1AT, and factor IX, in particular IFN alpha, in step c) is preferably carried out at a temperature of about 0 to about 25 °C, in particular at about 0 °C or at about 5 °C or at about 10 °C or at about 21 °C.

The reaction time in step c) depends on the temperature, the ratio of HAS, in particular HES, derivative obtained in step b) and biologically active agent BA and the absolute concentration of the HAS derivative and biologically active agent BA.

The molar ratio of hydroxyalkyl starch derivative obtained in step b) to biologically active agent BA in step c) is generally from about 0.1:1 to 200:1, preferably from about 1:1 to about 100:1 equivalents, based on the weight average molecular weight ($M_w$) of the hydroxyalkyl starch derivative obtained in step b), preferably the molar ratio is from about 1:1 to about 40:1, in particular the molar ratio is from about 1:1 to about 20:1, preferably in case the biologically active agent is selected from the group consisting of IFN alpha, G-CSF, EPO, A1AT, and factor IX, in particular IFN-alpha.

In a particular preferred embodiment the concentration of the hydroxyalkyl starch derivative obtained in step b) used in step c) is higher than about 10 % m/v, in particular higher than about 15 % m/v.

In a preferred embodiment, the concentration of biologically active agent BA, in particular selected from the group consisting of IFN alpha, G-CSF, EPO, A1AT, and factor IX, in particular IFN-alpha, is higher than about 1 mg/mL, preferably higher than about 2 mg/mL, in particular higher than about 4 mg/mL.

The reaction product obtained in step c) can be isolated with conventional means, such as liquid chromatography, e.g. ion exchange chromatography, SEC, HPLC or any other means. The reaction may be stopped before purification by dilution, reduction of the reaction temperature, addition of primary amino compounds, e.g. amino acids or a combination of these methods.

In one of the particular preferred embodiments, in step a), hydroxyalkyl starch, preferably hydroxyethyl starch, which is not oxidized, is reacted at a reducing end of the hydroxyalkyl starch with compound (D) being

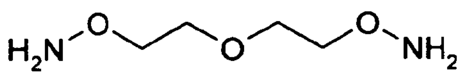

via one of the functional groups $-O-NH_2$, and in step b) the hydroxyalkyl starch derivative obtained in step a) is reacted via the functional group $-O-NH_2$ with one of the functional groups $-CH=O$ of compound (L) being

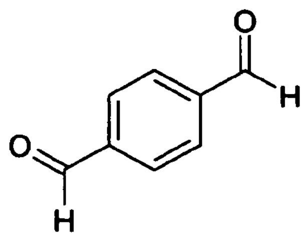

The hydroxyalkyl starch derivative obtained in step b) above is preferably reacted in step c) with a biologically

active agent BA selected from the group consisting of IFN alpha, G-CSF, EPO, factor IX, and A1AT, in particular IFN-alpha, wherein the hydroxyalkyl starch derivative obtained in step b) is reacted with IFN alpha in step c) under conditions for reductive amination, in particular in the presence of a suitable reducing agent, such as $NaBH(OAc)_3$, sodium borohydride, sodium cyanoborohydride, organic borane complex compounds such as a 4-(dimethylamin)pyridine borane complex, N-ethyldiisopropylamine borane complex, N-ethylmorpholine borane complex, N-methylmorpholine borane complex, N-phenylmorpholine borane complex, lutidine borane complex, triethylamine borane complex, or trimethylamine borane complex, preferably $NaCNBH_3$ or $NaBH_4$, is added.

It has surprisingly been found that when conducting the above described method, in particular under the preferred conditions for step c) as described above, the reaction product of the hydroxyalkyl starch derivative as obtained in step b) with a biologically active agent BA can be obtained in a high yield such as yields of up to 80 %, like in the range of from 70 to 80 %.

V. Pharmaceutical Composition and Use

The invention further relates to a pharmaceutical composition comprising, in a therapeutically effective amount, a hydroxyalkyl starch derivative obtainable by the above described method, in particular when comprising steps a) to step c), preferably, wherein biologically active agent BA is preferably selected from the group consisting of IFN-alpha, G-CSF, EPO, in particular IFN-alpha. The pharmaceutical composition can contain usual pharmaceutical excipients.

A "therapeutically effective amount" as used herein refers to that amount which provides therapeutic effect for a given condition and administration regimen.

As far as the pharmaceutical compositions according to the present invention comprising the HAS derivative comprising BA', as described above, are concerned, the HAS derivatives may be used in combination with a pharmaceutical excipient. Generally, the HAS derivative will be in a solid form which can be combined with a suitable pharmaceutical excipient that can be in either solid or liquid form. As excipients, carbohydrates, inorganic salts, antimicrobial agents, antioxidants, surfactants, buffers, acids, bases, and combinations thereof may be mentioned. A carbohydrate such as a sugar, a derivatized sugar such as an alditol, aldonic acid, an esterified sugar, and/or a sugar polymer may be present as an excipient. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol, sorbitol (glucitol), pyranosyl sorbitol, myoinositol, and the like. The excipient may also include an inorganic salt or buffer such as citric acid, sodium chloride, potassium chloride, sodium sulfate, potassium nitrate, sodium phosphate monobasic, sodium phosphate dibasic, and combinations thereof. The pharmaceutical composition according to the present invention may also comprise an antimicrobial agent for preventing or deterring microbial growth, such as, e.g., benzalkonium chloride, benzethonium chloride, benzyl alcohol, cetylpyridinium chloride, chlorobutanol, phenol, phenylethyl alcohol, phenylmercuric nitrate, thimersol, and combinations thereof. The pharmaceutical composition according to the present invention may also comprise an antioxidant. such as, e.g., ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof. The pharmaceutical composition according to the present invention may also comprise a surfactant may such as, e.g., polysorbates, or pluronics sorbitan esters; lipids, such as phospholipids such as lecithin and other phosphatidylcholines, phosphatidylethanolamines acids and fatty esters; steroids, such as cholesterol; and chelating agents, such as EDTA or zinc. The pharmaceutical composition according to the present invention may also comprise acids or bases such as, e.g., hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof, and/or sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumerate, and combinations thereof. Generally, the excipient will be present in pharmaceutical composition according to the present invention in an amount of 0.001 to 99.999 wt.%, preferably from 0.01 to 99.99 wt.%, more preferably from 0.1 to 99.9 wt.-%, in each case based on the total weight of the pharmaceutical composition.

The composition of the invention is preferably used in a formulation suitable for subcutaneous or intravenous or parenteral injection. For this, suitable excipients and carriers are e.g. sodium dihydrogen phosphate, disodium hydrogen phosphate, sodium chloride, sodium glutamate, mannitol, sorbitol, polysorbate 80, HSA and water for injection. The composition may be administered three times a week, preferably two times a week, more preferably once a week, and most preferably every two weeks.

Preferably, the pharmaceutical composition is administered in an amount of 0.01 μg/kg - 6 mg/kg body weight of the patient, preferably 0.01 - 10 μg/kg body weight of the patient, more preferably 0.1 - 5 μg/kg, in particular 0.1 - 1 μg/kg, or 0.2 - 0.9 μg/kg, most preferably 0.3 - 0.7 μg/kg, and most preferred 0.4 - 0.6 μg/kg body weight.

In general, preferably between 10 μg and 6 mg, preferably 10 μg and 200 μg, preferably between 15 μg and

100 $\mu g$ are administered per dose.

The invention further relates to a hydroxyalkyl starch derivative obtainable by a method as described above, in particular comprising steps a) to c) as a therapeutic or prophylactic agent.

The invention further relates to the use of a hydroxyalkyl starch derivative obtainable by a method as described above, in particular when comprising steps a) to c) and when in step c) biologically active agent BA is IFN-alpha, for the preparation of a medicament for the treatment of a disease selected from the group consisting of cancer, such as hairy cell leukaemia, malignant melanoma, follicular lymphoma and/or AIDS related Kaposi's sarcoma, condylomata acuminate, chronic hepatitis B and chronic hepatitis C, preferably chronic hepatitis B and hepatitis C.

The invention further relates to the use of a hydroxyalkyl starch derivative obtainable by a method as described above, in particular when comprising steps a) to c) and when in step c) biologically active agent BA is G-CSF, for the preparation of a medicament for the treatment of disorders in patients selected from the group consisting of cancer patients, such as cancer patients receiving myelosuppressive chemotherapy, patients with acute myeloid leukaemia receiving induction or consolidation chemotherapy and/or cancer patients receiving marrow bone transplant, patients undergoing peripheral blood progenitor cell collection and therapy, and patients with severe chronic neutropenia.

The invention further relates to the use of a hydroxyalkyl starch derivative obtainable by a method as described above, in particular when comprising steps a) to c) and when in step c) biologically active agent BA is EPO, for the preparation of a medicament for the treatment of anemia, such as of chronic renal failure patients, Zidovudine-treated HIV-infected patients, cancer patients on chemotherapy, or for the reduction of allogeneic blood transfusion in surgery patients.

The invention further relates to the use of a hydroxyalkyl starch derivative obtainable by a method as described above, in particular when comprising steps a) to c) and when in step c) biologically active agent BA is prolactin or a mutant thereof, for the preparation of a medicament for the prophylaxis or treatment of cancer, in particular breast cancer.

The invention further relates to a method for the treatment of disorders in patients selected from the group consisting of cancer patients, such as cancer patients receiving myelosuppressive chemotherapy, patients with acute myeloid leukaemia receiving induction or consolidation chemotherapy and/or cancer patients receiving marrow bone transplant, patients undergoing peripheral blood progenitor cell collection and therapy, and patients with severe chronic neutropenia; anemia, such as of chronic renal failure patients, Zidovudine-treated HIV-infected patients, cancer patients on chemotherapy, or for the reduction of allogeneic blood transfusion in surgery patients; and cancer, in particular breast cancer, by administering a therapeutically effective amount of a hydroxyalkyl starch conjugate as obtainable according to the method as described above.

The invention is further described in more detail with the following non-limiting examples:

**Description of figures:**

**Figure 1** shows an analysis of the crude protein-HES 60/1.0 conjugates by SDS gel electrophoresis.

Lane X: Mark12 MW Standard (Invitrogen, Karlsruhe, D) Molecular weight marker from top to bottom in kDa: 200, 116, 97, 66, 55, 37, 31, 22, 14, 6, 3.5, 2.5.
Lane A: 3.7 $\mu g$ IFN$\alpha$
Lane B: Synthesis of $Q^4$
Lane C: Synthesis of Q5.
Lane Y: Roti®-Mark STANDARD (Carl Roth GmbH + Co. KG, Karlsruhe, D) Molecular weight marker from top to bottom: 200 kDa, 119 kDa, 66 kDa, 43 kDa, 29 kDa, 20 kDa, 14.3 kDa.
Lane D: Synthesis of Q8.
Lane E: Synthesis of Q9.
Lane F: 10 $\mu g$ IFN$\alpha$.
Lane G: 5 $\mu g$ IFN$\alpha$.
Lane H: 2.5 $\mu g$ IFN$\alpha$.
Lane I: 1 $\mu g$ IFN$\alpha$.

**Figure 2** shows an analysis of the crude protein-HES 100/1.0 conjugates by SDS gel electrophoresis.

Lane X: Roti®-Mark STANDARD (Carl Roth GmbH + Co. KG, Karlsruhe, D) Molecular weight marker from top to bottom: 200 kDa, 119 kDa, 66 kDa, 43 kDa, 29 kDa, 20 kDa, 14.3 kDa.
Lane A: Synthesis of R83.
Lane B: Synthesis of R84.

Lane C: Synthesis of R85.

**Figure 3** shows a graph on the proliferative activity of Intron A against NIH-standard according to example 6.1.

**Figure 4** shows a graph of the *in vitro* activity of HES-IFN-alpha according to example 6.2.

**Figure 5** shows a graph with the half-life of HES-IFN-alpha according to example 7.2.

**Figure 6** shows an analysis of the HES-G-CSF conjugation reactions according to example 8 by SDS gel electrophoresis.

Lane M: Mark12 MW Standard (Invitrogen, Karlsruhe, D) Molecular weight marker from top to bottom in kDa: 200, 116, 97, 66, 55, 37, 31, 22, 14, 6, 3.5, 2.5.
Lane 1: 10 μg reaction mixture HES60/0.7-G-CSF
Lane 2: 10 μg reaction mixture HES60/1.0-G-CSF
Lane 3: 10 μg reaction mixture HES100/1.0-G-CSF
Lane 4: 10 μg reaction mixture HES100/1.3-G-CSF
Lane 5: 5 μg rh-metG-CSF

**Figure 7** shows an analysis of the crude protein-HES 60/1.3 conjugates obtained according to example 9 by SDS gel electrophoresis.

Lane X: Roti@-Mark STANDARD (Carl Roth GmbH + Co. KG, Karlsruhe, D) Molecular weight marker from top to bottom in kDa: 245, 123, 77, 42, 30 , 25.4 , 17.
Lane A: 10 μg reaction mixture HES60/1.3-EPO

**Experimental part:**

Specifications HES and HES- Derivatives:

$M_w$: Weight average molecular weight
$M_n$ : Number average molecular weight

$M_w$ and $M_n$ are determined by Gel Permeation Chromatography in combination with a light scattering detector
MS = Molar substitution: Average number of hydroxyethyl substituents per anhydro glucose residue of HES
MS is determined by Gas Chromatography after total hydrolysis of the HES molecule. MS values for Aldehydo-HES were not determined experimentally. MS values of respective HES starting material are given instead. The MS value is supposed not to be effected during the derivatization procedure.

<u>HES 60/1.0:</u>

HES 60/1.0 (Supramol Parenteral Products GmbH, Rosbach)

$M_w$: 62 kDa
$M_n$: 35 kDa
MS: 1.01

Aldehydo-HES- 60/1.0 (Synthesized as described in Example 1.2b)

$M_w$: 63 kDa
$M_n$: 35 kDa
MS: 1.01

HES100/1.0:

HES 100/1.0 (Supramol Parenteral Products GmbH, Rosbach)

$M_w$: 93 kDa
$M_n$: 41 kDa
MS: 1.01

Aldehydo-HES 100/1.0 (Synthesized as described in Example 1.2a)

$M_w$: 92 kDa
$M_n$: 41 kDa
MS: 1.01

Specification IFN$\alpha$:

Interferon Alfa-2b, which is described in the European Pharmacopoeia:

Interferon Alfa-2b concentrated solution, Ph.Eur. 01/2002:1110

**Example 1 Synthesis of Aldehydo-HES derivatives 60/1.0 and 100/1.0**

**Example 1.1** Preparation of Hydroxylamino-HES 60/1.0 and 100/1.0 from HES 60/1.0 and 100/1.0

HES A (**B** g, MW = C kDa, Lot **D**, Supplier Supramol Parenteral Products GmbH, Rosbach, D) was dissolved in reaction buffer (**F** mL, 0.1 M sodium acetate, pH 5.2), prepared from Ampuva water (Fresenius-Kabi, Bad Homburg, D), and O-[2-(2-aminooxy-ethoxy)-ethyl]-hydroxylamine (**G** g, prepared as described in Boturyn et al. Tetrahedron 53 (1997) p. 5485-5492 in 2 steps from commercially available starting materials) was added. After stirring at room temperature for **H** h, the reaction mixture was added to 2-propanol (**I** mL). The precipitated product was collected by centrifugation at 4°C. The crude product was dissolved in water (**J** mL), dialysed for 50 h against Milli-Q water (SnakeSkin dialysis tubing, 10 kDa MWCO, Perbio Sciences Deutschland GmbH, Bonn, D) and lyophilized. The yield of isolated product was **L** %.

Table 1 : Synthesis of Hydroxylamino-HES derivatives

| Example | A | B \|g\| | C \|kDa\| | D | F \|mL\| | G \|g\| | H \|h\| | I \|mL\| | J \|mL\| | L \|%\| |
|---|---|---|---|---|---|---|---|---|---|---|
| 1.1a | 100/1.0 | 15.46 | 93 | 538 | 155 | 2.12 4 | 21.5 | 1085 | 155 | 96 |
| 1.1b | 100/1.0 | 10.13 | 93 | 538 | 100 | 1.42 1 | 23 | 700 | 100 | 94 |
| 1.1c | 60/1.0 | 20.02 | 62 | 539 | 200 | 4.56 7 | 23 | 1400 | 200 | 93 |

**Example 1.2** Preparation of Aldehydo-HES 60/1.0 and 100/1.0 from Hydroxylamino-HES 60/1.0 and 100/1.0

Hydroxylamino-HES A (**B** g, MW = **C** kDa, prepared as described in example **D**) was dissolved in **E** ml DMF (Peptide synthesis grade, Biosolve, Valkenswaard, NL) and terephthalaldehyde (**F** g, Lancaster Synthesis, Frankfurt/Main, D) was added. After shaking for **G** h at room temperature the reaction mixture was added slowly to a 1:1 mixture of acetone and ethanol (**H** mL, v/v). The precipitated product was collected by centrifugation at 4°C, re-dissolved in DMF (**E** mL) and precipitated with a mixture of acetone and ethanol (**H** mL, v/v) as described above. After centrifugation, the precipitate was dissolved in water (E mL, Milli-Q), dialysed for **I** h against Milli-Q water (SnakeSkin dialysis tubing, 10 kDa MWCO, Perbio Sciences Deutschland GmbH, Bonn, D) and lyophilized. The yield of isolated product was **M** %.

Table 2: Synthesis of Aldehydo-HES derivatives

| Example | A | B \|g\| | C \|kDa\| | D | E \|mL\| | F \|g\| | G \|h\| | H \|mL\| | 1 \|h\| | M \|%\| |
|---|---|---|---|---|---|---|---|---|---|---|
| 1.2a | 100/1.0 | 21.97 | 99.0 | 1.1a, 1.1b | 220 | 2.987 | 21.5 | 1540 | 47 | 100 |
| 1.2b | 60/1.0 | 17.30 | 59.6 | 1.1c | 170 | 3.891 | 21.5 | 1200 | 47 | 96 |

**Example 2 Synthesis of HES (60/1.0)-IFN-α-conjugates**

**Example 2.1** Synthesis of HES (60/1.0)-IFNα-conjugate (Lot: Q4)

Buffer exchange

The IFNα solution (5.32 mL, 10 mg, 1.88 mg/mL) was centrifuged at 20°C for 20 min at 7.500 x g in an Amicon Ultra-4 concentrator (Millipore, 5 kDa MWCO). The washing procedure was repeated three times by dilution of the residual solution with reaction buffer (0.1 M sodium acetate, pH 4.0) to 5 mL and centrifugation for 16 min as described. The final volume was adjusted to 1.15 mL (calculated concentration of 8.7 mg/mL) with reaction buffer. The protein concentration was not checked experimentally.

Conjugation

Aldehydo-HES 60/1.0 (623 mg, prepared as described in Example 1.2b, 20 equiv.) was dissolved in protein solution (1.15 mL, 8.7 mg/mL, prepared as described above) and the mixture was thoroughly mixed with a pipette. After incubation for 15 min on ice, a pre-cooled sodium cyanoborohydride solution (40 μL, 1.24 M in reaction buffer, Fluka, Sigma-Aldrich Chemie GmbH, Taufkirchen, D) was added and the reaction mixture was incubated for 6 h at 0°C. Precipitation was observed on addition of sodium cyanoborohydride. The reaction was quenched by adding 3 mL reaction buffer and 2.97 mL of a 0.1 M sodium acetate solution containing 350 mM lysine (Merck, Darmstadt, D). The mixture was diluted to 10 mL with reaction buffer and stored on ice over night before purification. The reaction mixture was analyzed by SDS-PAGE (see Figure 1).

**Example 2.2** Synthesis of HES (60/1.0)-IFNα-conjugate (Lot: Q5)

Buffer exchange

The IFNα solution (5.32 mL, 10 mg, 1.88 mg/mL) was centrifuged at 20°C for 20 min at 7.500 x g in an Amicon Ultra-4 concentrator (Millipore, 5 kDa MWCO). The washing procedure was repeated three times by dilution of the residual solution with reaction buffer (0.1 M sodium acetate, pH 4.0) to 5 mL and centrifugation for 16 min as described. The final volume was adjusted to 0.926 mL (calculated concentration of 10.8 mg/mL) with reaction buffer. The protein concentration was not checked experimentally.

Conjugation

Aldehydo-HES 60/1.0 (309 mg, prepared as described in Example 1.2b, 10 equiv.) was dissolved in protein solution (0.926 mL, 10.8 mg/mL, prepared as described above) and the mixture was thoroughly mixed with a pipette. Pre-cooled sodium cyanoborohydride solution (20.3 μL, 1.24 M in reaction buffer, Fluka, Sigma-Aldrich Chemie GmbH, Taufkirchen, D) was added and the reaction mixture was incubated for 2 h at 21 °C. The reaction was quenched by adding 3 mL reaction buffer and 1.5 mL of a 0.1 M sodium acetate solution and containing 350 mM lysine (Merck, Darmstadt, D). The mixture was diluted to 10 mL with reaction buffer and stored over night on ice before purification. The reaction mixture was analyzed by SDS-PAGE (see Figure 1).

**Example 2.3** Synthesis of HES (60/1.0)-IFNα-conjugate (Lot: Q8)

Buffer exchange

The IFNα solution (5.32 mL, 10 mg, 1.88 mg/mL) was centrifuged at 20°C for 45 min at 3.939 x g in a Vivaspin 6 mL CONCENTRATOR (Viva Science, 5 kDa MWCO, Hannover, D). The washing procedure was repeated three times by dilution of the residual solution with reaction buffer (0.1 M sodium acetate, pH 4.0) to 6 mL and centrifugation for 40 min as described. The final volume was adjusted to 1.0 mL (calculated concentration of 10 mg/mL) with reaction buffer.

The protein concentration was not checked experimentally.

Conjugation

Aldehydo-HES 60/1.0 (232 mg, prepared as described in Example 1.2b, 7.5 equiv.) was dissolved in protein solution (1.0 mL, 10 mg/mL, prepared as described above) and the mixture was thoroughly mixed with a pipette. After incubation for 15 min on ice, a pre-cooled sodium cyanoborohydride solution (100 μL, 200 mM in reaction buffer, Fluka, Sigma-Aldrich Chemie GmbH, Taufkirchen, D) was added and the reaction mixture was incubated for 7 h at 10°C. The reaction was quenched with 100 equiv. lysine (1.95 mL, 0.2 M lysine hydrochloride (Fluka Biochemica, Sigma-Aldrich, Taufkirchen, D) in reaction buffer, pH 4.0) and 10 mM methionine (650 μL, 0.1 M methionine (Fluka Biochemica, Sigma-Aldrich, Taufkirchen, D) in reaction buffer, pH 4.1). The mixture was diluted to 6.5 mL with reaction buffer and stored over night on ice before purification. The reaction mixture was analyzed by SDS-PAGE (see Figure 1).

**Example 2.4** Synthesis of HES (60/1.0)-IFNα-conjugate (Lot Q9)

Buffer exchange

The buffer of the IFNα solution (5.32 mL, 10 mg, 1.88 mg/mL) was exchanged with a Vivaspin 6 mL CONCENTRATOR to 0.1 M sodium acetate, pH 4.0 as described in Example 2.3 and the solution was incubated in the concentrator for 15 min at 0°C.

Conjugation

An Aldehydo-HES solution (155 mg, prepared as described in Example 1.2b, 5 equiv. in 5 mL reaction buffer (0.1 M sodium acetate, pH 4.0), pre-cooled on ice) was added and the mixture was centrifuged for 2.5 h at 3.939 x g and 10°C. A pre-cooled sodium cyanoborohydride solution (5 mL, 20 mM in reaction buffer, Fluka, Sigma-Aldrich Chemie GmbH, Taufkirchen, D) was added to the remaining volume of approximately 700 μL reaction mixture. The solution was centrifuged for 18 h at 3939 x g and 10°C and was stored on ice before purification. The reaction mixture was analyzed by SDS-PAGE (see Figure 1).

**Example 3 Synthesis of HES (100/1.0)-IFN-α-conjugates**

**Example 3.1** Synthesis of HES (100/1.0)-IFNα-conjugate (Lot R83)

IFNα stock solution (19.68 ml, 37 mg, 1.88 mg/mL) was divided equally into two Vivaspin 15R concentrators (Viva Science, 5 kDa MWCO, Hannover, D), diluted with reaction buffer (0.1 M sodium acetate buffer, pH 4.0, prepared with Ampuwa-water (Fresenius-Kabi, Bad Homburg, D)) to 18 mL each and were centrifuged at 21°C for 36 min at 3939 x g. The washing procedure was repeated three times by dilution of the residual solutions with the reaction buffer to 18 mL and centrifugation for 35 min as described. The combined volume of the protein solutions was adjusted to 3.47 mL with reaction buffer (calculated protein concentration of 10.8 mg/mL). The protein concentration was not checked experimentally.

Aldehydo-HES 100/1.0 (1.156 g, prepared as described in Example 1.2a, 12 equiv.) was dissolved in reaction buffer (1.628 mL) and the protein solution (1.736 mL, 10.8 mg/mL, prepared as described above) was added. After incubation for 30 min at 0°C, a pre-cooled sodium cyanoborohydride solution (100 μL, 58.2 mg/mL in reaction buffer, Fluka, Sigma-Aldrich Chemie GmbH, Taufkirchen, D) was added and the mixture was thoroughly mixed. Small amounts of a fine precipitate formed on $NaCNBH_3$ addition. The solution cleared during mixing, its volume was adjusted to 4.6 mL with reaction buffer and the mixture was incubated for 18 h at 0°C. The reaction mixture was analyzed by SDS PAGE (see Figure 2).

**Example 3.2** Synthesis of HES (100/1.0)-IFNα-conjugate (Lot R84)

HES-Aldehyde100/1.0 (462 mg, prepared as described in Example 1.2a, 4.8 equiv.) was dissolved in the protein solution (1.736 mL, 10.8 mg/mL, prepared as described in Example 3.1). After incubation for 30 min at 0°C, a pre-cooled sodium cyanoborohydride solution (50 μL, 58.2 mg/mL in reaction buffer, Fluka, Sigma-Aldrich Chemie GmbH, Taufkirchen, D) was added and the mixture was thoroughly mixed. Small amounts of a fine precipitate formed on $NaCNBH_3$ addition. The solution cleared during mixing, its volume was adjusted to 2.3 mL with reaction buffer and the mixture was incubated for 17.5 h at 5°C. The reaction mixture was analyzed by SDS PAGE (see figure 2).

**Example 3.3** Synthesis of HES (100/1.0)-IFN-α-conjugate (Lot R85)

IFNα stock solution (10.63 ml, 20 mg, 1.88 mg/mL) was diluted with reaction buffer (0.1 M sodium acetate buffer, pH 4.0, prepared with Ampuwa-water (Fresenius-Kabi, Bad Homburg, D) to 18 mL and was centrifuged at 21°C for 40 min at 3939 x g in a Vivaspin 15R concentrator (Viva Science, 5 kDa MWCO, Hannover, D). The washing procedure was repeated three times by dilution of the residual solution with the reaction buffer to 18 mL and centrifugation for 35 min as described. The volume of the protein solution was adjusted to 1.85 mL with reaction buffer (calculated protein concentration of 10.8 mg/mL), 40 μL were diluted to 1 mL with reaction buffer and were measured at 279 nm against reaction buffer as a control. The amount of protein was calculated with a molar extinction coefficient of 18000 and the $OD_{279}$ (0.382) to 18.9 mg resulting in a protein recovery of 95%.

HES-Aldehyde100/1.0 (616 mg, prepared as described in Example 1.2a, 6 equiv.) was dissolved in the protein solution (1.85 mL, 10.8 mg/mL, prepared as described above). Sodium cyanoborohydride (3.2 mg, Fluka, Sigma-Aldrich Chemie GmbH, Taufkirchen, D) was added and the mixture was thoroughly mixed. Small amounts of a fine precipitate formed on $NaCNBH_3$ addition. The solution cleared during mixing and the mixture was incubated for 2 h at 21 °C. The reaction mixture was analyzed by SDS PAGE (see Figure 2).

**Example 4 SDS PAGE analysis of HES-IFN-α-conjugates**

Samples containing 10 μg protein (the corresponding volume was determined from the calculated protein concentration in each reaction mixture) of the reaction mixtures obtained from example 2 and 3 were investigated. A XCell Sure Lock Mini Cell (Invitrogen GmbH, Karlsruhe, D) and a Consort E143 power supply (CONSORTnv, Turnhout, B) were employed for SDS gel electrophoresis. A 4-12 % Bis-Tris gel together with a MOPS SDS running buffer at reducing conditions (both Invitrogen GmbH, Karlsruhe, D) were used according to the manufacturers instruction.

**Example 5 Purification of the IFN-alpha -HES conjugates**

**5.1 Purification of HES-IFN-α**

The purification of all samples was performed at room temperature using an AKTA explorer 10 equipment. The column containing 10 ml Q-Sepharose Fast Flow was one day before use treated with 1 M NaOH and at a flow rate of 1 ml/min. regenerated and afterwards equilibrated with buffer A (20 mM Tris/HCl, pH 8.0). The samples were diluted 1: 8 with buffer A under steril conditions and were applied by using the sample pump at a flow rate of 1,5 ml/min. Following washing of the sample pump with 25 ml of buffer A, the column was further washed with 30 ml of buffer A at a flow rate of 1.5 ml/min. Elution was performed by using a linear gradient from 0-100% of buffer B (0.3 M NaCl in 20 mM Tris/HCl, pH 8.0) over 60 ml and an isocratic run with 20 ml of buffer B at a flow rate of 1.2 ml/min. The column was regenerated by using 30 ml of buffer C (1.5 M NaCl in 20 mM Tris/HCl, pH 8.0) followed by 10 ml of buffer B at a flow rate of 1.2 ml/min. Reequilibration for the next run was performed by using 50 ml of buffer A and a flow rate of 1.5 ml/min.

**5.2 Materials and Methods**

| | |
|---|---|
| Equipment: | ÄKTA explorer 10 (Amersham Pharmacia Biotech), with:<br>Pump P-903<br>Mixer M-925, with 0.6 ml chamber<br>Monitor UV-900, with 10 mm flow cell<br>Monitor pH/C-900<br>Pump P-950 (sample pump)<br>Software Unicorn Version 3.21 |
| Column: | Amersham Biosciences XK 16/20 |
| Column material: | Q-Sepharose Fast Flow, Code no. 17-0510-01, Lot no. 307552 |
| Column volume: | 10 ml |

**5.2.1** Method

Buffer A: 20 mM Tris/HCl, pH 8.0
Buffer B: 0.3 M NaCl in 20 mM Tris/HCl, pH 8.0
Buffer C: 1.5 M NaCl in 20 mM Tris/HCl, pH 8.0

| Volume | Step | Buffer | Flow rate |
|---|---|---|---|
| 25 ml | Equilibration | 100% buffer A | 1.5 ml/min |
| | Load sample | | 2-3 ml/min |
| 25 ml | Wash sample pump | 100% buffer A | 3.0 ml/min |
| 30 ml | Wash out unbound sample | 100% buffer A | 1.5 ml/min |
| | Start Fractionation | | |
| 60 ml | Elution, linear gradient | 0-100% buffer B | 1.2 ml/min |
| 20 ml | Elution, isocratic | 100 % buffer B | 1.2 ml/min |
| 30 ml | Regeneration | 100% buffer C | 1.2 ml/min |
| 10 ml | Regeneration | 100% buffer B | 1.2 ml/min |
| | Stop Fractionation | | |
| 50 ml | Reequilibration | 100% buffer A | 1.5 ml/min |

Detection: 280 nm, 260 nm, 220 nm
Conductivity
Fractionation: 1,5 ml fractions

5.2.2 Method

Buffer A: 10 mM Tris/HCl, pH 8.0
Buffer B: 0.3 M NaCl in 20 mM Tris/HCl, pH 8.0
Buffer C: 1.5 M NaCl in 20 mM Tris/HCl, pH 8.0

| Volume | Step | Buffer | Flow rate |
|---|---|---|---|
| 25 ml | Equilibration | 100% buffer A | 1.5 ml/min |
| | Load sample | | 2.0 ml/min |
| 15 ml | Wash sample pump | 100% buffer A | 3.0 ml/min |
| 30 ml | Wash out unbound sample | 100% buffer A | 1.5 ml/min |
| | Start Fractionation | | |
| 60 ml | Elution, linear gradient | 0-100% buffer B | 1.2 ml/min |
| 20 ml | Elution, isocratic | 100 % buffer B | 1.2 ml/min |
| 30 ml | Regeneration | 100% buffer C | 1.2 ml/min |
| 10 ml | Regeneration | 100% buffer B | 1.2 ml/min |
| | Stop Fractionation | | |
| 50 ml | Reequilibration | 100% buffer A | 1.5 ml/min |

Detection: 280 nm, 260 nm, 220 nm
Conductivity
Fractionation: 1,5 ml fractions

**5.3 Results**

**5.3.1** Sample according to Example 2.1

Purification according to method 5.2.1

| | |
|---|---|
| starting volume: | 10 ml, diluted 1:8 in buffer A =80 ml |
| fractions: | 80/1.5 ml |
| run no.: | QS 172 Q04 |

As result of the high lysine concentration in the sample composition the main part of the protein conjugate was not capable of binding to the column. Therefore a further purification step was performed.

**5.3.2** Sample according to Example 2.1

sample composition: 220 ml flow through from example 5.3.1. Purification according to method 5.2.1

| | |
|---|---|
| starting volume: | 220 ml, diluted 1:2 in buffer A =440 ml |
| Flow rate: | 3,5 ml/min (Load sample) |
| fractions: | 80/1.5 ml |
| run no.: | QS174 Q04 |

**5.3.3** Sample according to Example 2.2

Purification according to method 5.2.1

| | |
|---|---|
| starting volume: | 10 ml, diluted 1:8 in buffer A =80 ml |
| fractions: | 80/1.5 ml |
| run no.: | QS173 Q05 |

As result of the high lysine and methionine concentration in the sample composition the main part of the protein conjugate was not capable of binding to the column. Therefore a further purification step was performed.

**5.3.4** Sample according to Example 2.2

sample composition: 220 ml flow through from example 5.3.3. Purification according to method 5.2.1

| | |
|---|---|
| starting volume: | 220 ml, diluted 1:2 in buffer A =440 ml |
| Flow rate: | 3,5 ml/min (Load sample) |
| fractions: | 80/1.5 ml |
| run no.: | QS177 Q05 |

**5.3.5** Sample according to Example 2.3

Purification according to method 5.2.1

| | |
|---|---|
| starting volume: | 6.5 ml, diluted 1:3 in buffer A =20 ml |
| run no.: | QS 176 Q08 |

**5.3.6** Sample according to Example 2.4

Purification according to method 5.2.1

| | |
|---|---|
| starting volume: | 0.8 ml, diluted 1:25 in buffer A =20 ml |
| fractions: | 80/1.5 ml |
| run no.: | QS 175 Q09 |

**5.3.7** Sample according to Example 3.1

Purification according to method 5.2.2

| | |
|---|---|
| starting volume: | 4.6 ml, diluted 1:17 in buffer A =80 ml, adjusted with 48 μl 12,5% ammonia solution to pH 8-8,5 |
| fractions: | 80/1.5 ml |
| run no.: | QS183 R83 |

**5.3.8** Sample according to Example 3.2

Purification according to method 5.2.2

| | |
|---|---|
| starting volume: | 2.3 ml, diluted 1:17 in buffer A =40 ml, adjusted with 48 μl 12,5% ammonia solution to pH 8-8,5 |
| fractions: | 80/1.5 ml |
| run no.: | QS1184 R84 |

**5.3.9** Sample according to Example 3.3

Purification according to method 5.2.2

| | |
|---|---|
| starting volume: | 2.5 ml, diluted 1:17 in buffer A =43ml, adjusted with 48 μl 12,5% ammonia solution to pH 8-8,5 |
| fractions: | 80/1.5 ml |
| run no.: | QS177 R85 |

**5.4 Comparison of the results**

Protein concentrations were determined from pooled fractions of example 5.3 Fractions: Q4 8-24; Q5 8-23; Q8 9-24; Q9 7-16; R83 9-20 ; R84 9-20; R85 9-20 The protein concentration was determined in RP-HPLC at 280 nm and 221 nm. Quantification was performed with an external IFN-a standard with a calibration function. Calculation of the concentration determined photometrically was performed using the extinction coefficient ε = 0.9054 ml $mg^{-1}cm^{-1}$.

As a standard α-Interferon CRS-IFN-α-2b was used, which had a protein concentration of 1.724 mg/ml. For the calibration concentrations of 10 μg, 20 μg and 40 μg were used. The calibration was calculated from the mean area of two injections of the calibration standards. To calculate the protein concentration of the samples, two injections were performed and the mean area was calculated.

| Sample Code | Conc. (Photometer) (280nm) | | | Conc. (HPLC) (280nm) | | | Conc. (HPLC) (221nm) | | | Yield (%) (HPLC221nm) |
|---|---|---|---|---|---|---|---|---|---|---|
| | | total | | | total | | | total | | |
| | [mg/ml] | [mg] | [ml] | [mg/ml] | [mg] | [ml] | [mg/ml] | [mg] | [ml] | |
| Q4 | 2.18 | 6.5 | 3 | 1.74 | 5.2 | 3 | 1.02 | 3.1 | 3 | 36 |
| Q5 | 2.14 | 8.6 | 4 | 1.88 | 7.5 | 4 | 1.06 | 4.2 | 4 | 49 |
| Q8 | 2.29 | 13.7 | 6 | 1.88 | 11.3 | 6 | 1.09 | 6.5 | 6 | 73 |
| Q9 | 2.62 | 15.7 | 6 | 2.08 | 12.5 | 6 | 1.14 | 6.8 | 6 | 76 |
| R83 | - | - | - | 2.14 | 21.4 | 10 | 1.23 | 12.3 | 10 | 72 |
| R84 | - | - | - | 2.09 | 20.9 | 10 | 1.22 | 12.2 | 10 | 71 |
| R85 | - | - | - | 2.19 | 21.9 | 10 | 1.28 | 12.8 | 10 | 70 |

For all further investigations the protein concentration refers to the HPLC(221nm) value.

**Example 6: Description of IFN alpha antiviral activity bioassay**

**Description of the Test Procedure: Antiviral activity of Interferon-alpha**

After pre-diluting the Test Items in cell culture medium, serial two-fold dilutions were prepared. In 96 well microtiter plates, diluted Interferon was added -in four-fold replicate per dilution- to freshly trypsinized MDBK cells (40.000 cells per well). The assays were incubated for 24 hours at 37 °C (total volume per well: 175 μl.

Subsequently, 50 μL diluted VSV stock solution were added to each well (except for the positive control wells) resulting in a multiplicity of infection of 0.1.

The following controls were included in each assay: 12 wells that received virus plus cell culture medium instead of Interferon (negative control) and 12 wells that received cell culture medium instead of Interferon and virus (positive control).

The assays were incubated for 42 hours at 37 °C.

At the end of the incubation period the cell culture supernatant of each well was replaced with 50 μL of a solution of MTT (at least 2 mg/mL in cell culture medium). The cells were incubated for three hours. The purple formazan dye formed by the proliferating cells was solubilized by adding 100 μL solution of isopropanol/HCl (isopropanol with 40 mM HCl) to each well. Subsequently, the absorbance values of the solutions were measured at 570/630 nm in a microtiter plate reader.

The proliferative activity of MDBK cells grown in the presence of Interferon and VSV was calculated for each dilution of Interferon as follows:

$$\frac{((\text{Mean absorbance of four Interferon treated wells}) - (\text{Mean absorbance of negative control})) * 100}{(\text{Mean absorbance of positive control}) - (\text{Mean absorbance of negative control})}$$

The antiviral activity of Interferon-alpha was determined in four separate assays for each of the Test Items.

**Example 6.1: Antiviral activity of Intron A relative to NIH standard**

In all experiments, Intron A (IFN-alpha 2b, Schering-Plough), calibrated against NIH-standard rhIFN-alpha 2a (NIAID, NIH, Bethesda, USA, Gxa01-901-535) was used as an internal lab reference. The NIH-standard had a specific activity of 9,000 IU/ml. The internal lab reference Intron A had a specific activity of 8,487,000 IU/ml in the test.

Proliferative activity of Intron A compared to NIH standard rhIFN-alpha 2a is shown in Fig. 3

**Example 6.2: Antiviral activity of IFN-alpha-HES conjugates relative to Intron A**

In the assay system described in Example 6, the conjugates (Q4; Q5; Q8; Q9; R83; R84; R85) were tested compared to unmodified IFN-alpha starting material, Intron A and Pegasys (Roche). The CPE50 concentration of the

materials was calculated. All IFN-alpha-HES conjugates retained an antiviral activity which was substantially higher than that of Pegasys.

The relative *in vitro* activity of IFN-alpha-HES conjugates compared to unmodified IFN-alpha starting material and Intron A is shown in Fig. 4

**Example 7: *In vivo* bioactivity of IFN-alpha-HES conjugates (PK study in mice)**

**Example 7.1: Influence of mouse serum on assay system**

Dilutions of Interferon-alpha were prepared in cell culture medium (control) and in mouse serum (1:40 dilution and 1:80 dilution). The assay was performed as described in Example 6.

The antiviral activity of Interferon-alpha was determined in two separate assays for the control, for mouse serum 1:40 diluted as well as for mouse serum 1:80 diluted. The results indicated that mouse serum at 1:40 dilution and 1:80 does not affect the bioassay for antiviral activity of Interferon-alpha.

**Example 7.2: *In vivo* study in mice**

Antiviral activity of pooled serum was tested in the antiviral assay. Serum was collected from two mice (female BALB/c mice, aged 8 weeks) at each time, which were sacrificed 2h, 4h, 12h, and 24h post i.v.-injection of 30μg/kg (based on the protein content) of IFN-alpha or the IFN-alpha-HES conjugate.

The serum samples were thawed and thoroughly homogenized by vortexing (and diluted). Serial two-fold dilutions were prepared in cell culture medium. A vial of Intron A (diluted) was thawed and thoroughly homogenized by vortexing. Serial two-fold dilutions were prepared in cell culture medium. The EC50-dilutions in the CPE-assay were determined from dose response curves of a 1:2 dilution series as described in Example 6.

The half life of the materials was determined compared to unmodified starting material and Pegasys. The half life was calculated from a semi-logarithmic plot of the EC50-dilution vs. time post injection. Antiviral activity was detected for HES-IFN-$\alpha$: Q4; Q5; Q8; Q9; R83; R84; R85 up to 24 h. As can be seen from Fig. 5, the half-life lies between 6 to 8.7 h. For unmodified IFN-alpha, the antiviral activity of serum was too low to calculate a serum half-life. In K.R. Reddy et al. Advanced Drug Delivery Reviews 54 (2002) 571-586 a serum half-life of IFN-alpha in rats (i.v.) of 2 h was determined.

**Example 8: Synthesis of HES-G-CSF conjugates**

For the coupling reaction (reaction conditions see table below) the rh-metG-CSF solution (e.g. Neupogen® or a bioequivalent preparation) formulated in an acetate buffer (10 mM, pH 4-5) was filled into a 50 ml Falcon tube equipped with a magnetic stirrer bar. The respective Aldehydo-HES (al-HES) derivative (prepared in analogy to Example 1) was dissolved in reaction buffer (0.1 M sodium acetate, pH 5.0) and slowly added dropwise to the protein under stirring (~200 rpm) to yield a HES derivative/G-CSF ratio of 30:1. Mixing was continued until the sample appeared homogenous. The reaction was started by addition of a 25fold concentrated $NaCNBH_3$ solution (0.5 M; final concentration 20 mM) again under mixing with a stirrer. The Falcon tube was incubated over night (16 h) without further stirring in a refrigerator set to 10 °C.

**Coupling conditions for the preparation of HES-G-CSF conjugates**

| al-HES (Mw/Ms) | rhG-CSF mass | rh-G-CSF volume | al-HES mass | 0.5 M $NaCNBH_3$ volume | reaction buffer volume | resulting rh-G-CSF conc. | al-HES conc. in % w/v |
|---|---|---|---|---|---|---|---|
| 60/0.7 | 30 mg | 16 ml | 3.34 g | 0.9 ml | 2.0 ml | 1.4 g/l | 15 |
| 60/1.0 | 29 mg | 6 ml | 2.55 g | 0.5 ml | 3.7 ml | 2.2 g/l | 20 |
| 100/1.0 | 30 mg | 16 ml | 5.98 g | 1.2 ml | 6.7 ml | 1.0 g/l | 20 |
| 100/1.3 | 33 mg | 10 ml | 7.52 g | 1.5 ml | 18.6 ml | 0.9 g/l | 20 |

Successful conjugation of G-CSF to HES was shown by SDS-PAGE analysis (cf. Fig. 6).

**Example 9: Synthesis of HES-EPO conjugates**

Buffer exchange

The EPO solution (17.5 mL, 35 mg, 2 mg/mL, e.g. Epocrit® or a bioequivalent preparation) were centrifuged at 20°C for 30 min at 3939 x g in a Vivaspin 15R concentrator (Viva Science, 10 kDa MWCO, Hannover, D). The washing procedure was repeated twice by dilution of the residual solution with the reaction buffer (0.1 M sodium acetate, pH 5.0) to 18 mL and centrifugation for 35 min as described.

Conjugation

Aldehydo-HES derivative 60/1.3 (prepared in analogy to Example 1, 2.893 g, 40 equiv.) was dissolved in the protein solution and reaction buffer to a final volume of 6.0 mL. The mixture was thoroughly mixed with a pipette and incubated at 0°C. Sodium cyanoborohydride solution (430 μL, 300 mM in reaction buffer, pre-cooled to 0°C, Fluka, Sigma-Aldrich Chemie GmbH, Taufkirchen, D) was added giving a final calculated protein concentration of 5.4 mg/ml and a final HES concentration of 45 % [w/v] after mixing with a pipette and centrifugation. The reaction mixture was incubated for 19 h on ice. A sample of the reaction mixture (10 μg) was analysed by SDS gel electrophoresis. A XCell Sure Lock Mini Cell (Invitrogen GmbH, Karlsruhe, D) and a Consort E143 power supply (CONSORTnv, Turnhout, B) were employed for SDS gel electrophoresis. A 4-12 % Bis/Tris gel together with a MOPS running buffer at reducing conditions (both Invitrogen, Karlsruhe, D) were used according to the manufacturers instruction.

Successful conjugation of EPO to HES was shown by SDS-PAGE analysis (cf. Fig. 7).

**List or Reference**

- Sommermeyer et al., 1987, Krankenhauspharmazie, 8(8), 271-278
- Weidler et al., 1991, Arzneimittelforschung/Drug Res., 41, 494-498
- DE 26 16 086
- Spivak and Hogans, 1989, Blood 73, 90
- McMahon et al., 1990, Blood 76, 1718
- WO 94/28024
- WO 02/080979
- WO 03/074087
- WO 03/074088
- WO 2005/014024
- WO 2005/092390
- WO 2004/024777
- WO 2004/024776
- DE 30 29 307 A1
- WO 2005/092928
- US 2006/0194940 A1
- US 7,157,546 B2
- EP 1 591 467 A1
- WO 2004/022630 A2
- US 6,916,962 B2
- US 6,956,135 B2
- WO 03/049699 A2
- US 5,990,237
- Klemm D. et al, Comprehensive Cellulose Chemistry Vol. 2, 1998, Wiley-VCH, Weinheim, New York, especially chapter 4.4, Esterification of Cellulose (ISBN 3-527-29489-9
- WO 00/66633 A
- WO 00/18893 A
- US 4,454,161
- EP 0 418 945 A
- JP 2001294601 A
- US 2002/065410 A
- K.R. Reddy et al. Advaced Drug Delivery Reviews 54 (2002) pp. 571-586

## Claims

1. A method of producing a hydroxyalkyl starch (HAS) derivative, comprising

a) reacting optionally oxidized hydroxyalkyl starch with a compound (D) comprising at least two functional groups $-O-NH_2$ or a salt thereof, and
b) reacting the hydroxyalkyl starch derivative obtained in step a) with a compound (L) comprising at least two functional groups $W_1$ and $W_2$ independently selected from the group consisting of an aldehyde group, a suitably protected aldehyde group, a keto group, and a suitably protected keto group.

wherein compound (L) used in step b) has the structure according to formula (III)

$$W_1\text{-}R_9\text{-}W_2 \qquad \text{(III)}$$

wherein $R_9$ is selected from a chemical bond, preferably a single bond, a saturated or unsaturated, cyclic or linear, branched or unbranched, substituted or unsubstituted alkylene, possibly containing heteroatoms in the alkylene chain, a substituted or unsubstituted arylene and a substituted or unsubstituted heteroarylene, a substituted or unsubstituted aralkylene, a substituted or unsubstituted alkarylene, and a substituted or unsubstituted heteroarylene, a substituted or unsubstituted heteroaralkylene, and a substituted or unsubstituted alkheteroarylene.

2. The method as claimed in claim 1, wherein said hydroxyalkyl starch has the structure according to formula (I)

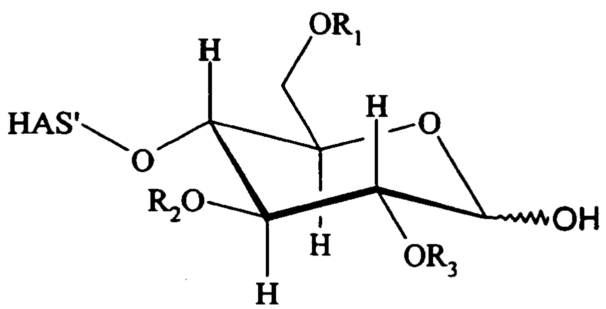

(I)

wherein HAS' is the remainder of the hydroxyalkyl starch molecule and $R_1$, $R_2$ and $R_3$ are independently hydrogen or a linear or branched hydroxyalkyl group.

3. The method as claimed in claim 2, wherein $R_1$, $R_2$ and $R_3$ are independently a group $(CH_2CH_2O)_n$-H, wherein n is an integer, preferably 0, 1, 2, 3, 4, 5, or 6.

4. The method as claimed in any of the preceding claims, wherein the hydroxyalkyl starch is hydroxyethyl starch.

5. The method as claimed in any of the preceding claims, wherein the hydroxyalkyl starch is not oxidized prior to the reaction with compound (D) or a salt thereof in step a).

6. The method as claimed in any of the preceding claims, wherein compound (L) comprises at least two functional groups $W_1$ and $W_2$ independently selected from the group consisting of an aldehyde group, a hemiacetal group, $-CH(OH)_2$, an acetal group, a keto group, and a ketal group,
preferably selected from the group consisting of a hemiacetal group,$-CH(OH)_2$, an acetal group, a ketal group, and the group -C(O)-R, wherein R is selected from the group consisting of hydrogen, a saturated or unsaturated, cyclic or linear, branched or unbranched, substituted or unsubstituted alkyl and a substituted or unsubstituted aryl group, wherein, in case group -C(O)-R is a keto group, the residue R preferably is selected from the group consisting of $C_1$-$C_6$ alkyl and $C_6$-$C_{14}$ aryl, even more preferably selected from the group consisting of optionally substituted, preferably non-substituted methyl, ethyl, n-propyl, isopropyl, n-butyl, isobutyl, and tert-butyl.

7. The method as claimed in any of the preceding claims, wherein the hydroxyalkyl starch is reacted with compound (D) or a salt thereof at a reducing end of the hydroxyalkyl starch, which is not oxidized prior to the reaction in step a).

8. The method as claimed in any of the preceding claims, wherein compound (D) or a salt thereof is reacted via at

least one of the at least two functional groups $-O-NH_2$ with an aldehyde and/or hemiacetal group of the hydroxyalkyl starch in step a).

**9.** The method as claimed in any of the preceding claims, wherein compound (D) or a salt thereof is reacted via at least one of the at least two functional groups $-O-NH_2$ with a reducing end of the hydroxyalkyl starch and wherein the hydroxyalkyl starch is not oxidized prior to the reaction in step a).

**10.** The method as claimed in any of the preceding claims, wherein step a) additionally comprises that the hydroxyalkyl starch derivative obtained is reduced prior to step b).

**11.** The method as claimed in any of the preceding claims, wherein compound (L) is reacted via at least one of the at least two functional groups $W_1$ and $W_2$ with at least one of the functional groups $H_2N-O-$ of the hydroxyalkyl starch derivative obtained in step a) or a salt thereof.

**12.** The method as claimed in any of the preceding claims, wherein compound (D) used in step a) has the structure according to formula (II)

$$H_2N-O-R_4-O-NH_2 \qquad (II)$$

or a salt thereof wherein $R_4$ is selected from a saturated or unsaturated, cyclic or linear, branched or unbranched, substituted or unsubstituted alkylene, possibly containing heteroatoms in the alkylene chain, a substituted or unsubstituted arylene, a substituted or unsubstituted aralkylene, a substituted or unsubstituted alkarylene, and a substituted or unsubstituted heteroarylene, a substituted or unsubstituted heteroaralkylene, and a substituted or unsubstituted alkheteroarylene.

**13.** The method as claimed in claim 12, wherein $R_4$ is selected from the group consisting of $C_1$-$C_{12}$ alkylene, $C_6$-$C_{14}$ arylene, and-$[(CR_5R_6)_mO]_n[CR_7R_8]_o$-, wherein
$R_5$, $R_6$, $R_7$ and $R_8$ are independently of each other selected from the group consisting of hydrogen, alkyl and aryl,
m is 2 to 4;
n is 0 to 20; and
o is 0 to 20, wherein in case n is 0, o is not 0.

**14.** The method of claim 13, wherein $R_4$ in compound (D) is-$[(CR_5R_6)_mO]_n[CR_7R_8]_o$-, wherein $R_5$, $R_6$, $R_7$ and $R_8$ are independently of each other selected from the group consisting of hydrogen, $C_1$-$C_6$ alkyl and $C_6$-$C_{14}$ aryl,
m is 2;
n is 1; and
o is 2.

**15.** The method of claim 14, wherein compound (D) used in step a) is

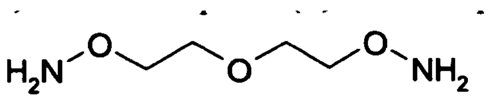

or a salt thereof.

**16.** The method as claimed in any of claims 1 to 15, wherein $R_9$ is a substituted or unsubstituted arylene or substituted or unsubstituted alkylene, in particular wherein $R_9$ is an unsubstituted $C_6$-$C_{14}$ arylene or $-(CH_2)_n-$, with n being preferably 1 - 6, preferably phenylene.

**17.** The method as claimed in claim 16, wherein $R_9$ is an unsubstituted $C_6$-$C_{14}$ arylene.

**18.** The method as claimed in claim 17, wherein compound (L) used in step b) is

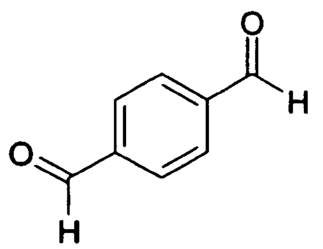

**19.** The method as claimed in any of the preceding claims, wherein step b) additionally comprises that the hydroxyalkyl starch derivative obtained is reduced.

**20.** The method as claimed in any of the preceding claims, wherein the hydroxyalkyl starch derivative obtained in step b) is

c) reacted with at least one biologically active agent.

**21.** The method as claimed in claim 20, wherein the at least one biologically active agent used in step c) comprises at least one functional group $NH_2$.

**22.** The method as claimed in any of claims 20 or 21, wherein the at least one biologically active agent used in step c) is selected from the group consisting of a peptide, polypeptide, a protein and a functional derivative, fragment or mimetic of the polypeptide or protein.

**23.** The method as claimed in claim 22, wherein the polypeptide is selected from the group consisting of erythropoietin (EPO), such as recombinant human EPO (rhEPO) or an EPO mimetic, colony-stimulating factors (CSF), such as G-CSF like recombinant human G-CSF (rhG-CSF), alpha-Interferon (IFN alpha), beta-Interferon (IFN beta) or gamma-Interferon (IFN gamma), such as IFN alpha and IFN beta like recombinant human IFN alpha or IFN beta (rhIFN alpha or rhIFN beta), interleukines, e.g. IL-1 to IL-18 such as IL-2 or IL-3 like recombinant human IL-2 or IL-3 (rhIL-2 or rhIL-3), serum proteins such as coagulation factors II-XIII like factor VIII, factor VII, factor IX, factor II, factor III, factor IV, factor V, factor VI, factor X, factor XI, factor XII, factor XIII, alpha1-antitrypsin (A1AT), activated protein C (APC), plasminogen activators such as tissue-type plasminogen activator (tPA), such as human tissue plasminogen activator (hTPA), AT III such as recombinant human AT III (rhAT III), myoglobin, albumin such as bovine serum albumin (BSA), growth factors, such as epidermal growth factor (EGF), thrombocyte growth factor (PDGF), fibroblast growth factor (FGF), brain-derived growth factor (BDGF), nerve growth factor (NGF), B-cell growth factor (BCGF), brain-derived neurotrophic growth factor (BDNF), ciliary neurotrophic factor (CNTF), transforming growth factors such as TGF alpha or TGF beta, BMP (bone morphogenic proteins), growth hormones such as human growth hormone, tumor necrosis factors such as TNF alpha or TNF beta, somatostatine, somatotropine, somatomedines, hemoglobin, hormones or prohormones such as insulin, gonadotropin, melanocyte-stimulating hormone (alpha-MSH), triptorelin, hypthalamic hormones such as antidiuretic hormones (ADH and oxytocin as well as releasing hormones and release-inhibiting hormones, parathyroid hormone, thyroid hormones such as thyroxine, thyrotropin, thyroliberin, calcitonin, glucagon, glucagon-like peptides (GLP-1, GLP-2 etc.), exendines such as exendin-4, leptin, vasopressin, gastrin, secretin, integrins, glycoprotein hormones (e.g. LH, FSH etc.), melanoside-stimulating hormones, lipoproteins and apo-lipoproteins such as apo-B, apo-E, apo-$L_a$, immunoglobulins such as IgG, IgE, IgM, IgA, IgD and fragments thereof, hirudin, tissue-pathway inhibitor, plant proteins such as lectin or ricin, bee-venom, snake-venom, immunotoxins, antigen E, alpha-proteinase inhibitor, ragweed allergen, melanin, oligolysine proteins, RGD proteins or optionally corresponding receptors for one of these proteins; prolactin or a mutant thereof, such as G129R, in which the wild type amino acid at position 129, glycine is replaced by arginine and a functional derivative or fragment of any of these proteins or receptors.

**24.** The method as claimed in claim 23, wherein the polypeptide is selected from the group consisting of erythropoietin (EPO), such as recombinant human EPO (rhEPO), colony-stimulating factors (CSF), such as recombinant human G-CSF (rhG-CSF), alpha-Interferon (IFN alpha), beta-Interferon (IFN beta), gamma-Interferon (IFN gamma), such as recombinant human IFN alpha or IFN beta (rhIFN alpha or rhIFN beta), A1AT and factor IX.

**25.** The method as claimed in claim 24, wherein the polypeptide is IFN alpha.

**26.** The method as claimed in claim 24, wherein the polypeptide is G-CSF.

**27.** The method as claimed in claim 24, wherein the polypeptide is EPO.

**28.** The method as claimed in any of claims 20 to 27, wherein the hydroxyalkyl starch derivative obtained in step b) is reacted in step c) with the at least one biologically active agent via at least one of the functional groups $W_1$ and $W_2$ introduced into the hydroxyalkyl starch derivative through compound (L) in step b).

**29.** The method as claimed in claim 28, wherein the hydroxyalkyl starch derivative obtained in step b) is reacted in step c) via the at least one functional group $-NH_2$ of the biologically active agent with at least one of the functional groups $W_1$ and $W_2$ introduced into the hydroxyalkyl starch derivative through compound (L) in step b).

**30.** The method as claimed in any of the preceding claims 21 to 29, wherein the at least one functional group $NH_2$ comprised in the at least one biologically active agent used in step c) is the N-terminal functional group $-NH_2$ of a polypeptide or a protein.

**31.** The method as claimed in any of claims 21 to 30, wherein step c) is performed under the reaction conditions for a reductive amination.

**32.** The method as claimed in any of claims 21 to 31, wherein step c) is conducted with a reducing agent, preferably with a borane, in particular with $NaCNBH_3$.

**33.** The method as claimed in any of the preceding claims 20 to 32, wherein in step c) the molar ratio of hydroxyalkyl starch derivative obtained in step b) to biologically active agent is from about 1:1 to about 100:1 equivalents, based on the weight average molecular weight ($M_w$) of the hydroxyalkyl starch derivative obtained in step b).

**34.** The method as claimed in claim 33, wherein the ratio is from about 1:1 to about 20:1.

**35.** The method as claimed in any of claims 31 to 34, wherein the concentration of the hydroxyalkyl starch derivative obtained in step b) used in step c) is higher than about 10 % m/v.

**36.** The method as claimed in any of claims 20 to 35, wherein the temperature in step c) is from about 0 °C to about 25 °C.

**37.** The method as claimed in any of the preceding claims, wherein in step a) hydroxyalkyl starch which is not oxidized is reacted at a reducing end of the hydroxyalkyl starch with compound (D) being

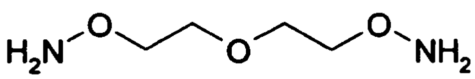

via one of the functional groups $-O-NH_2$, and
in step b) the hydroxyalkyl starch derivative obtained in step b) is reacted via the functional group $-O-NH_2$ with one of the functional groups -CH=O of compound (L) being

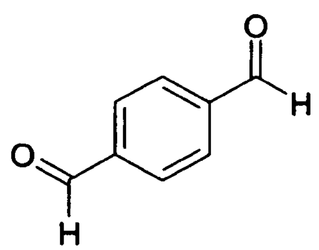

**38.** The method as claimed in claim 37, wherein the hydroxyalkyl starch derivative obtained in step b) is reacted with IFN alpha in step c) under conditions for reductive amination, in particular in the presence of $NaCNBH_3$.

**39.** A hydroxyalkyl starch derivative obtainable by a method as claimed in any of claims 1 to 38.

**40.** A HAS derivative, preferably a HES derivative, according to structure

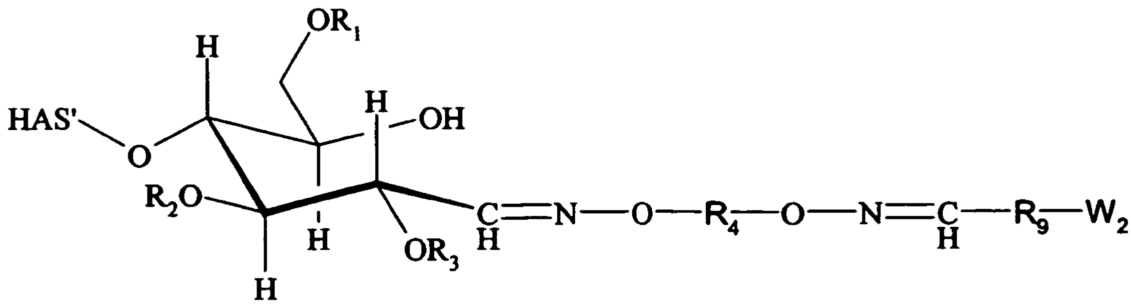

and/or the corresponding ring structure

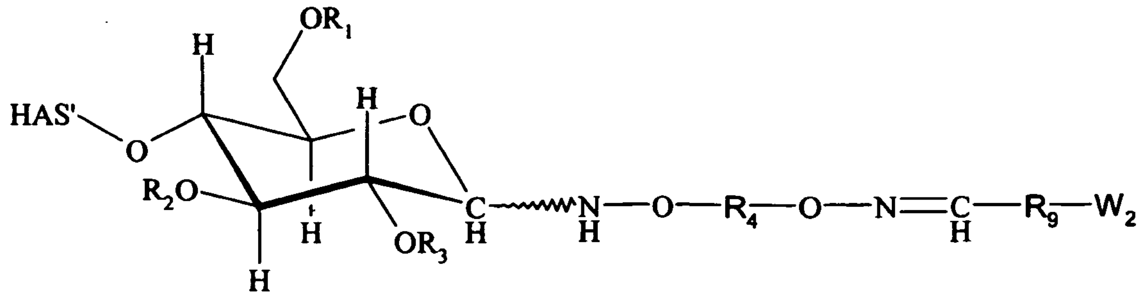

and/or

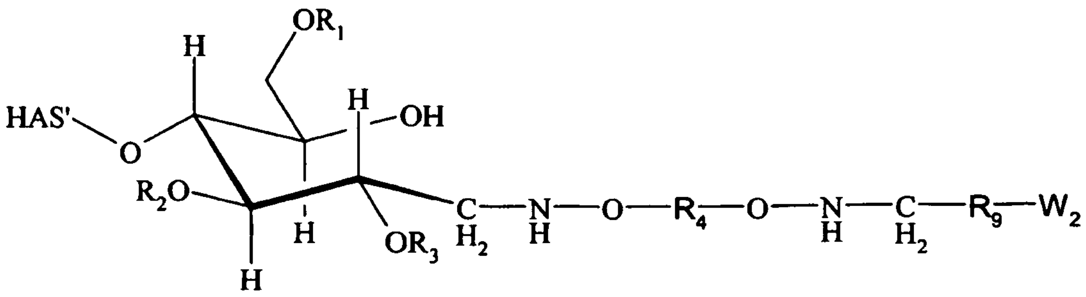

wherein, more preferably, HES has a mean molecular weight from about 1 to about 1000 kDa, more preferably from about 1 to about 800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa, a molar substitution of 0.1 to 3, preferably 0.4 to 1.3, such as 0.4, 0.5, 0.6, 0.7 0.8, 0.9, 1.0, 1.1, 1.2, or 1.3, and a ratio of $C_2$ : $C_6$ substitution of preferably in the range of from 2 to 20, more preferably in the range of from 2 to 15 and even more preferably in the range of from 3 to 12; wherein HAS' is the remainder of the hydroxyalkyl starch molecule and $R_1$, $R_2$ and $R_3$ are independently hydrogen, a linear or branched hydroxyalkyl group or the group

$$-[(CR^1R^2)_mO]_n[CR^3R^4]_o-OH$$

wherein $R^1$, $R^2$, $R^3$, and $R^4$ are independently selected from the group consisting of hydrogen, and alkyl group, preferably hydrogen and methyl group,
m is 2 to 4, wherein the residues $R^1$ and $R^2$ may be the same or different in the m groups $CR^1R^2$;
n is 0 to 20, preferably 0 to 4;
o is 2 to 20, preferably 2 to 4, wherein the residues $R^3$ and $R^4$ may be the same or different in the o groups $CR^3R^4$;
wherein $R_4$ is selected from a saturated or unsaturated, cyclic or linear, branched or unbranched, substituted or unsubstituted alkylene, possibly containing heteroatoms in the alkylene chain, a substituted or unsubstituted arylene, a substituted or unsubstituted aralkylene, a substituted or unsubstituted alkarylene, and a

substituted or unsubstituted heteroarylene, a substituted or unsubstituted heteroaralkylene, and a substituted or unsubstituted alkheteroarylene;
wherein $R_9$ is selected from a chemical bond, preferably a single bond, a saturated or unsaturated, cyclic or linear, branched or unbranched, substituted or unsubstituted alkylene, possibly containing heteroatoms in the alkylene chain, a substituted or unsubstituted arylene and a substituted or unsubstituted heteroarylene, a substituted or unsubstituted aralkylene, a substituted or unsubstituted alkarylene, and a substituted or unsubstituted heteroarylene, a substituted or unsubstituted heteroaralkylene, and a substituted or unsubstituted alkheteroarylene;

and wherein $W_2$ is selected from the group consisting of an aldehyde group, a suitably protected aldehyde group, a keto group, and a suitably protected keto group, preferably selected from the group consisting of an aldehyde group, a hemiacetal group, - $CH(OH)_2$, an acetal group, a keto group, and a ketal group,
preferably selected from the group consisting of a hemiacetal group, $-CH(OH)_2$, an acetal group, a ketal group, and the group -C(O)-R, wherein R is selected from the group consisting of hydrogen, a saturated or unsaturated, cyclic or linear, branched or unbranched, substituted or unsubstituted alkyl and a substituted or unsubstituted aryl group, wherein, in case group -C(O)-R is a keto group, the residue R preferably is selected from the group consisting of $C_1$-$C_6$ alkyl and $C_6$-$C_{14}$ aryl, even more preferably selected from the group consisting of optionally substituted, preferably non-substituted methyl, ethyl, n-propyl, isopropyl, n-butyl, isobutyl, and tert-butyl.

**41.** A HAS derivative, preferably a HES derivative, according to structure

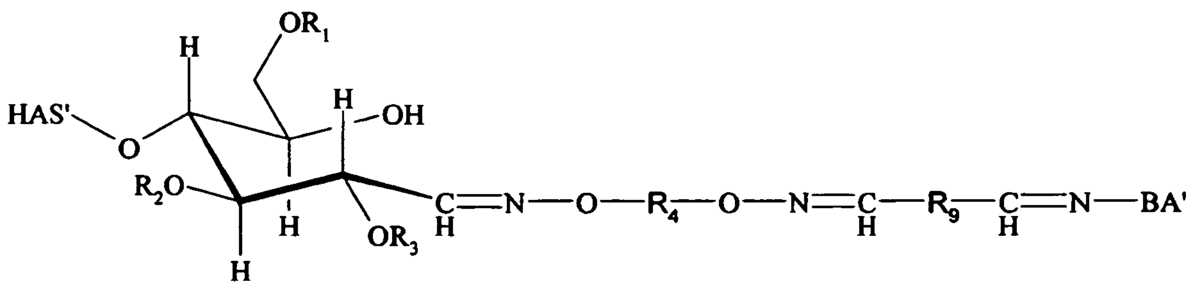

and/or the corresponding ring structure

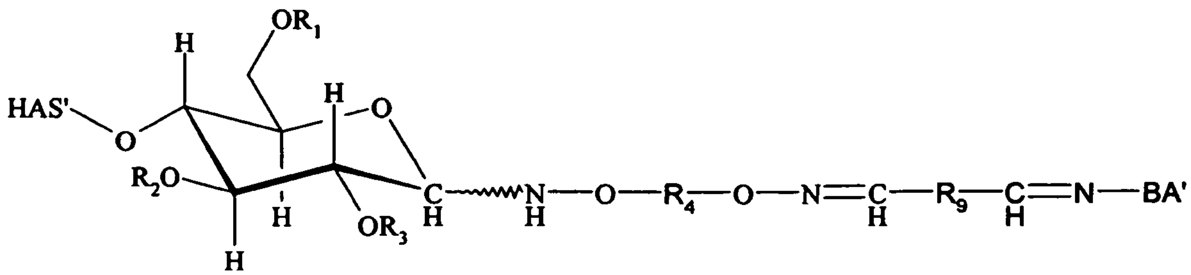

and/or

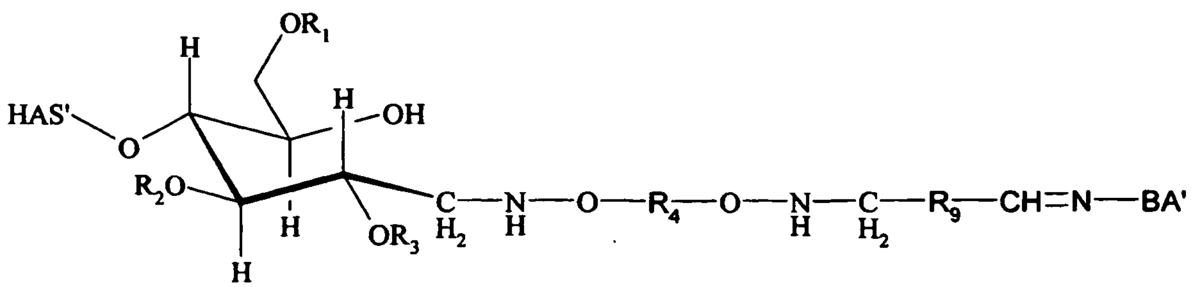

and/or

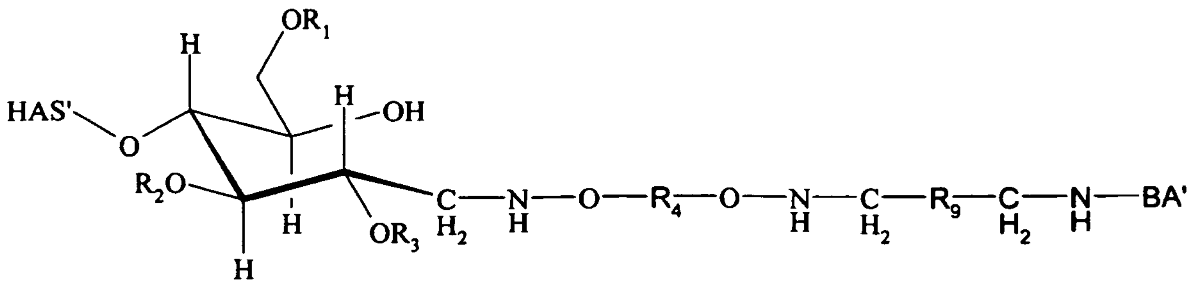

and/or

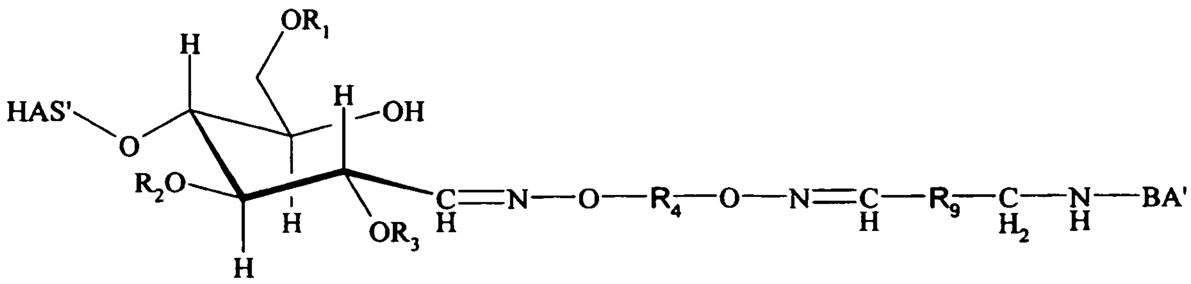

and/or the corresponding ring structure

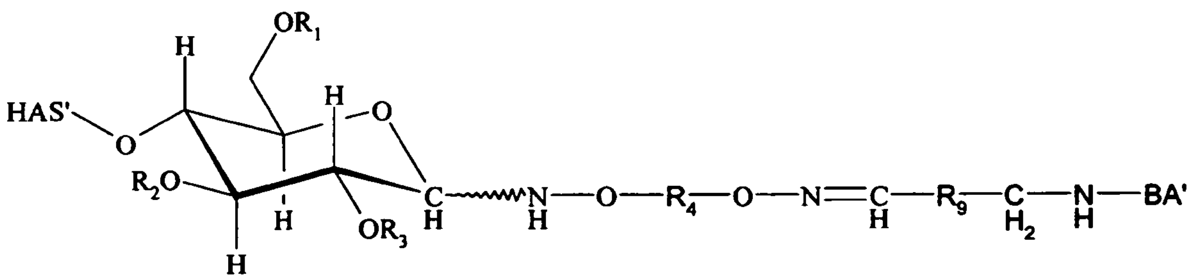

wherein, more preferably, HES has a mean molecular weight from about 1 to about 1000 kDa, more preferably from about 1 to about 800 kDa, more preferably from about 1 to about 500 kDa, more preferably from about 2 to about 400 kDa, more preferably from about 5 to about 300 kDa, more preferably from about 10 to about 200 kDa, in particular from about 50 to about 150 kDa, a molar substitution of 0.1 to 3, preferably 0.4 to 1.3, such as 0.4, 0.5, 0.6, 0.7 0.8, 0.9, 1.0, 1.1, 1.2, or 1.3, and a ratio of $C_2 : C_6$ substitution of preferably in the range of from 2 to 20, more preferably in the range of from 2 to 15 and even more preferably in the range of from 3 to 12; wherein HAS' is the remainder of the hydroxyalkyl starch molecule and $R_1$, $R_2$ and $R_3$ are independently hydrogen, a linear or branched hydroxyalkyl group or the group

$$-[(CR^1R^2)_mO]_n[CR^3R^4]_o-OH$$

wherein $R^1$, $R^2$, $R^3$, and $R^4$ are independently selected from the group consisting of hydrogen, and alkyl group, preferably hydrogen and methyl group,

m is 2 to 4, wherein the residues $R^1$ and $R^2$ may be the same or different in the m groups $CR^1R^2$;

n is 0 to 20, preferably 0 to 4;

o is 2 to 20, preferably 2 to 4, wherein the residues $R^3$ and $R^4$ may be the same or different in the o groups $CR^3R^4$;

wherein $R_4$ is selected from a saturated or unsaturated, cyclic or linear, branched or unbranched, substituted or unsubstituted alkylene, possibly containing heteroatoms in the alkylene chain, a substituted or unsubstituted arylene, a substituted or unsubstituted aralkylene, a substituted or unsubstituted alkarylene, and a

substituted or unsubstituted heteroarylene, a substituted or unsubstituted heteroaralkylene, and a substituted or unsubstituted alkheteroarylene;

wherein $R_9$ is selected from a chemical bond, preferably a single bond, a saturated or unsaturated, cyclic or linear, branched or unbranched, substituted or unsubstituted alkylene, possibly containing heteroatoms in the alkylene chain, a substituted or unsubstituted arylene and a substituted or unsubstituted heteroarylene, a substituted or unsubstituted aralkylene, a substituted or unsubstituted alkarylene, and a substituted or unsubstituted heteroarylene, a substituted or unsubstituted heteroaralkylene, and a substituted or unsubstituted alkheteroarylene;

and wherein BA is a biologically active agent, BA comprising at least one functional group $-NH_2$, BA being represented as $H_2N$-BA', wherein BA' is the remainder of BA, BA preferably being selected from the group consisting of erythropoietin (EPO), such as recombinant human EPO (rhEPO), colony-stimulating factors (CSF), such as recombinant human G-CSF (rhG-CSF), alpha-Interferon (IFN alpha), beta-Interferon (IFN beta), gamma-Interferon (IFN gamma), such as recombinant human IFN alpha or IFN beta (rhIFN alpha or rhIFN beta), A1AT and factor IX,

especially preferably

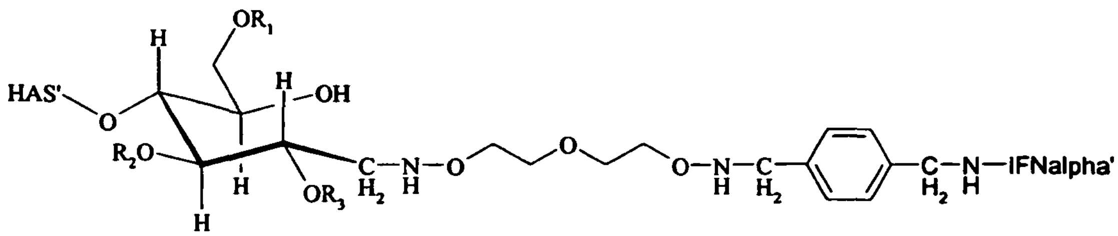

**42.** A pharmaceutical composition comprising, in a therapeutically effective amount, a hydroxyalkyl starch derivative obtainable by a method as claimed in any of claims 20 to 38 or a hydroxyalkyl starch derivative as claimed in claim 41.

**43.** A pharmaceutical composition as claimed in claim 42, wherein the hydroxyalkyl starch derivative is obtainable by a method as claimed in claim 25.

**44.** A hydroxyalkyl starch derivative obtainable by a method as claimed in any of claims 20 to 38, or a hydroxyalkyl starch derivative of claim 41, as a therapeutic or prophylactic agent.

**45.** Use of a hydroxyalkyl starch derivative obtainable by a method as claimed in claim 25 for the preparation of a medicament for the treatment of a disease selected from the group consisting of cancer, such as hairy cell leukaemia, malignant melanoma, follicular lymphoma and/or AIDS related Kaposi's sarcoma, condylomata acuminate, chronic hepatitis B and chronic hepatitis C, preferably chronic hepatitis B and hepatitis C.

**46.** Use of a hydroxyalkyl starch derivative obtainable by a method as claimed in claim 26 for the preparation of a medicament for the treatment of disorders in patients selected from the group consisting of cancer patients, such as cancer patients receiving myelosuppressive chemotherapy, patients with acute myeloid leukaemia receiving induction or consolidation chemotherapy and/or cancer patients receiving marrow bone transplant, patients undergoing peripheral blood progenitor cell collection and therapy, and patients with severe chronic neutropenia.

**47.** Use of a hydroxyalkyl starch derivative obtainable by a method as claimed in claim 27 for the preparation of a medicament for the treatment of anemia, such as of chronic renal failure patients, Zidovudine-treated HIV-infected patients, cancer patients on chemotherapy, or for the reduction of allogeneic blood transfusion in surgery patients.

**48.** A hydroxyalkyl starch derivative as obtainable according to the method as claimed in any one of claims 25 to 27 for the treatment of disorders in patients selected from the group consisting of cancer patients, such as cancer patients receiving myelosuppressive chemotherapy, patients with acute myeloid leukaemia receiving induction or consolidation chemotherapy and/or cancer patients receiving marrow bone transplant, patients undergoing peripheral blood progenitor cell collection and therapy, and patients with severe chronic neutropenia; anemia, such as of chronic renal failure patients, Zidovudine-treated HIV-infected patients, cancer patients on chemotherapy, or for the reduction of allogeneic blood transfusion in surgery patients.

## Patentansprüche

1. Verfahren zur Herstellung eines Hydroxyalkylstärke-(HAS-) Derivats, umfassend

   a) Umsetzen gegebenenfalls oxidierter Hydroxyalkylstärke mit einer wenigstens zwei funktionelle Gruppen -O-$NH_2$ umfassenden Verbindung (D) oder einem Salz davon und
   b) Umsetzen des in Schritt a) erhaltenen Hydroxyalkylstärkederivats mit einer wenigstens zwei unabhängig aus der aus einer Aldehydgruppe, einer in geeigneter Weise geschützten Aldehydgruppe, einer Ketogruppe und einer in geeigneter Weise geschützten Ketogruppe bestehenden Gruppe ausgewählte funktionelle Gruppen $W_1$ und $W_2$ umfassenden Verbindung (L),

   wobei die in Schritt b) eingesetzte Verbindung (L) die Struktur gemäß der Formel (III)

   $$W_1\text{-}R_9\text{-}W_2 \qquad \text{(III)}$$

   aufweist, wobei $R_9$ aus einer chemischen Bindung, vorzugsweise einer Einfachbindung, einem gesättigten oder ungesättigten, cyclischen oder linearen, verzweigten oder unverzweigten, substituierten oder unsubstituierten Alkylen, gegebenenfalls mit Heteroatomen in der Alkylenkette, einem substituierten oder unsubstituierten Arylen und einem substituierten oder unsubstituierten Heteroarylen, einem substituierten oder unsubstituierten Aralkylen, einem substituierten oder unsubstituierten Alkarylen sowie einem substituierten oder unsubstituierten Heteroarylen, einem substituierten oder unsubstituierten Heteroaralkylen und einem substituierten oder unsubstituierten Alkheteroarylen ausgewählt ist.

2. Verfahren nach Anspruch 1, wobei die Hydroxyalkylstärke die Struktur gemäß der Formel (I)

   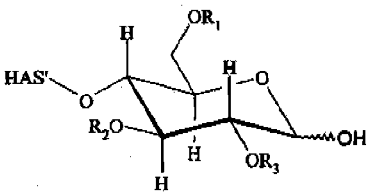
   (I)

   aufweist, wobei HAS' für den Rest des Hydroxyalkylstärkemoleküls steht und $R_1$, $R_2$ und $R_3$ unabhängig für Wasserstoff oder eine lineare oder verzweigte Hydroxyalkylgruppe stehen.

3. Verfahren nach Anspruch 2, wobei $R_1$, $R_2$ und $R_3$ unabhängig für eine Gruppe $(CH_2CH_2O)_n$-H stehen, wobei n für eine ganze Zahl, vorzugsweise 0, 1, 2, 3, 4, 5 oder 6, steht.

4. Verfahren nach einem der vorhergehenden Ansprüche, wobei es sich bei der Hydroxyalkylstärke um Hydroxyethylstärke handelt.

5. Verfahren nach einem der vorhergehenden Ansprüche, wobei die Hydroxyalkylstärke vor der Reaktion mit Verbindung (D) oder einem Salz davon in Schritt a) nicht oxidiert wird.

6. Verfahren nach einem der vorhergehenden Ansprüche, wobei Verbindung (L) wenigstens zwei unabhängig aus der aus einer Aldehydgruppe, einer Halbacetalgruppe, $-CH(OH)_2$, einer Acetalgruppe, einer Ketogruppe und einer Ketalgruppe bestehenden Gruppe ausgewählte,
   vorzugsweise aus der aus einer Halbacetalgruppe, $-CH(OH)_2$, einer Acetalgruppe, einer Ketalgruppe und der Gruppe -C(O)-R bestehenden Gruppe ausgewählte funktionelle Gruppen $W_1$ und $W_2$ enthält, wobei R aus der aus Wasserstoff, einem gesättigten oder ungesättigten, cyclischen oder linearen, verzweigten oder unverzweigten, substituierten oder unsubstituierten Alkyl und einer substituierten oder unsubstituierten Arylgruppe bestehenden Gruppe ausgewählt ist, wobei, falls es sich bei der Gruppe -C(O)-R um eine Ketogruppe handelt, der Rest R vorzugsweise aus der aus $C_1$-$C_6$-Alkyl und $C_6$-$C_{14}$-Aryl bestehenden Gruppe, noch stärker bevorzugt aus der aus gegebenenfalls substituiertem, vorzugsweise nicht substituiertem Methyl, Ethyl, n-Propyl, Isopropyl, n-Butyl, Isobutyl und tert.-Butyl bestehenden Gruppe, ausgewählt ist.

7. Verfahren nach einem der vorhergehenden Ansprüche, wobei die Hydroxyalkylstärke mit Verbindung (D) oder einem

Salz davon an einem reduzierenden Ende der Hydroxyalkylstärke, die vor der Reaktion in Schritt a) nicht oxidiert wird, umgesetzt wird.

**8.** Verfahren nach einem der vorhergehenden Ansprüche, wobei Verbindung (D) oder ein Salz davon über wenigstens eine der wenigstens zwei funktionellen Gruppen $-O-NH_2$ mit einer Aldehyd- und/oder Halbacetalgruppe der Hydroxyalkylstärke in Schritt a) umgesetzt wird.

**9.** Verfahren nach einem der vorhergehenden Ansprüche, wobei Verbindung (D) oder ein Salz davon über wenigstens eine der wenigstens zwei funktionellen Gruppen $-O-NH_2$ mit einem reduzierenden Ende der Hydroxyalkylstärke umgesetzt wird und wobei die Hydroxyalkylstärke vor der Reaktion in Schritt a) nicht oxidiert wird.

**10.** Verfahren nach einem der vorhergehenden Ansprüche, wobei Schritt a) zusätzlich umfasst, dass das erhaltene Hydroxyalkylstärkederivat vor Schritt b) reduziert wird.

**11.** Verfahren nach einem der vorhergehenden Ansprüche, wobei Verbindung (L) über wenigstens eine der wenigstens zwei funktionellen Gruppen $W_1$ und $W_2$ mit wenigstens einer der funktionellen Gruppen $H_2N-O-$des in Schritt a) erhaltenen Hydroxyalkylstärkederivats oder einem Salz davon umgesetzt wird.

**12.** Verfahren nach einem der vorhergehenden Ansprüche, wobei die in Schritt a) eingesetzte Verbindung (D) die Struktur gemäß der Formel (II)

$$H_2N-O-R_4-O-NH_2 \qquad (II)$$

oder eines Salzes davon aufweist, wobei $R_4$ aus einem gesättigten oder ungesättigten, cyclischen oder linearen, verzweigten oder unverzweigten, substituierten oder unsubstituierten Alkylen, gegebenenfalls mit Heteroatomen in der Alkylenkette, einem substituierten oder unsubstituierten Arylen, einem substituierten oder unsubstituierten Aralkylen, einem substituierten oder unsubstituierten Alkarylen sowie einem substituierten oder unsubstituierten Heteroarylen, einem substituierten oder unsubstituierten Heteroaralkylen und einem substituierten oder unsubstituierten Alkheteroarylen ausgewählt ist.

**13.** Verfahren nach Anspruch 12, wobei $R_4$ aus der aus $C_1$-$C_{12}$-Alkylen, $C_6$-$C_{14}$-Arylen und $-[(CR_5R_6)_nO]_n[CR_7R_8]_o-$ bestehenden Gruppe ausgewählt ist, wobei $R_5$, $R_6$, $R_7$ und $R_8$ unabhängig voneinander aus der aus Wasserstoff, Alkyl und Aryl bestehenden Gruppe ausgewählt sind,
m gleich 2 bis 4 ist;
n gleich 0 bis 20 ist; und
o gleich 0 bis 20 ist, wobei, falls n gleich 0 ist, o ungleich 0 ist.

**14.** Verfahren nach Anspruch 13, wobei $R_4$ in Verbindung (D) für $-[(CR_5R_6)_mO]_n[CR_7R_8]_o-$ steht, wobei $R_5$, $R_6$, $R_7$ und $R_8$ unabhängig voneinander aus der aus Wasserstoff, $C_1$-$C_6$-Alkyl und $C_1$-$C_{14}$-Aryl bestehenden Gruppe ausgewählt sind,
m gleich 2 ist;
n gleich 1 ist; und
o gleich 2 ist.

**15.** Verfahren nach Anspruch 14, wobei es sich bei der in Schritt a) eingesetzten Verbindung (D) um

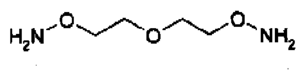

oder ein Salz davon handelt.

**16.** Verfahren nach einem der Ansprüche 1 bis 15, wobei $R_9$ für ein substituiertes oder unsubstituiertes Arylen oder substituiertes oder unsubstituiertes Alkylen steht, insbesondere wobei $R_9$ für ein unsubstituiertes $C_6$-$C_{14}$-Arylen oder $-(CH_2)_n-$, vorzugsweise mit n gleich 1-6, vorzugsweise Phenylen, steht.

**17.** Verfahren nach Anspruch 16, wobei $R_9$ für ein unsubstituiertes $C_6$-$C_{14}$-Arylen steht.

**18.** Verfahren nach Anspruch 17, wobei es sich bei der in Schritt b) eingesetzten Verbindung (L) um

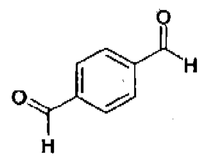

handelt.

**19.** Verfahren nach einem der vorhergehenden Ansprüche, wobei Schritt b) zusätzlich umfasst, dass das erhaltene Hydroxyalkylstärkederivat reduziert wird.

**20.** Verfahren nach einem der vorhergehenden Ansprüche, wobei das in Schritt b) erhaltene Hydroxyalkylstärkederivat

c) mit wenigstens einem biologisch aktiven Agens umgesetzt wird.

**21.** Verfahren nach Anspruch 20, wobei das wenigstens eine in Schritt c) verwendete biologisch aktive Agens wenigstens eine funktionelle Gruppe $-NH_2$ umfasst.

**22.** Verfahren nach einem der Ansprüche 20 oder 21, wobei das wenigstens eine in Schritt c) verwendete biologisch aktive Agens aus der aus einem Peptid, Polypeptid, einem Protein sowie einem funktionellen Derivat, Fragment oder Mimetikum des Polypeptids oder Proteins bestehenden Gruppe ausgewählt ist.

**23.** Verfahren nach Anspruch 22, wobei das Polypeptid aus der aus Erythropoetin (EPO), wie etwa rekombinantem menschlichem EPO (rhEPO) oder einem EPO-Mimetikum, koloniestimulierenden Faktoren (CSF), wie etwa G-CSF wie rekombinantem menschlichem G-CSF (rhG-CSF), Alpha-Interferon (IFN-alpha), Beta-Interferon (IFN-beta) oder Gamma-Interferon (IFN-gamma), wie etwa IFN-alpha bzw. IFN-beta wie rekombinantem menschlichem IFN-alpha bzw. IFN-beta (rhIFN-alpha bzw. rhIFN-beta), Interleukinen, z.B. IL-1 bis IL-18, wie etwa IL-2 bzw. IL-3 wie rekombinantem menschlichem IL-2 bzw. IL-3 (rhIL-2 bzw. rhIL-3), Serumproteinen, wie etwa Gerinnungsfaktoren II-XIII wie Faktor VIII, Faktor VII, Faktor IX, Faktor II, Faktor III, Faktor IV, Faktor V, Faktor VI, Faktor X, Faktor XI, Faktor XII, Faktor XIII, Alphal-Antitrypsin (A1AT), aktiviertem Protein C (APC), Plasminogenaktivatoren, wie etwa Gewebetyp-Plasminogenaktivator (tPA), wie etwa menschlichem Gewebe-Plasminogenaktivator (hTPA), AT III, wie etwa rekombinantem menschlichem AT III (rhAT III), Myoglobin, Albumin, wie etwa Rinderserumalbumin (BSA), Wachstumsfaktoren, wie etwa epidermalem Wachstumsfaktor (EGF), Thrombozyten-Wachstums faktor (PDGF), Fibroblasten-Wachstumsfaktor (FGF), aus Gehirn stammendem Wachstumsfaktor (BDGF), Nervenwachstumsfaktor (NGF), B-Zellen-Wachstumsfaktor (BCGF), aus Gehirn stammendem neurotrophem Wachstumsfaktor (BDNF), ciliärem neurotrophem Faktor (CNTF), transformierenden Wachstumsfaktoren, wie etwa TGF-alpha oder TGF-beta, BMP (bone morphogenetic proteins, knochenbildenden Proteinen), Wachstumshormonen, wie etwa menschlichem Wachstumshormon, Tumornekrosefaktoren, wie etwa TNF-alpha oder TNF-beta, Somatostatin, Somatotropin, Somatomedinen, Hämoglobin, Hormonen oder Prohormonen, wie etwa Insulin, Gonadotropin, Melanozyten stimulierendem Hormon (Alpha-MSH), Triptorelin, Hypothalamushormonen, wie etwa antidiuretischen Hormonen (ADH) und Oxytocin ebenso wie freisetzenden Hormonen und freisetzungshemmenden Hormonen, Parathormon, Schilddrüsenhormonen, wie etwa Thyroxin, Thyrotropin, Thyroliberin, Calcitonin, Glucagon, glucagonähnlichen Peptiden (GLP-1, GLP-2 usw.), Exendinen, wie etwa Exendin-4, Leptin, Vasopressin, Gastrin, Secretin, Integrinen, Glykoproteinhormonen (z.B. LH, FSH usw.), melanozytenstimulierenden Hormonen, Lipoproteinen und Apolipoproteinen, wie etwa Apo-B, Apo-E, Apo-$L_a$, Immunglobulinen, wie etwa IgG, IgE, IgM, IgA, IgD, und Fragmenten davon, Hirudin, Gewebeweg-Inhibitor, Pflanzenproteinen, wie etwa Lectin oder Ricin, Bienengift, Schlangengift, Immunotoxinen, Antigen E, Alpha-Proteinase-Inhibitor, Traubenkrautallergen, Melanin, Oligolysinproteinen, RGD-Proteinen oder gegebenenfalls entsprechenden Rezeptoren für eines dieser Proteine; Prolactin oder einer Mutante davon, wie etwa G129R, bei der die Wildtypaminosäure in Position 129, Glycin, durch Arginin ersetzt ist, sowie einem funktionellen Derivat oder Fragment eines dieser Proteine oder Rezeptoren bestehenden Gruppe ausgewählt ist.

**24.** Verfahren nach Anspruch 23, wobei das Polypeptid aus der aus Erythropoetin (EPO), wie etwa rekombinantem menschlichem EPO (rhEPO), koloniestimulierenden Faktoren (CSF), wie etwa rekombinantem menschlichem G-CSF (rhG-CSF), Alpha-Interferon (IFN-alpha), Beta-Interferon (IFN-beta), Gamma-Interferon (IFN-gamma), wie etwa rekombinantem menschlichem IFN-alpha bzw. IFN-beta (rhIFN-alpha bzw. rhIFN-beta), A1AT und Faktor IX

bestehenden Gruppe ausgewählt ist.

**25.** Verfahren nach Anspruch 24, wobei es sich bei dem Polypeptid um IFN-alpha handelt.

**26.** Verfahren nach Anspruch 24, wobei es sich bei dem Polypeptid um G-CSF handelt.

**27.** Verfahren nach Anspruch 24, wobei es sich bei dem Polypeptid um EPO handelt.

**28.** Verfahren nach einem der Ansprüche 20 bis 27, wobei das in Schritt b) erhaltene Hydroxyalkylstärkederivat in Schritt c) mit dem wenigstens einen biologisch aktiven Agens über wenigstens eine der in das Hydroxyalkylstärkederivat durch Verbindung (L) in Schritt b) eingeführten funktionellen Gruppen $W_1$ und $W_2$ umgesetzt wird.

**29.** Verfahren nach Anspruch 28, wobei das in Schritt b) erhaltene Hydroxyalkylstärkederivat in Schritt c) über die wenigstens eine funktionelle Gruppe - $NH_2$ des biologisch aktiven Agens mit wenigstens einer der in das Hydroxyalkylstärkederivat durch Verbindung (L) in Schritt b) eingeführten funktionellen Gruppen $W_1$ und $W_2$ umgesetzt wird.

**30.** Verfahren nach einem der vorhergehenden Ansprüche 21 bis 29, wobei es sich bei der wenigstens einen in dem wenigstens einen in Schritt c) verwendeten biologisch aktiven Agens enthaltenen funktionellen Gruppe $-NH_2$ um die N-terminale funktionelle Gruppe $-NH_2$ eines Polypeptids oder eines Proteins handelt.

**31.** Verfahren nach einem der Ansprüche 21 bis 30, wobei Schritt c) unter den Reaktionsbedingungen für eine reduktive Aminierung durchgeführt wird.

**32.** Verfahren nach einem der Ansprüche 21 bis 31, wobei Schritt c) mit einem Reduktionsmittel, vorzugsweise mit einem Boran, insbesondere mit $NaCNBH_3$, ausgeführt wird.

**33.** Verfahren nach einem der vorhergehenden Ansprüche 20 bis 32, wobei in Schritt c) das Molverhältnis von in Schritt b) erhaltenem Hydroxyalkylstärkederivat zu biologisch aktivem Agens etwa 1:1 bis etwa 100:1 Äquivalente, bezogen auf das gewichtsgemittelte Molekulargewicht ($M_w$) des in Schritt b) erhaltenen Hydroxyalkylstärkederivats, beträgt.

**34.** Verfahren nach Anspruch 33, wobei das Verhältnis etwa 1:1 bis etwa 20:1 beträgt.

**35.** Verfahren nach einem der Ansprüche 31 bis 34, wobei die in Schritt c) verwendete Konzentration des in Schritt b) erhaltenen Hydroxyalkylstärkederivats höher als etwa 10% m/v ist.

**36.** Verfahren nach einem der Ansprüche 20 bis 35, wobei die Temperatur in Schritt c) bei etwa 0 °C bis etwa 25 °C liegt.

**37.** Verfahren nach einem der vorhergehenden Ansprüche, wobei in Schritt a) Hydroxyalkylstärke, die nicht oxidiert ist, an einem reduzierenden Ende der Hydroxyalkylstärke mit Verbindung (D), bei der es sich um

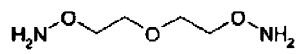

handelt, über eine der funktionellen Gruppen $-O-NH_2$ und in Schritt b) das in Schritt b) erhaltene Hydroxyalkylstärkederivat über die funktionelle Gruppe $-O-NH_2$ mit einer der funktionellen Gruppen -CH=O der Verbindung (L), bei der es sich um

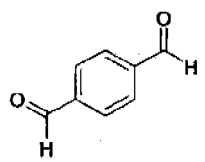

handelt, umgesetzt wird.

**38.** Verfahren nach Anspruch 37, wobei das in Schritt b) erhaltene Hydroxyalkylstärkederivat mit IFN-alpha in Schritt

c) unter Bedingungen für reduktive Aminierung, insbesondere in Gegenwart von $NaCNBH_3$, umgesetzt wird.

**39.** Hydroxyalkylstärkederivat, erhältlich mit einem Verfahren nach einem der Ansprüche 1 bis 38.

**40.** HAS-Derivat, vorzugsweise HES-Derivat, gemäß der Struktur

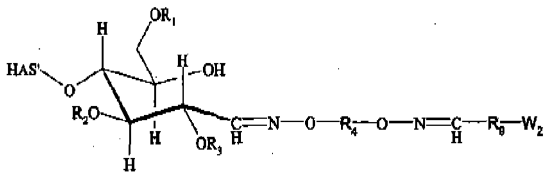

und/oder der entsprechenden Ringstruktur

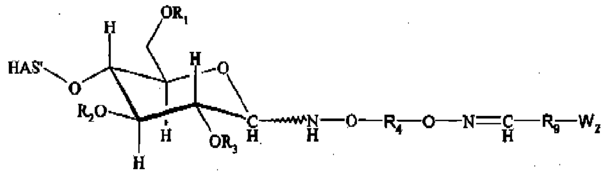

und/oder

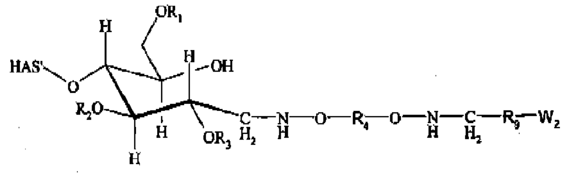

wobei stärker bevorzugt HES ein mittleres Molekulargewicht von etwa 1 bis etwa 1000 kDa, stärker bevorzugt von etwa 1 bis etwa 800 kDa, stärker bevorzugt von etwa 1 bis etwa 500 kDa, stärker bevorzugt von etwa 2 bis etwa 400 kDa, stärker bevorzugt von etwa 5 bis etwa 300 kDa, stärker bevorzugt von etwa 10 bis etwa 200 kDa, insbesondere von etwa 50 bis etwa 150 kDa, eine molare Substitution von 0,1 bis 3, vorzugsweise 0,4 bis 1,3, wie etwa 0,4, 0,5, 0,6, 0,7, 0,8, 0,9, 1,0, 1,1, 1,2 oder 1,3, und ein C2:C6-Substitutionsverhältnis vorzugsweise im Bereich von 2 bis 20, stärker bevorzugt im Bereich von 2 bis 15 und noch stärker bevorzugt im Bereich von 3 bis 12 aufweist;
wobei HAS‘ für den Rest des Hydroxyalkylstärkemoleküls steht und $R_1$, $R_2$ und $R_3$ unabhängig für Wasserstoff, eine lineare oder verzweigte Hydroxyalkylgruppe oder die Gruppe

$$-[(CR^1R^2)_mO]_n[CR^3R^4]_o\text{-OH}$$

stehen, wobei $R^1$, $R^2$, $R^3$ und $R^4$ unabhängig aus der aus Wasserstoff und Alkylgruppe, vorzugsweise Wasserstoff und Methylgruppe, bestehenden Gruppe ausgewählt sind,
m gleich 2 bis 4 ist, wobei die Reste $R^1$ und $R^2$ in den m Gruppen $CR^1R^2$ gleich oder verschieden sein können;
n gleich 0 bis 20, vorzugsweise 0 bis 4 ist;
o gleich 2 bis 20, vorzugsweise 2 bis 4 ist, wobei die Reste $R^3$ und $R^4$ in den o Gruppen $CR^3R^4$ gleich oder verschieden sein können;
wobei $R_4$ aus einem gesättigten oder ungesättigten, cyclischen oder linearen, verzweigten oder unverzweigten, substituierten oder unsubstituierten Alkylen, gegebenenfalls mit Heteroatomen in der Alkylenkette, einem substituierten oder unsubstituierten Arylen, einem substituierten oder unsubstituierten Aralkylen, einem substituierten oder unsubstituierten Alkarylen sowie einem substituierten oder unsubstituierten Heteroarylen, einem substituierten oder unsubstituierten Heteroaralkylen und einem substituierten oder unsubstituierten Alkheteroarylen ausgewählt ist;
wobei $R_9$ aus einer chemischen Bindung, vorzugsweise einer Einfachbindung, einem gesättigten oder ungesättigten, cyclischen oder linearen, verzweigten oder unverzweigten, substituierten oder unsubstituierten Alkylen, gegebenenfalls mit Heteroatomen in der Alkylenkette, einem substituierten oder unsubstituierten Arylen und einem substituierten oder unsubstituierten Heteroarylen, einem substituierten oder unsubstituierten Aralkylen, einem substituierten oder unsubstituierten Alkarylen sowie einem substituierten oder unsubstituierten Heteroarylen, einem substituierten oder unsubstituierten Heteroaralkylen und einem substituierten oder unsubstituierten Alkheteroarylen ausgewählt ist;
und wobei $W_2$ aus der aus einer Aldehydgruppe, einer in geeigneter Weise geschützten Aldehydgruppe, einer Ketogruppe und einer in geeigneter Weise geschützten Ketogruppe bestehenden Gruppe, vorzugsweise aus der aus einer Aldehydgruppe, einer Halbacetalgruppe, $-CH(OH)_2$, einer Acetalgruppe, einer Ketogruppe und einer Ketalgruppe bestehenden Gruppe,
vorzugsweise aus der aus einer Halbacetalgruppe, $-CH(OH)_2$, einer Acetalgruppe, einer Ketalgruppe und der Gruppe -C(O)-R bestehenden Gruppe, ausgewählt ist, wobei R aus der aus Wasserstoff, einem gesättigten oder ungesättigten, cyclischen oder linearen, verzweigten oder unverzweigten, substituierten oder unsubstituierten Alkyl und einer substituierten oder unsubstituierten Arylgruppe bestehenden Gruppe ausgewählt ist, wobei, falls es sich bei der Gruppe -C(O)-R um eine Ketogruppe handelt, der Rest R vorzugsweise aus der aus $C_1$-$C_6$-Alkyl und $C_6$-$C_{14}$-Aryl bestehenden Gruppe, noch stärker bevorzugt aus der aus gegebenenfalls substituiertem, vorzugsweise nicht substituiertem Methyl, Ethyl, n-Propyl, Isopropyl, n-Butyl, Isobutyl und tert.-Butyl bestehenden Gruppe, ausgewählt ist.

**41.** HAS-Derivat, vorzugsweise HES-Derivat, gemäß der Struktur

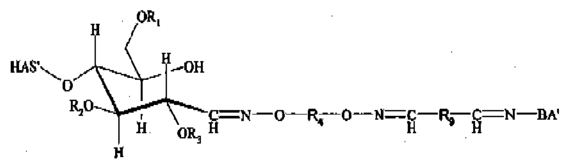

und/oder der entsprechenden Ringstruktur

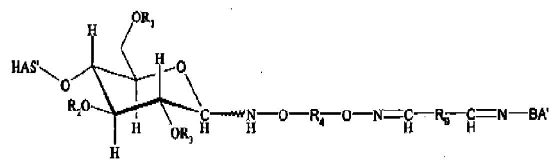

und/oder

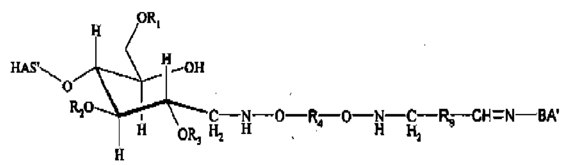

und/oder

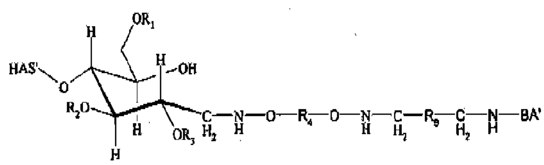

und/oder

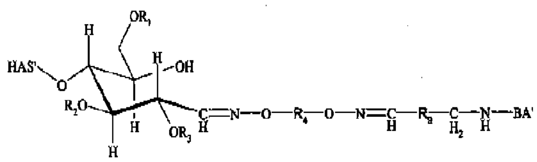

und/oder der entsprechenden Ringstruktur

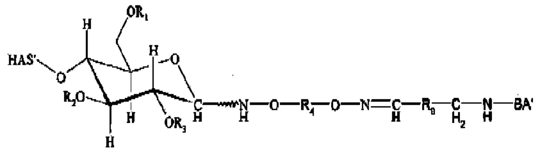

wobei stärker bevorzugt HES ein mittleres Molekulargewicht von etwa 1 bis etwa 1000 kDa, stärker bevorzugt von etwa 1 bis etwa 800 kDa, stärker bevorzugt von etwa 1 bis etwa 500 kDa, stärker bevorzugt von etwa 2 bis etwa 400 kDa, stärker bevorzugt von etwa 5 bis etwa 300 kDa, stärker bevorzugt von etwa 10 bis etwa 200 kDa, insbesondere von etwa 50 bis etwa 150 kDa, eine molare Substitution von 0,1 bis 3, vorzugsweise 0,4 bis 1,3, wie etwa 0,4, 0,5, 0,6, 0,7, 0,8, 0,9, 1,0, 1,1, 1,2 oder 1,3, und ein C2:C6-Substitutionsverhältnis vorzugsweise im Bereich von 2 bis 20, stärker bevorzugt im Bereich von 2 bis 15 und noch stärker bevorzugt im Bereich von 3 bis 12 aufweist;
wobei HAS' für den Rest des Hydroxyalkylstärkemoleküls steht und $R_1$, $R_2$ und $R_3$ unabhängig für Wasserstoff, eine lineare oder verzweigte Hydroxyalkylgruppe oder die Gruppe

$$-[(CR^1R^2)_mO]_n[CR^3R^4]_o\text{-OH}$$

stehen, wobei $R^1$, $R^2$, $R^3$ und $R^4$ unabhängig aus der aus Wasserstoff und Alkylgruppe, vorzugsweise Wasserstoff und Methylgruppe, bestehenden Gruppe ausgewählt sind,
m gleich 2 bis 4 ist, wobei die Reste $R^1$ und $R^2$ in den m Gruppen $CR^1R^2$ gleich oder verschieden sein können;
n gleich 0 bis 20, vorzugsweise 0 bis 4 ist;
o gleich 2 bis 20, vorzugsweise 2 bis 4 ist, wobei die Reste $R^3$ und $R^4$ in den o Gruppen $CR^3R^4$ gleich oder verschieden sein können;
wobei $R_4$ aus einem gesättigten oder ungesättigten, cyclischen oder linearen, verzweigten oder unverzweigten, substituierten oder unsubstituierten Alkylen, gegebenenfalls mit Heteroatomen in der Alkylenkette, einem substituierten oder unsubstituierten Arylen, einem substituierten oder unsubstituierten Aralkylen, einem substituierten oder unsubstituierten Alkarylen sowie einem substituierten oder unsubstituierten Heteroarylen, einem substituierten oder unsubstituierten Heteroaralkylen und einem substituierten oder unsubstituierten Alkheteroarylen ausgewählt ist;
wobei $R_9$ aus einer chemischen Bindung, vorzugsweise einer Einfachbindung, einem gesättigten oder ungesättigten, cyclischen oder linearen, verzweigten oder unverzweigte, substituierten oder unsubstituierten Alkylen, gegebenenfalls mit Heteroatomen in der Alkylenkette, einem substituierten oder unsubstituierten Arylen und einem substituierten oder unsubstituierten Heteroarylen, einem substituierten oder unsubstituierten Aralkylen, einem substituierten oder unsubstituierten Alkarylen sowie einem substituierten oder unsubstituierten Heteroarylen, einem substituierten oder unsubstituierten Heteroaralkylen und einem substituierten oder unsubstituierten Alkheteroarylen ausgewählt ist;
und wobei BA für ein biologisch aktives Agens steht, wobei BA wenigstens eine funktionelle Gruppe $-NH_2$ umfasst, wobei BA als $H_2N$-BA' dargestellt ist, wobei BA' für den Rest von BA steht, wobei BA vorzugsweise aus der aus Erythropoetin (EPO), wie etwa rekombinantem menschlichem EPO (rhEPO), koloniestimulierenden Faktoren (CSF), wie etwa rekombinantem menschlichem G-CSF (rhG-CSF), Alpha-Interferon (IFN-alpha), Beta-Interferon (IFN-beta), Gamma-Interferon (IFN-gamma), wie etwa rekombinantem menschlichem IFN-alpha bzw. IFN-beta (rhIFN-alpha bzw. rhIFN-beta), A1AT und Faktor IX bestehenden Gruppe ausgewählt ist,
ganz besonders bevorzugt

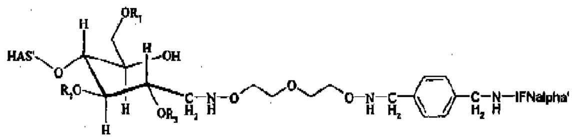

**42.** Pharmazeutische Zusammensetzung, umfassend in einer therapeutisch wirksamen Menge ein mit einem Verfahren nach einem der Ansprüche 20 bis 38 erhältliches Hydroxyalkylstärkederivat oder ein Hydroxyalkylstärkederivat nach Anspruch 41.

**43.** Pharmazeutische Zusammensetzung nach Anspruch 42, wobei das Hydroxyalkylstärkederivat mit einem Verfahren nach Anspruch 25 erhältlich ist.

**44.** Hydroxyalkylstärkederivat, erhältlich mit einem Verfahren nach einem der Ansprüche 20 bis 38 oder Hydroxyalkylstärkederivat nach Anspruch 41, als Therapeutikum oder Prophylaktikum.

**45.** Verwendung eines mit einem Verfahren nach Anspruch 25 erhältlichen Hydroxyalkylstärkederivats zur Herstellung eines Arzneimittels zur Behandlung einer aus der aus Krebs, wie etwa Haarzellleukämie, malignem Melanom, follikulärem Lymphom und/oder mit AIDS assoziiertem Kaposi-Sarkom, Condylomata acuminata, chronischer Hepatitis B und chronischer Hepatitis C, vorzugsweise chronischer Hepatitis B und C, bestehenden Gruppe ausgewählten Krankheit.

**46.** Verwendung eines mit einem Verfahren nach Anspruch 26 erhältlichen Hydroxyalkylstärkederivats zur Herstellung eines Arzneimittels zur Behandlung von Störungen bei aus der aus Krebspatienten, wie etwa myelosuppressive Chemotherapie erhaltenden Krebspatienten, Induktions- oder Konsolidierungschemotherapie erhaltenden Patienten mit akuter myeloischer Leukämie und/oder Knochenmarktransplantation erhaltenden Krebspatienten, Patienten, bei denen Vorläuferzellen des peripheren Bluts gesammelt werden und eine Therapie damit durchgeführt wird, und Patienten mit schwerer chronischer Neutropenie bestehenden Gruppe ausgewählten Patienten.

**47.** Verwendung eines mit einem Verfahren nach Anspruch 27 erhältlichen Hydroxyalkylstärkederivats zur Herstellung eines Arzneimittels zur Behandlung von Anämie, wie etwa bei Patienten mit chronischem Nierenversagen, mit Zidovudin behandelten HIV-infizierten Patienten, Krebspatienten mit Chemotherapie, oder zur Reduzierung allogener Bluttransfusion bei Operationspatienten.

**48.** Hydroxyalkylstärkederivat, wie es gemäß dem Verfahren nach einem der Ansprüche 25 bis 27 erhältlich ist, zur Behandlung von Störungen bei aus der aus Krebspatienten, wie etwa myelosuppressive Chemotherapie erhaltenden Krebspatienten, Induktions- oder Konsolidierungschemotherapie erhaltenden Patienten mit akuter myeloischer Leukämie und/oder Knochenmark - transplantation erhaltenden Krebspatienten, Patienten, bei denen Vorläuferzellen des peripheren Bluts gesammelt werden und eine Therapie damit durchgeführt wird, und Patienten mit schwerer chronischer Neutropenie bestehenden Gruppe ausgewählten Patienten; Anämie, wie etwa bei Patienten mit chronischem Nierenversagen, mit Zidovudin behandelten HIV-infizierten Patienten, Krebspatienten mit Chemotherapie, oder zur Reduzierung allogener Bluttransfusion bei Operationspatienten.

## Revendications

**1.** Méthode de production d'un dérivé d'amidon hydroxyalkylé (HAS), comprenant

a) la réaction d'amidon hydroxyalkylé éventuellement oxydé avec un composé (D) comprenant au moins deux fonctions $-O-NH_2$ ou un sel de celui-ci, et
b) la réaction du dérivé d'amidon hydroxyalkylé obtenu à l'étape a) avec un composé (L) comprenant au moins deux fonctions $W_1$ et $W_2$ choisies indépendamment dans le groupe constitué d'un groupement aldéhyde, d'un groupement aldéhyde protégé de façon appropriée, d'un groupement céto, et d'un groupement céto protégé de façon appropriée,

le composé (L) utilisé à l'étape b) ayant la structure selon la formule (III)

$$W_1\text{-}R_9\text{-}W_2 \qquad \text{(III)}$$

dans laquelle $R_9$ est choisi parmi une liaison chimique, de préférence une simple liaison, un alkylène saturé ou insaturé, cyclique ou linéaire, ramifié ou non ramifié, substitué ou non substitué, contenant éventuellement des hétéroatomes dans la chaîne alkyléne, un arylène substitué ou non substitué et un hétéroarylène substitué ou non substitué, un aralkylène substitué ou non substitué, un alkarylène substitué ou non substitué, et un hétéroarylène substitué ou non substitué, un hétéroaralkylène substitué ou non substitué, et un alkhétéroarylène substitué ou non substitué.

2. Méthode selon la revendication 1, **caractérisée en ce que** ledit amidon hydroxyalkylé a la structure selon la formule (I)

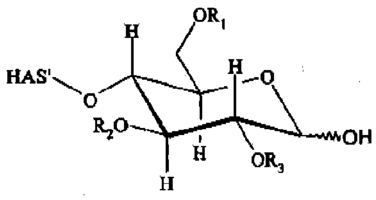

(I)

dans laquelle HAS' est le reste de la molécule d'amidon hydroxyalkylé et $R_1$, $R_2$ et $R_3$ sont indépendamment hydrogène ou un groupement hydroxyalkylé linéaire ou ramifié.

3. Méthode selon la revendication 2, **caractérisée en ce que** $R_1$, $R_2$ et $R_3$ sont indépendamment un groupement $(CH_2CH_2O)_n$-H, dans lequel n est un entier, de préférence 0, 1, 2, 3, 4, 5, ou 6.

4. Méthode selon l'une quelconque des revendications précédentes, **caractérisée en ce que** l'amidon hydroxyalkylé est un amidon hydroxyéthylé.

5. Méthode selon l'une quelconque des revendications précédentes, **caractérisée en ce que** l'amidon hydroxyalkylé n'est pas oxydé préalablement à la réaction avec le composé (D) ou un sel de celui-ci à l'étape a).

6. Méthode selon l'une quelconque des revendications précédentes, **caractérisée en ce que** le composé (L) comprend au moins deux fonctions $W_1$ et $W_2$ choisies indépendamment dans le groupe constitué par un groupement aldéhyde, un groupement hémiacétal, $-CH(OH)_2$, un groupement acétal, un groupement céto, et un groupement cétal, choisies de préférence dans le groupe constitué par un groupement hémiacétal, $-CH(OH)_2$, un groupement acétal, un groupement cétal et le groupement -C(O)-R, dans lequel R est choisi dans le groupe constitué par hydrogène, un alkyle saturé ou insaturé, cyclique ou linéaire, ramifié ou non ramifié, substitué ou non substitué et un groupement aryle substitué ou non substitué, où, dans le cas où le groupement -C(O)-R est un groupement céto, le résidu R est choisi de préférence dans le groupe constitué par alkyle en $C_1$-$C_6$ et aryle en $C_6$-$C_{14}$, encore plus préférablement choisi dans le groupe constitué par méthyle, éthyle, n-propyle, isopropyle, n-butyle, isobutyle, et tert-butyle éventuellement substitués, de préférence non substitués.

7. Méthode selon l'une quelconque des revendications précédentes, **caractérisée en ce que** l'amidon hydroxyalkylé est mis en réaction avec le composé (D) ou un sel de celui-ci à l'extrémité réductrice de l'amidon hydroxyalkylé, qui n'est pas oxydé préalablement à la réaction à l'étape a).

8. Méthode selon l'une quelconque des revendications précédentes, **caractérisée en ce que** le composé (D) ou un sel de celui-ci est mis en réaction par l'intermédiaire d'au moins l'une desdites au moins deux fonctions $-O-NH_2$ avec un groupement aldéhyde et/ou hémiacétal de l'amidon hydroxyalkylé à l'étape a).

9. Méthode selon l'une quelconque des revendications précédentes, **caractérisée en ce que** le composé (D) ou un sel de celui-ci est mis en réaction par l'intermédiaire d'au moins l'une desdites au moins deux fonctions $-O-NH_2$ avec une extrémité réductrice de l'amidon hydroxyalkylé et **en ce que** l'amidon hydroxyalkylé n'est pas oxydé préalablement à la réaction à l'étape a).

10. Méthode selon l'une quelconque des revendications précédentes, **caractérisée en ce que** l'étape a) comprend en outre la réduction du dérivé d'amidon hydroxyalkylé obtenu préalablement à l'étape b).

**11.** Méthode selon l'une quelconque des revendications précédentes, **caractérisée en ce que** le composé (L) est mis en réaction par l'intermédiaire d'au moins l'une desdites au moins deux fonctions $W_1$ et $W_2$ avec au moins l'une des fonctions $H_2N$-O- du dérivé d'amidon hydroxyalkylé obtenu à l'étape a) ou un sel de celui-ci.

**12.** Méthode selon l'une quelconque des revendications précédentes, **caractérisée en ce que** le composé (D) utilisé à l'étape a) a la structure selon la formule (II)

$$H_2N\text{-}O\text{-}R_4\text{-}O\text{-}NH_2 \qquad \text{(II)}$$

ou un sel de celle-ci,

dans laquelle $R_4$ est choisi parmi un alkylène saturé ou insaturé, cyclique ou linéaire, ramifié ou non ramifié, substitué ou non substitué, contenant éventuellement des hétéroatomes dans la chaîne alkylène, un arylène substitué ou non substitué, un aralkylène substitué ou non substitué, un alkarylène substitué ou non substitué, et un hétéroarylène substitué ou non substituez, un hétéroaralkylène substitué ou non substitué, et un alkhétéroarylène substitué ou non substitué.

**13.** Méthode selon la revendication 12, **caractérisée en ce que** $R_4$ est choisi dans le groupe constitué d'alkylène en $C_1$-$C_{12}$, d'arylène en $C_6$-$C_{14}$, et de - $[(CR_5R_6)_mO]_n[CR_7R_8]_o$-, où
$R_5$, $R_6$, $R_7$ et $R_8$ sont, indépendamment les uns des autres, choisis dans le groupe constitué d'hydrogène, d'alkyle et d'aryle,
m est 2 à 4 ;
n est 0 à 20 ; et
o est 0 à 20, où au cas où n est 0, o n'est pas 0.

**14.** Méthode selon la revendication 13, **caractérisée en ce que** $R_1$ dans le composé (D) est -$[(CR_5R_6)_mO]_n[CR_7R_8]_o$-, où $R_5$, $R_6$, $R_7$ et $R_8$ sont, indépendamment les uns des autres, choisis dans le groupe constitué d'hydrogène, d'alkyle en $C_1$-$C_6$ et d'aryle en $C_6$-$C_{14}$,
m est 2 ;
n est 1 ; et
o est 2.

**15.** Méthode selon la revendication 14, **caractérisée en ce que** le composé (D) utilisé à l'étape a) est

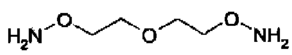

ou son sel.

**16.** Méthode selon l'une quelconque des revendications 1 à 15, **caractérisée en ce que** $R_9$ est un arylène substitué ou non substitué ou alkylène substitué ou non substitué, en particulier **en ce que** $R_9$ est un arylène en $C_6$-$C_{14}$ non substitué ou -$(CH_2)_n$-, n étant de préférence 1-6, de préférence phénylène.

**17.** Méthode selon la revendication 16, **caractérisée en ce que** $R_9$ est un arylène en $C_6$-$C_{14}$ non substitué.

**18.** Méthode selon la revendication 17, **caractérisée en ce que** le composé (L) utilisé à l'étape b) est

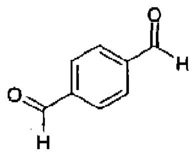

**19.** Méthode selon l'une quelconque des revendications précédentes, **caractérisée en ce que** l'étape b) comprend en outre la réduction du dérivé d'amidon hydroxyalkylé obtenu.

**20.** Méthode selon l'une quelconque des revendications précédentes, **caractérisée en ce que** le dérivé d'amidon hydroxyalkylé obtenu à l'étape b) est c) mis en réaction avec au moins un agent biologiquement actif.

**21.** Méthode selon la revendication 20, **caractérisée en ce que** ledit au moins un agent biologiquement actif utilisé à l'étape c) comprend au moins une fonction $-NH_2$.

**22.** Méthode selon l'une ou l'autre des revendications 20 et 21, **caractérisée en ce que** ledit au moins un agent biologiquement actif utilisé à l'étape c) est choisi dans le groupe constitué par un peptide, un polypeptide, une protéine et un dérivé, fragment ou mimétique fonctionnel du polypeptide ou de la protéine.

**23.** Méthode selon la revendication 22, **caractérisée en ce que** le polypeptide est choisi dans le groupe constitué par l'érythropoïétine (EPO), telle que l'EPO recombinante humaine (rhEPO) ou un mimétique d'EPO, les facteurs de stimulation des colonies (CSF), tels que le G-CSF comme le G-CSF recombinant humain (rhG-CSF), l'alpha-Interféron (IFN alpha), le bêta-Interféron (IFN bêta) ou le gamma-Interféron (IFN gamma), tels que l'IFN alpha et l'IFN bêta comme l'IFN alpha ou l'IFN bêta recombinants humains (rhIFN alpha ou rhIFN bêta), les interleukines, par exemple l'IL-1 à l'IL-18 telles que l'IL-2 ou l'IL-3 comme l'IL-2 ou l'IL-3 recombinantes humaines (rhIL-2 ou rhIL-3), les protéines sériques telles que les facteurs de coagulation II-XIII comme le facteur VIII, le facteur VII, le facteur IX, le facteur II, le facteur III, le facteur IV, le facteur V, le facteur VI, le facteur X, le facteur XI, le facteur XII, le facteur XIII, l'alpha1-antitrypsine (A1AT), la protéine C activée (APC), les activateurs du plasminogène tels que l'activateur du plasminogène de type tissulaire (tPA), tel que l'activateur tissulaire du plasminogène humain (hTPA), l'AT III telle que l'AT III recombinante humaine (rhAT III), la myoglobine, l'albumine telle que la sérum albumine bovine (BSA), les facteurs de croissance, tels que le facteur de croissance épidermique (EGF), le facteur de croissance des thrombocytes (PDGF), le facteur de croissance des fibroblastes (FGF), le facteur de croissance dérivé du cerveau (BDGF), le facteur de croissance des nerfs (NGF), le facteur de croissance des cellules B (BCGF), le facteur de croissance neurotrophique dérivé du cerveau (BDNF), le facteur neurotrophique ciliaire (CNTF), les facteurs de croissance transformants tels que le TGF alpha ou le TGF bêta, les BMP (protéines morphogéniques osseuses), les hormones de croissance telles que l'hormone de croissance humaine, les facteurs de nécrose de tumeurs tels que le TNF alpha ou le TNF bêta, la somatostatine, la somatotropine, les somatomédines, l'hémoglobine, les hormones ou les prohormones telles que l'insuline, la gonadotropine, l'hormone de stimulation des mélanocytes (alpha-MSH), la triptoréline, les hormones hypothalamiques telles que les hormones antidiurétiques (ADH) et l'ocytocine ainsi que les hormones de libération et les hormones inhibant la libération, l'hormone parathyroïdienne, les hormones thyroïdiennes telles que la thyroxine, la thyrotropine, la thyrolibérine, la calcitonine, le glucagon, les peptides de type glucagon (GLP-1, GLP-2, et similaires), les exendines telles que l'exendine-4, la leptine, la vasopressine, la gastrine, la sécrétine, les intégrines, les hormones glycoprotéiques (par exemple LH, FSH et similaires), les hormones de stimulation des mélanocytes, les lipoprotéines et les apolipoprotéines telles que l'apo-B, l'apo-E, l'apo-$L_a$, les immunoglobulines telles que l'IgG, l'IgE, l'IgM, l'IgA, l'IgD et des fragments de celles-ci, l'hirudine, l'inhibiteur de la voie tissulaire, les protéines végétales telles que la lectine ou la ricine, le venin d'abeille, le venin de serpent, les immunotoxines, l'antigène E, l'inhibiteur de l'alpha-protéinase, l'allergène d'ambroisie, la mélanine, les protéines d'oligolysine, les protéines d'RGD ou les récepteurs éventuellement correspondants pour l'une de ces protéines ; la prolactine ou un mutant de celle-ci, tel que G129R, dans lequel l'acide aminé de type sauvage en position 129, la glycine, est remplacé par l'arginine et un dérivé ou fragment fonctionnel de l'un quelconque de ces protéines ou récepteurs.

**24.** Méthode selon la revendication 23, **caractérisée en ce que** le polypeptide est choisi dans le groupe constitué par l'érythropoïétine (EPO), telle que l'EPO recombinante humaine (rhEPO), les facteurs de stimulation des colonies (CSF), tels que le G-CSF recombinant humain (rhG-CSF), l'alpha-Interféron (IFN alpha), le bêta-Interféron (IFN bêta), le gamma-Interféron (IFN gamma), tels que l'IFN alpha ou l'IFN bêta recombinants humains (rhIFN alpha ou (rhIFN bêta), l'A1AT et le facteur IX.

**25.** Méthode selon la revendication 24, **caractérisée en ce que** le polypeptide est l'IFN alpha.

**26.** Méthode selon la revendication 24, **caractérisée en ce que** le polypeptide est le G-CSF.

**27.** Méthode selon la revendication 24, **caractérisée en ce que** le polypeptide est l'EPO.

**28.** Méthode selon l'une quelconque des revendications 20 à 27, **caractérisée en ce que** le dérivé d'amidon hydroxyalkylé obtenu à l'étape b) est mis en réaction à l'étape c) avec ledit au moins un agent biologiquement actif par l'intermédiaire d'au moins l'une des fonctions $W_1$ et $W_2$ introduites dans le dérivé d'amidon hydroxyalkylé par le

composé (L) à l'étape b).

**29.** Méthode selon la revendication 28, **caractérisée en ce que** le dérivé d'amidon hydroxyalkylé obtenu à l'étape b) est mis en réaction à l'étape c) par l'intermédiaire de ladite au moins une fonction -$NH_2$ de l'agent biologiquement actif avec au moins l'une des fonctions $W_1$ et $W_2$ introduites dans le dérivé d'amidon hydroxyalkylé par le composé (L) à l'étape b).

**30.** Méthode selon l'une quelconque des revendications 21 à 29 précédentes, **caractérisée en ce que** ladite au moins une fonction -$NH_2$ contenue dans ledit au moins un agent biologiquement actif utilisé à l'étape c) est la fonction N-terminale -$NH_2$ d'une polypeptide ou d'une protéine.

**31.** Méthode selon l'une quelconque des revendications 21 à 30, **caractérisée en ce que** l'étape c) est effectuée dans les conditions réactionnelles pour une amination réductrice.

**32.** Méthode selon l'une quelconque des revendications 21 à 31, **caractérisée en ce que** l'étape c) est effectuée avec un agent de réduction, de préférence avec une borane, en particulier avec le $NaCNBH_3$.

**33.** Méthode selon l'une quelconque des revendications 20 à 32 précédentes, **caractérisée en ce qu'**à l'étape c), le rapport molaire entre le dérivé d'amidon hydroxyalkylé obtenu à l'étape b) et l'agent biologiquement actif est d'environ 1:1 à environ 100:1 équivalents, sur la base du poids moléculaire moyen en poids ($M_w$) du dérivé d'amidon hydroxyalkylé obtenu à l'étape b).

**34.** Méthode selon la revendication 33, **caractérisée en ce que** le rapport est d'environ 1:1 à environ 20:1.

**35.** Méthode selon l'une quelconque des revendications 31 à 34, **caractérisée en ce que** la concentration du dérivé d'amidon hydroxyalkylé obtenu à l'étape b) utilisée à l'étape c) est supérieure à environ 10 % m/v.

**36.** Méthode selon l'une quelconque des revendications 20 à 35, **caractérisée en ce que** la température à l'étape c) est d'environ 0°C à environ 25°C.

**37.** Méthode selon l'une quelconque des revendications précédentes, **caractérisée en ce qu'**à l'étape a), l'amidon hydroxyalkylé qui n'est pas oxydé est mis en réaction à une extrémité réductrice de l'amidon hydroxyalkylé avec le composé (D) qui est

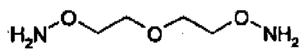

par l'intermédiaire de l'une des fonctions -O-$NH_2$, et à l'étape b), le dérivé d'amidon hydroxyalkylé obtenu à l'étape b) est mis en réaction par l'intermédiaire de la fonction -O-$NH_2$ avec l'une des fonctions -CH=O du composé (L) qui est

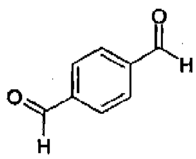

**38.** Méthode selon la revendication 37, **caractérisée en ce que** le dérivé d'amidon hydroxyalkylé obtenu à l'étape b) est mis en réaction avec de l'IFN alpha à l'étape c) dans des conditions d'amination réductrice, en particulier en présence de $NaCNBH_3$.

**39.** Dérivé d'amidon hydroxyalkylé susceptible d'être obtenu par une méthode selon l'une quelconque des revendications 1 à 38.

**40.** Dérivé d'HAS, de préférence dérivé d'HES, selon la structure

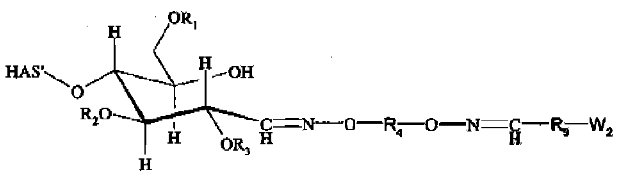

et/ou la structure cyclique correspondante

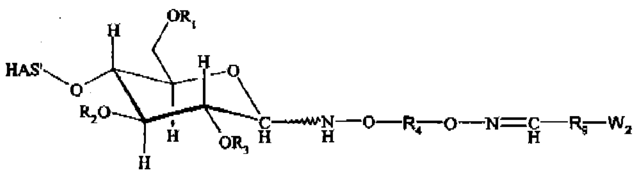

et/ou

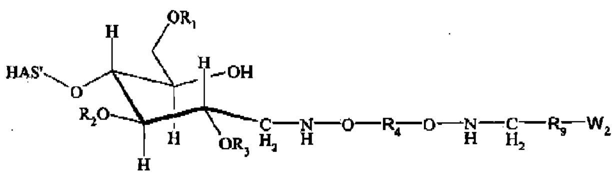

où, plus préférablement, HES a une poids moléculaire moyen d'environ 1 à environ 1000 kDa, plus préférablement d'environ 1 à environ 800 kDa, plus préférablement d'environ 1 à environ 500 kDa, plus préférablement d'environ 2 à environ 400 kDa, plus préférablement d'environ 5 à environ 300 kDa, plus préférablement d'environ 10 à environ 200 kDa, en particulier d'environ 50 à environ 150 kDa, une substitution molaire de 0,1 à 3, de préférence 0,4 à 1,3, par exemple 0,4, 0,5, 0,6, 0,7, 0,8, 0,9, 1,0, 1,1, 1,2 ou 1,3, et un rapport de substitution $C_2$:$C_6$ de préférence dans la gamme de 2 à 20, plus préférablement dans la gamme de 2 à 15, et encore plus préférablement dans la gamme de 3 à 12 ;
où HAS' est le reste de la molécule d'amidon hydroxyalkylé et $R_1$, $R_2$ et $R_3$ sont indépendamment hydrogène, un groupement hydroxyalkylé linéaire ou ramifié ou le groupement

$$-[(CR^1R^2)_mO]_n[CR^3R^4]_o\text{-OH}$$

où $R^1$, $R^2$, $R^3$ et $R^4$ sont choisis indépendamment dans le groupe constitué d'hydrogène, et d'un groupement alkyle, de préférence hydrogène et un groupement méthyle,
m est 2 à 4, où les résidus $R^1$ et $R^2$ peuvent être identiques ou différents dans les m groupements $CR^1R^2$ ;
n est 0 à 20, de préférence 0 à 4 ;
o est 2 à 20, de préférence 2 à 4, où les résidus $R^3$ et $R^4$ peuvent être identiques ou différents dans les o groupements $CR^3R^4$ ;
dans laquelle $R_4$ est choisi parmi un alkylène saturé ou insaturé, cyclique ou linéaire, ramifié ou non ramifié, substitué ou non substitué, contenant éventuellement des hétéroatomes dans la chaîne alkylène, un arylène substitué ou non substitué, un aralkylène substitué ou non substitué, un alkarylène substitué ou non substitué, et un hétéroarylène substitué ou non substitué, un hétéroaralkylène substitué ou non substitué, et un alkhétéroarylène substitué ou non substitué ;
dans laquelle $R_9$ est choisi parmi une liaison chimique, de préférence une simple liaison, un alkylène saturé ou insaturé, cyclique ou linéaire, ramifié ou non ramifié, substitué ou non substitué, contenant éventuellement des hétéroatomes dans la chaîne alkylène, un arylène substitué ou non substitué et un hétéroarylène substitué ou non substitué, un aralkylène substitué ou non substitué, un alkarylène substitué ou non substitué, et un hétéroarylène substitué, ou non substitué, un hétéroaralkylène substitué ou non substitué, et un alkhétéroarylène substitué ou non substitué ;
et où $W_2$ est choisi dans le groupe constitué d'un groupement aldéhyde, d'un groupement aldéhyde protégé de façon appropriée, d'un groupement céto, et d'un groupement céto protégé de façon appropriée, de préférence choisi dans le groupe constitué par un groupement aldéhyde, un groupement hémiacétal, $-CH(OH)_2$, un grou-

pement acétal, un groupement céto, et un groupement cétal, choisi de préférence dans le groupe constitué par un groupement hémiacétal, $-CH(OH)_2$, un groupement acétal, un groupement cétal et le groupement -C(O)-R, dans lequel R est choisi dans le groupe constitué par hydrogène, un alkyle saturé ou insaturé, cyclique ou linéaire, ramifié ou non ramifié, substitué ou non substitué et un groupement aryle substitué ou non substitué, où, dans le cas où le groupement -C(O)-R est un groupement céto, le résidu R est choisi de préférence dans le groupe constitué par alkyle en $C_1$-$C_6$ et aryle en $C_6$-$C_{14}$, encore plus préférablement choisi dans le groupe constitué par méthyle, éthyle, n-propyle, isopropyle, n-butyle, isobutyle, et tert-butyle éventuellement substitués, de préférence non substitués.

**41.** Dérivé d'HAS, de préférence dérivé d'HES, selon la structure

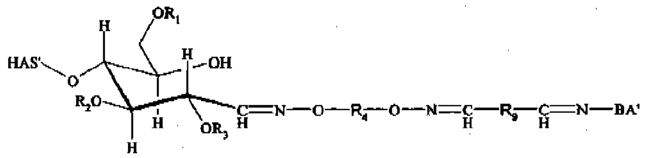

et/ou la structure cyclique correspondante

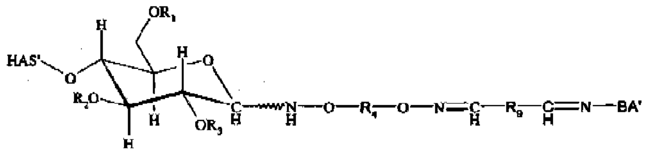

et/ou

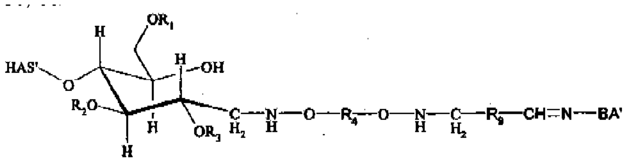

et/ou

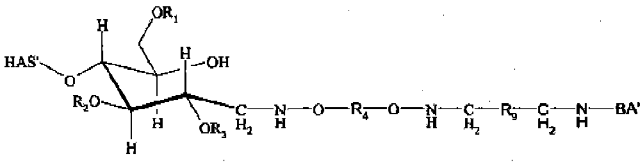

et/ou

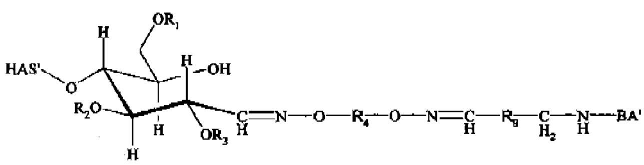

et/ou la structure cyclique correspondante

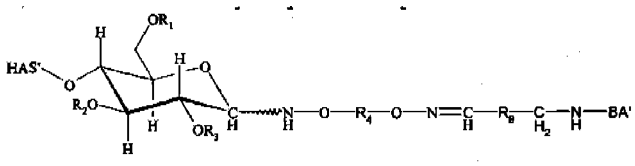

où, plus préférablement, HES a un poids moléculaire moyen d'environ 1 à environ 1000 kDa, plus préférablement d'environ 1 à environ 800 kDa, plus préférablement d'environ 1 à environ 500 kDa, plus préférablement d'environ 2 à environ 400 kDa, plus préférablement d'environ 5 à environ 300 kDa, plus préférablement d'environ 10 à environ 200 kDa, en particulier d'environ 50 à environ 150 kDa, une substitution molaire de 0,1 à 3, de préférence 0,4 à 1,3, par exemple 0, 4, 0,5, 0, 6, 0,7, 0,8, 0,9, 7.,0, 1,1, 1,2 ou 1,3, et un rapport de substitution $C_2$:$C_6$ de préférence dans la gamme de 2 à 20, plus préférablement dans la gamme de 2 à 15, et encore plus préférablement dans la gamme de 3 à 12 ;
où HAS' est le reste de la molécule d'amidon hydroxyalkylé et $R_1$, $R_2$ et $R_3$ sont indépendamment hydrogène, un groupement hydroxyalkylé linéaire ou ramifié ou le groupement

$$-[(CR^1R^2)_mO]_n[CR^3R^4]_o\text{-OH}$$

où $R^1$, $R^2$, $R^3$ et $R^4$ sont choisis indépendamment dans le groupe constitué d'hydrogène, et d'un groupement alkyle, de préférence hydrogène et un groupement méthyle,
m est 2 à 4, où les résidus $R^1$ et $R^2$ peuvent être identiques ou différents dans les m groupements $CR^1R^2$ ;
n est 0 à 20, de préférence 0 à 4 ;
o est 2 à 20, de préférence 2 à 4, où les résidus $R^3$ et $R^4$ peuvent être identiques ou différents dans les o groupements $CR^3R^4$ ;
dans laquelle $R_4$ est choisi parmi un alkylène saturé ou insaturé, cyclique ou linéaire, ramifié ou non ramifié, substitué ou non substitué, contenant éventuellement des hétéroatomes dans la chaîne alkylène, un arylène substitué ou non substitué, un aralkylène substitué ou non substitué, un alkarylène substitué ou non substitué, et un hétéroarylène substitué ou non substitué, un hétéroaralkylène substitué ou non substitué, et une alkhétéroarylène substitué ou non substitué ;
dans laquelle $R_9$ est choisi parmi une liaison chimique, de préférence une simple liaison, un alkylène saturé ou insaturé, cyclique ou linéaire, ramifié ou non ramifié, substitué ou non substitué, contenant éventuellement des hétéroatomes dans la chaîne alkylène, un arylène substitué ou non substitué et un hétéroarylène substitué ou non substitué, un aralkylène substitué ou non substitué, un alkarylène substitué ou non substitué, et un hétéroarylène substitué ou non substitué, un hétéroaralkylène substitué ou non substitué, et un alkhétéroarylène substitué ou non substitué ;
et où BA est un agent biologiquement actif, BA comprenant au moins une fonction $-NH_2$, BA étant représenté en tant que $H_2N$-BA', où BA' est le reste de BA, BA étant de préférence choisi dans le groupe constitué par l'érythropoïétine (EPO), telle que l'EPO recombinante humaine (rhEPO), les facteurs de stimulation des colonies (CSF), tels que le G-CSF recombinant humain (rhG-CSF), l'alpha-Interféron (IFN alpha), le bêta-Interféron (IFN bêta), le gamma-Interféron (IFN gamma), tels que l'IFN alpha ou l'IFN bêta recombinants humains (rhIFN alpha ou rhIFN bêta), l'A1AT et le facteur IX,
notamment préférablement

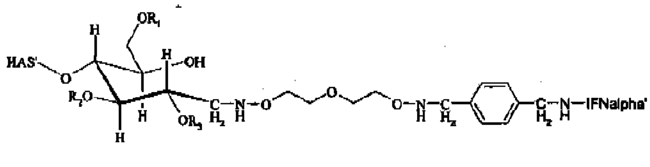

**42.** Composition pharmaceutique comprenant, dans une quantité thérapeutiquement efficace, un dérivé d'amidon hydroxyalkylé susceptible d'être obtenu par une méthode selon l'une quelconque des revendications 20 à 38 ou un dérivé d'amidon hydroxyalkylé selon la revendication 41.

**43.** Composition pharmaceutique selon la revendication 42, **caractérisée en ce que** le dérivé d'amidon hydroxyalkylé est susceptible d'être obtenu par une méthode selon la revendication 25.

**44.** Dérivé d'amidon hydroxyalkylé susceptible d'être obtenu par une méthode selon l'une quelconque des revendications

20 à 38, ou dérivé d'amidon hydroxyalkylé selon la revendication 41, en tant qu'agent thérapeutique ou prophylactique.

**45.** Utilisation d'un dérivé d'amidon hydroxyalkylé susceptible d'être obtenu par une méthode selon la revendication 25, pour la préparation d'un médicament destiné au traitement d'une maladie choisie dans le groupe constitué par le cancer, tel que la leucémie à tricholeucocytes, le mélanome malin, le lymphome folliculaire et/ou le sarcome de Kaposi associé au SIDA, les condylomes acuminés, l'hépatite B chronique et l'hépatite C chronique, de préférence l'hépatite B et l'hépatite C chroniques.

**46.** Utilisation d'un dérivé d'amidon hydroxyalkylé susceptible d'être obtenu par une méthode selon la revendication 26, pour la préparation d'un médicament destiné au traitement de troubles chez des patients choisis dans le groupe constitué par les patients atteints du cancer, tels que les patients atteints du cancer recevant une chimiothérapie myélosuppressive, les patients atteints de leucémie myéloïde aiguë recevant une chimiothérapie d'induction ou de consolidation et/ou les patients atteints du cancer recevant une greffe de la moelle osseuse, les patients subissant un prélèvement et une thérapie de cellules progénitrices du sang périphérique, et les patients atteints de neutropénie chronique grave.

**47.** Utilisation d'un dérivé d'amidon hydroxyalkylé susceptible d'être obtenu par une méthode selon la revendication 27, pour la préparation d'un médicament destiné au traitement de l'anémie, par exemple de patients atteints d'insuffisance rénale chronique, de patients infectés par le VIH traités par la zidovudine, de patients atteints du cancer ayant une chimiothérapie, ou pour la réduction de la transfusion de sang allogénique chez les patients ayant subi une opération chirurgicale.

**48.** Dérivé d'amidon hydroxyalkylé susceptible d'être obtenu selon la méthode selon l'une quelconque des revendications 25 à 27, destiné au traitement de troubles chez des patients choisis dans le groupe constitué par les patients atteints du cancer, tels que les patients atteints du cancer recevant une chimiothérapie myélosuppressive, les patients atteints de leucémie myéloïde aiguë recevant une chimiothérapie d'induction ou de consolidation et/ou les patients atteints du cancer recevant une greffe de la moelle osseuse, les patients subissant un prélèvement et une thérapie de cellules progénitrices du sang périphérique, et les patients atteints de neutropénie chronique grave ; de l'anémie, par exemple de patients atteints d'insuffisance rénale chronique, de patients infectés par le VIH traités par la zidovudine, de patients atteints du cancer ayant une chimiothérapie, ou pour la réduction de la transfusion de sang allogénique chez les patients ayant subi une opération chirurgicale.

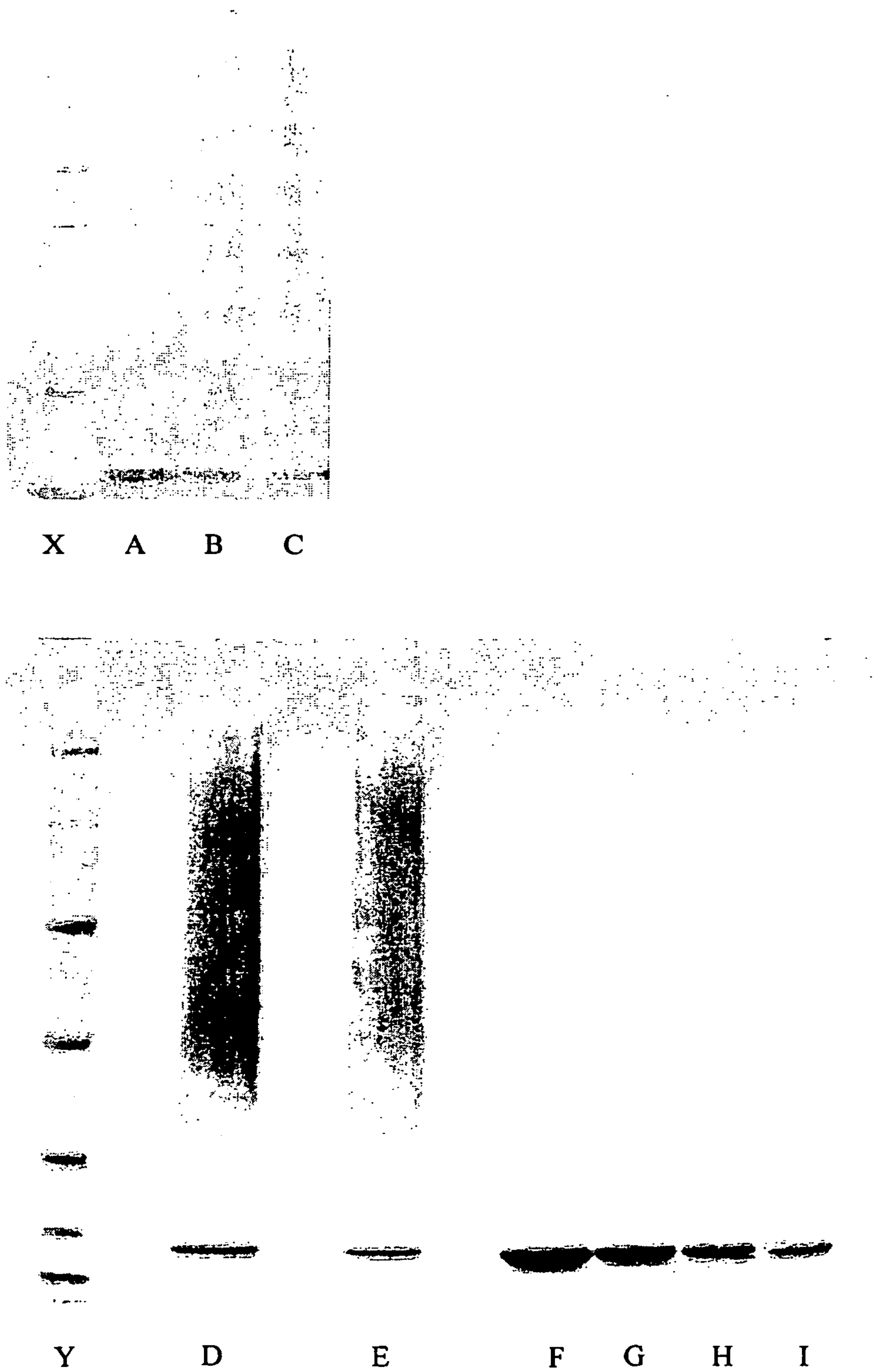


Fig. 1

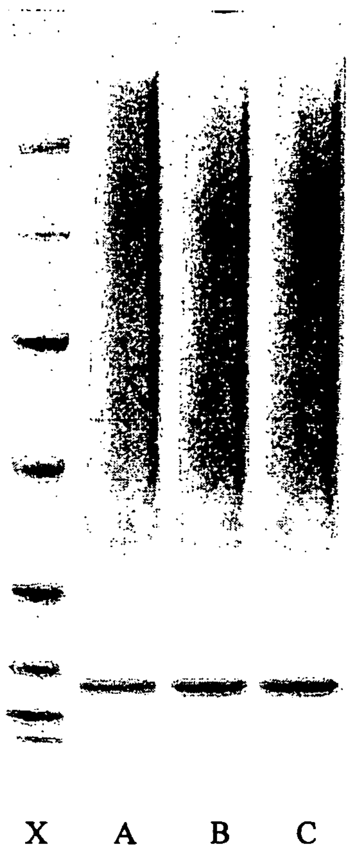


Fig. 2

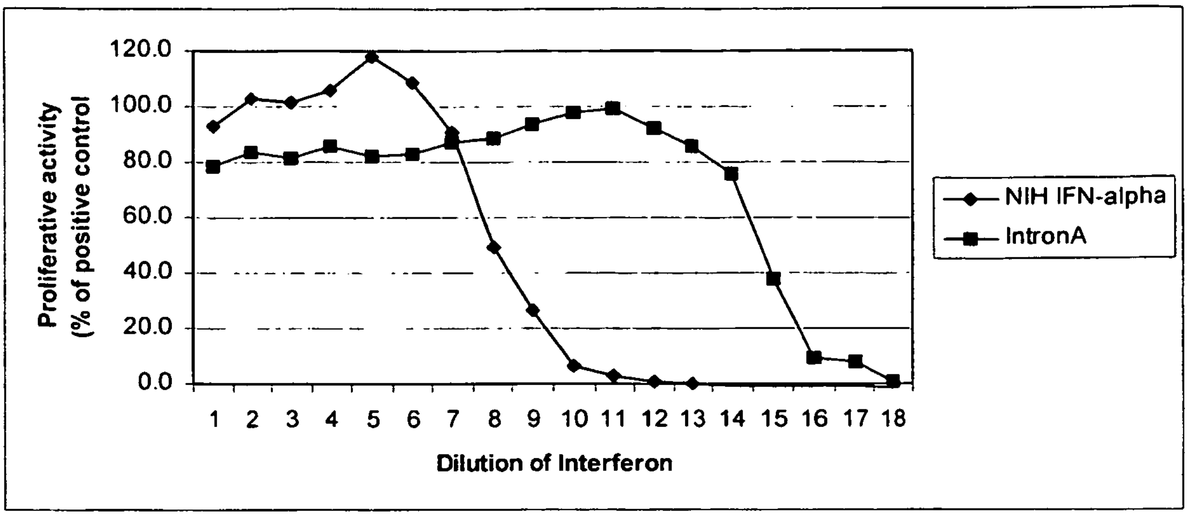

120.0
100.0
80.0
60.0
40.0
20.0
0.0
Proliferative activity
(% of positive control
1 2 3 4 5 6 7 8 9 10 11 12 13 14 15 16 17 18
Dilution of Interferon
NIH IFN-alpha
IntronA


Fig. 3

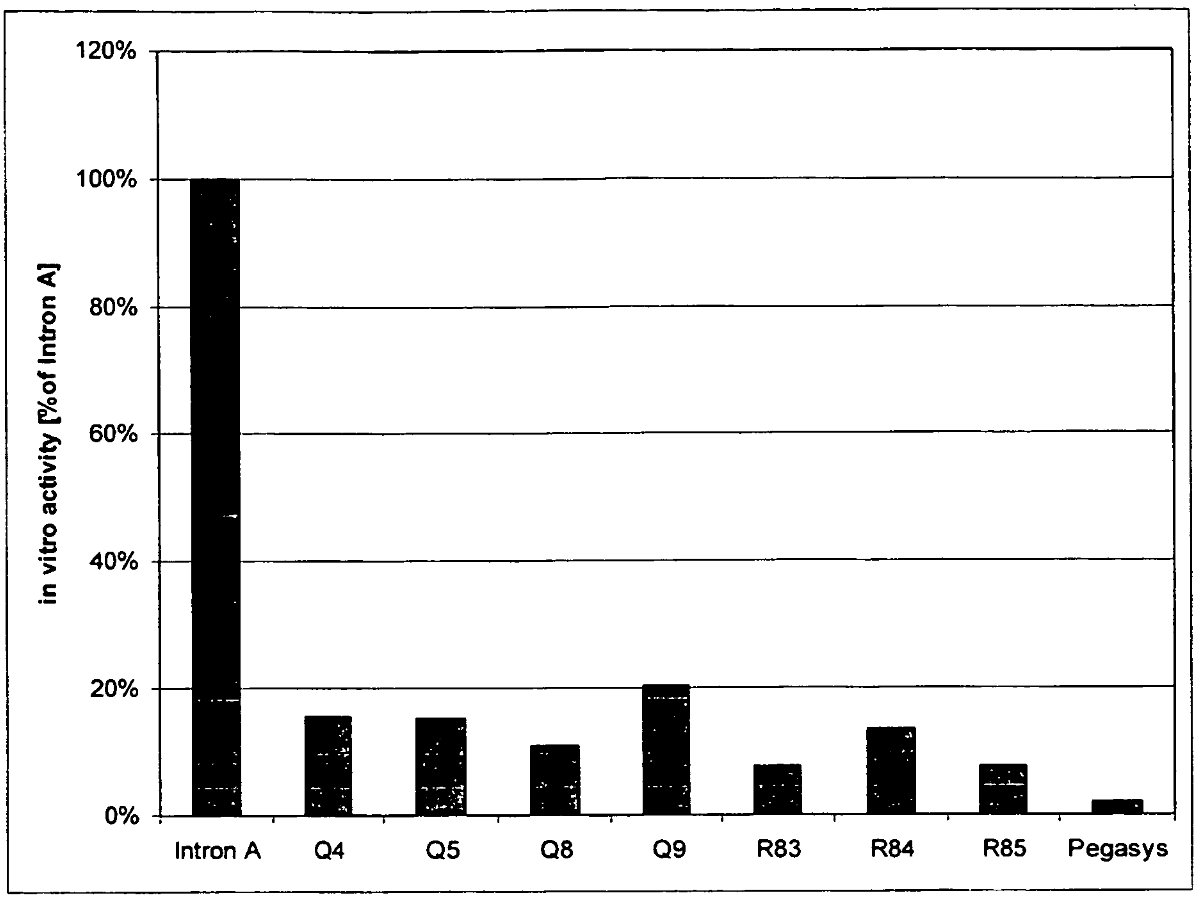
120%
100%
80%
60%
40%
20%
0%
in vitro activity [%of Intron A]
Intron A
Q4
Q5
Q8
Q9
R83
R84
R85
Pegasys

Fig. 4

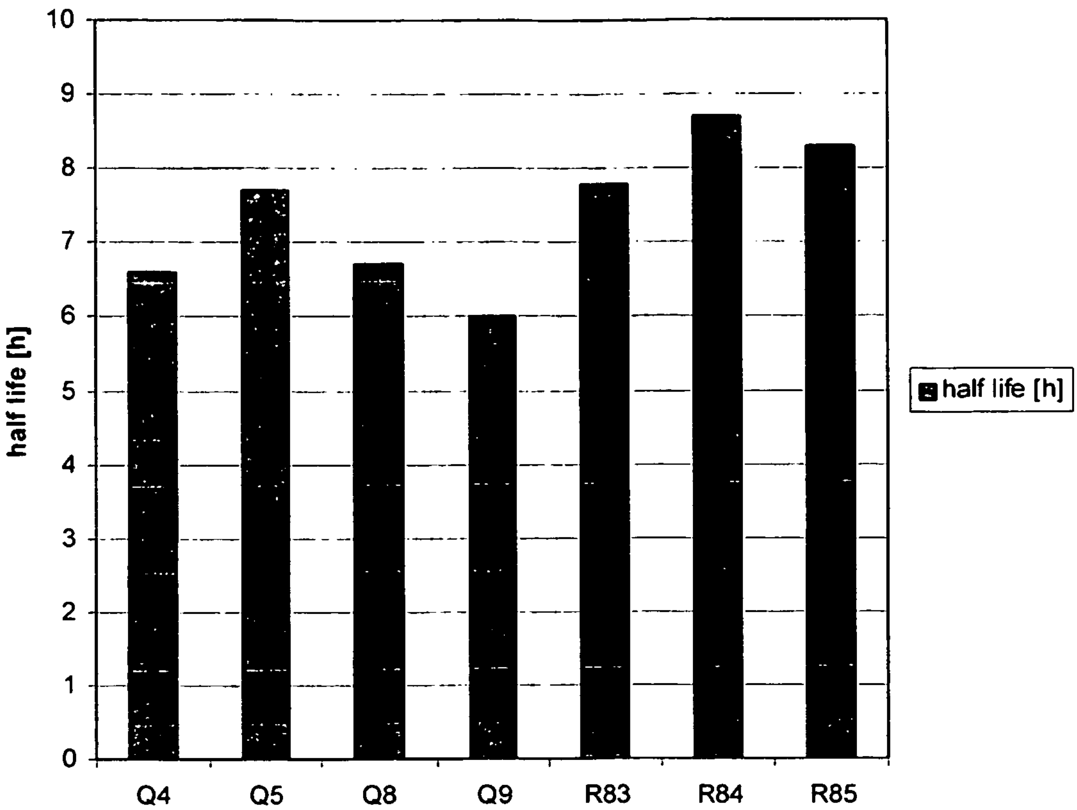


Fig. 5

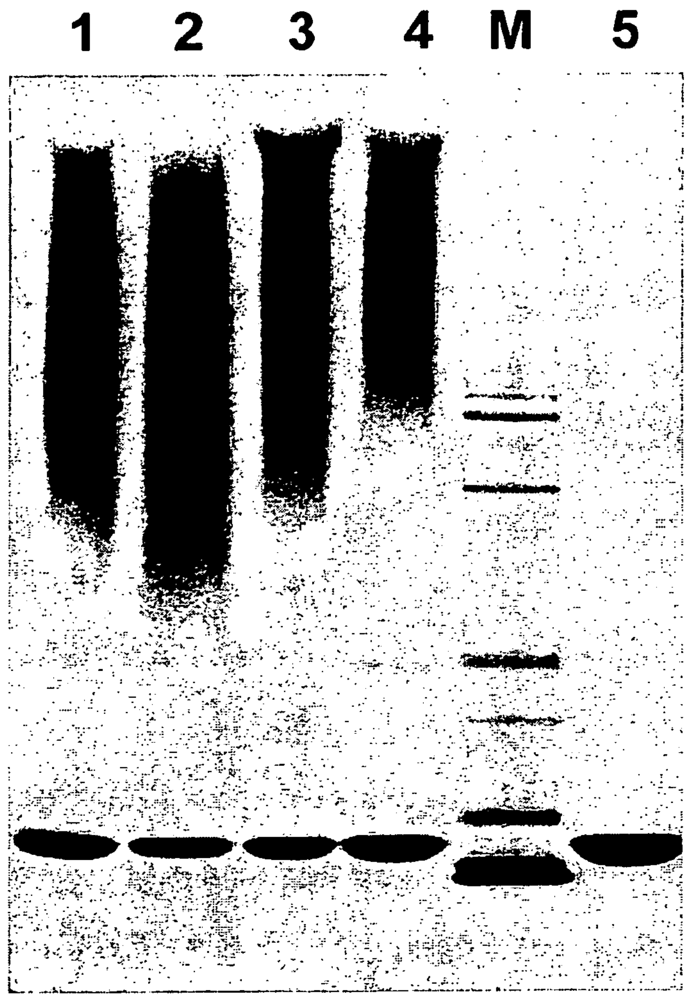


Fig. 6

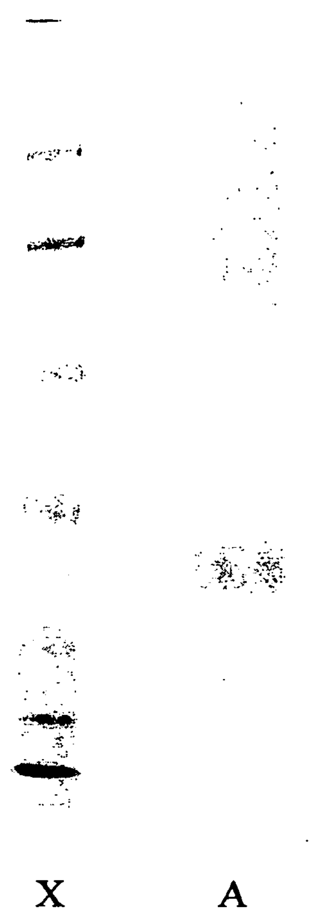


Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 2616086
- WO 9428024 A
- WO 02080979 A
- WO 03074087 A
- WO 03074088 A
- WO 2005014024 A
- WO 2005092390 A
- WO 2004024777 A
- WO 2004024776 A
- DE 3029307 A1
- WO 2005092928 A
- US 20060194940 A1
- US 7157546 B2
- EP 1591467 A1
- WO 2004022630 A2
- US 6916962 B2
- US 6956135 B2
- WO 03049699 A2
- US 3990237 A
- US 5990237 A
- WO 0066633 A
- WO 0018893 A
- US 4454161 A
- EP 0418945 A
- JP 2001294601 A
- US 2002065410 A


### Non-patent literature cited in the description


- **Sommermeyer et al.** *Krankenhauspharmazie,* 1987, vol. 8 (8), 271-278
- **Weidler et al.** *Arzrieimittelforschung/Drug Res.,* 1991, vol. 41, 494-498
- **Spivak ; Hogans.** *Blood,* 1989, vol. 73, 90
- **McMahon et al.** *Blood,* 1990, vol. 76, 1718
- **Klemm D. et al.** Comprehensive Cellulose Chemistry. Wiley-VCH, 1998, vol. 2
- **Weidler et al.** *Arzneim.-Forschung/Drug Res.,* 1991, vol. 41, 494-498
- **Boturyn et al.** *Tetrahedron,* 1997, vol. 53, 5485-5492
- **K.R. Reddy et al.** *Advanced Drug Delivery Reviews,* 2002, vol. 54, 571-586
- **Weidler et al.** *Arzneimittelforschung/Drug Res.,* 1991, vol. 41, 494-498
- **K.R. Reddy et al.** *Advaced Drug Delivery Reviews,* 2002, vol. 54, 571-586